# EUROPEAN PATENT SPECIFICATION

(11) **EP 4 102 514 B1**
(45) Date of publication and mention of the grant of the patent: **23.04.2025**
(21) Application number: 21750360.6
(22) Date of filing: 02.02.2021
(51) Int. Cl.: G16H 20/60

(54) **PROGRAM, INFORMATION PROCESSING METHOD, AND INFORMATION PROCESSING DEVICE**
PROGRAMM, INFORMATIONSVERARBEITUNGSVERFAHREN UND INFORMATIONSVERARBEITUNGSVORRICHTUNG
PROGRAMME, PROCÉDÉ DE TRAITEMENT D'INFORMATIONS ET DISPOSITIF DE TRAITEMENT D'INFORMATIONS

(30) Priority: 03.02.2020 JP 2020016395; 07.08.2020 WO PCT/JP2020/030341
(43) Date of publication of application: 14.12.2022
(73) Proprietor: Mizkan Holdings Co., Ltd., Handa-shi, Aichi 475-8585 (JP)
(72) Inventor: ISHIGAKI, Kouji, Handa-shi, Aichi 475-8585 (JP); KISHI, Mikiya, Handa-shi, Aichi 475-8585 (JP); ICHIKAWA, Kohei, Handa-shi, Aichi 475-8585 (JP)
(74) Representative: Hasegawa, Kan
(86) International application number: PCT/JP2021/003615
(87) International publication number: WO 2021/157540

(56) References cited:
- WO-A1-2018/213737
- JP-A- 2007 328 464
- JP-A- 2007 328 464
- JP-A- 2008 262 504
- JP-A- 2011 221 637
- JP-A- 2013 250 698
- JP-A- 2014 021 723
- JP-A- 2015 176 414
- JP-A- 2016 091 061
- JP-A- 2016 184 403
- JP-A- 2017 097 895
- JP-A- 2018 094 242
- JP-A- 2018 124 702
- JP-A- 2018 190 422
- JP-A- 2018 200 730
- KR-A- 20190 139 501
- KR-B1- 101 940 754
- HOSADA SHOU: "Meal analysis service utilizing Nestlé", LINE AND AL-BASED MEAL ANALYSIS SERVICE KEIT..., 28 May 2018 (2018-05-28), XP055847549, Retrieved from the Internet <URL:https://web.archive.org/web/20180528150952/https://k-tai.watch.impress.co.jp/docs/news/1124285.html> [retrieved on 20210430]

## Description

### [Technical Field]

The present disclosure relates to a program, an information processing method, and an information processing apparatus.

### [Background Art]

With the spread of the Internet, various kinds of information are being provided via the Internet. Document JP 2019 023919 A discloses a healthcare server which provides, based on information on a healthcare subject, optimum advice with respect to a state of health, a nutritional status, or the like of the healthcare subject. The healthcare server disclosed in Document JP 2019 023919 A enables expert advice to be provided in accordance with nutritional information of the healthcare subject.

Document JP 2007 328464 A discloses a buying activity management device provided with: a biological information acquisition part and a user information acquisition part for acquiring the biological information and user information of a user to be used for deciding whether or not food as the object of buying is suitable for being taken by the user; a buying activity acquisition part for acquiring new buying activity information showing the content of new buying activity by the user; and a buying activity decision part for deciding the validity/invalidity of new buying activity on the basis of the biological information and the user information and the new buying activity information, thereby it is possible to give the advice to the user whether or not the food is suitable for being taken by the user on the basis of basic information of the user. [Summary of Invention]

### [Problems to be Solved by Invention]

However, with conventional systems such as that disclosed in Document JP 2019 023919 A, it is difficult to accurately comprehend detailed information on food (dietary content) consumed by a user and, in a state where detailed information cannot be collected, there is a problem in that it is difficult to provide appropriate advice in consideration of food (dietary content) consumed by the user or food specified by the user. In addition, conventional methods are limited to advising a subject in a passive manner and it is difficult to provide advice that supports a user in attaining a goal based on an item that is achieved by consuming a specific article of food.

The present disclosure has been devised in consideration of such circumstances and an object thereof is to provide a program or the like capable of providing a user with appropriate advice.

### [Means for Solving Problems]

The problem is solved by the teachings of the independent claims. Further embodiments are defined in the dependent claims. The invention is defined by the appended claims.

### [Effects of Invention]

In an aspect of the present disclosure, a user can be provided with appropriate advice.

### [Brief Description of Drawings]

FIG. 1 is a schematic view showing a configuration example of an information processing system.
FIG. 2 is a block diagram showing a configuration example of a server and a user terminal.
FIG. 3A is a schematic view showing a configuration example of a DB stored in a server.
FIG. 3B is a schematic view showing a configuration example of a DB stored in a server.
FIG. 4 is a schematic view showing a configuration example of a DB stored in a server.
FIG. 5 is a schematic view showing a configuration example of a DB stored in a server.
FIG. 6 is a flow chart showing an example of a registration processing procedure of user information.
FIG. 7 is a flow chart showing an example of a registration processing procedure of user information.
FIG. 8A is a schematic view showing a screen example of a user terminal.
FIG. 8B is a schematic view showing a screen example of a user terminal.
FIG. 9A is a schematic view showing a screen example of a user terminal.
FIG. 9B is a schematic view showing a screen example of a user terminal.
FIG. 10A is a schematic view showing a screen example of a user terminal.
FIG. 10B is a schematic view showing a screen example of a user terminal.
FIG. 11 is a flow chart showing an example of a provision processing procedure of an advice.
FIG. 12A is a schematic view showing a screen example of a user terminal.
FIG. 12B is a schematic view showing a screen example of a user terminal.
FIG. 13 is a schematic view showing a configuration example of a learning model.
FIG. 14 is a flow chart showing an example of a registration processing procedure of user information according to an example of the present disclosure.
FIG. 15A is a schematic view showing a screen example of a user terminal.
FIG. 15B is a schematic view showing a screen example of a user terminal.
FIG. 16 is a schematic view showing a configuration example of a score calculation rule DB.
FIG. 17 is a flow chart showing an example of a provision processing procedure of an advice according to an example of the present disclosure.
FIG. 18 is a schematic view showing a screen example of a user terminal.
FIG. 19 is a schematic view showing a configuration example of a member information DB according to an example of the present disclosure.
FIG. 20 is a schematic view showing a configuration example of a classification condition DB.
FIG. 21 is a flow chart showing an example of a user classification processing procedure.
FIG. 22 is a flow chart showing an example of a sharing processing procedure in a class.
FIG. 23A is a schematic view showing a screen example of a user terminal.
FIG. 23B is a schematic view showing a screen example of a user terminal.
FIG. 24 is a flow chart showing an example of a registration processing procedure of user information according to an example of the present disclosure.
FIG. 25A is a schematic view showing a screen example of a user terminal.
FIG. 25B is a schematic view showing a screen example of a user terminal.
FIG. 26 is a flow chart showing an example of a registration processing procedure of user information according to an example of the present disclosure.
FIG. 27 is a schematic view showing a configuration example of a target amount DB.
FIG. 28A is a schematic view showing a registration screen example according to an example of the present disclosure.
FIG. 28B is a schematic view showing an input screen example of target information.
FIG. 29 is a flow chart showing an example of a provision processing procedure of an advice according to an example of the present disclosure.
FIG. 30A is a schematic view showing a screen example of a user terminal.
FIG. 30B is a schematic view showing a screen example of a user terminal.
FIG. 31 is a flow chart showing an example of a provision processing procedure of an advice according to an eighth embodiment.
FIG. 32A is a schematic view showing a screen example of a user terminal.
FIG. 32B is a schematic view showing a screen example of a user terminal.
FIG. 33 is a flow chart showing an example of a provision processing procedure of an advice according to an example of the present disclosure.
FIG. 34A is a schematic view showing a screen example of a user terminal.
FIG. 34B is a schematic view showing a screen example of a user terminal.
FIG. 35 is a flow chart showing an example of a provision processing procedure of an advice according to an example of the present disclosure.
FIG. 36A is a schematic view showing a screen example of a user terminal.
FIG. 36B is a schematic view showing a screen example of a user terminal.
FIG. 37 is a flow chart showing an example of a provision processing procedure of an advice according to an eleventh embodiment.
FIG. 38 is a schematic view showing a screen example of a user terminal.
FIG. 39A is a schematic view showing a setting example of a target value.
FIG. 39B is a schematic view showing a setting example of a target value.

### [Mode for Carrying out Invention]

Hereinafter, a program, an information processing method, and an information processing apparatus according to the present disclosure will be described in detail with reference to drawings illustrating embodiments thereof.

### (First embodiment, which is not encompassed by the wording of the claims but is useful for understanding the invention)

An information processing system which provides a user who purchases food being sold by a subscription system with various kinds of advice will be described. In the present embodiment, a subscription system refers to a sales system that, unlike conventional systems in which a fee is paid every time a product is purchased, enables a user to consume, as much as possible, products within a certain range within an arbitrary period (generally, within a period of a day or more) for a certain price. In such a sales system, for example, an upper limit of an amount of consumption of products during a predetermined period is set in accordance with an amount paid by the user and the user can consume products up to the upper limit of the amount of consumption within the predetermined period. When selling food by such a subscription system, for example, a predetermined amount (the upper limit of the amount of consumption) of food is delivered to the user at predetermined intervals and the user can select and eat whatever he/she wants whenever he/she wants from the delivered food. Although food purchased using a subscription system may become available to the user through any method such as hand-delivery from a sales clerk at a store and delivery to a residence or the like of the user, a method of delivery to the residence or the like of the user to ensure that the food is available whenever he/she wants to eat is preferable and a method of collectively delivering food to the user for a certain period of time is more preferable. Delivering food in bulk lowers the bar for placing food within reach of the user and the user can consume food while being more faithful to his/her own consumption desire. When collecting an amount of consumption of food (amount of ingested food) consumed by a user while being faithful to his/her consumption desire together with a date and time of consumption (date and time of ingestion), consumption information (ingestion information of food) that is faithful to the user's consumption desire can be collected. In addition, with a subscription system, collecting ingestion information in a product lineup also enables non-ingestion information of the food (information related to products which are included in the product lineup but were not consumed by the user despite being in a consumable state or, in other words, "food other than food purchased by the subscription system") to be simultaneously comprehended. Therefore, unless otherwise designated in the present disclosure, in an embodiment using "ingestion information of subscription food (which may also be simply referred to as "ingestion information of food")", "non-ingestion information of subscription food" can also be used and, accordingly, unique information related to food consumed while being faithful to one's desire as will be described later can be obtained. Accordingly, a period during which information is acquired from the user is preferably long since a long period enables more useful consumption information to be obtained and, specifically, the period preferably extends for a week or more and more preferably extends for a month or more. Such consumption information is unique information that cannot be obtained by conventional sales systems involving decision-making with respect to a purchase by the user. In conventional sales systems, since only products purchased after the user determines whether or not to make a purchase become consumption objects, products that the user wants but were not purchased do not become consumption objects. In other words, as circumstances specific to the subscription system, consumption information may include not only product information on products consumed (purchased or ingested) by the user (which may also be simply referred to as "consumption information") but also information related to products which are included in the product lineup but were not consumed by the user despite being in a consumable state (which may also be simply referred to as "non-consumption information"). In the food sector, such consumption information and non-consumption information (in particular, non-consumption information) with respect to subscription food are information not conventionally used, and unique information related to food consumed (or not consumed) by the user while being faithful to his/her desire can be obtained which completely differs from information which is based on food consumed by the user in a desultory manner (for example, everyday menus) and which had conventionally existed and information based on preference surveys (such as questionnaires) which may deviate from reality due to being biased such as carrying unconscious or conscious assumptions. Therefore, by collecting consumption information that considers products sold by the subscription system as consumption objects, consumption information that better reflects a consumption desire of the user than before can be collected. Note that, hereinafter, food that is sold by a subscription system according to the present embodiment will be referred to as "subscription food".

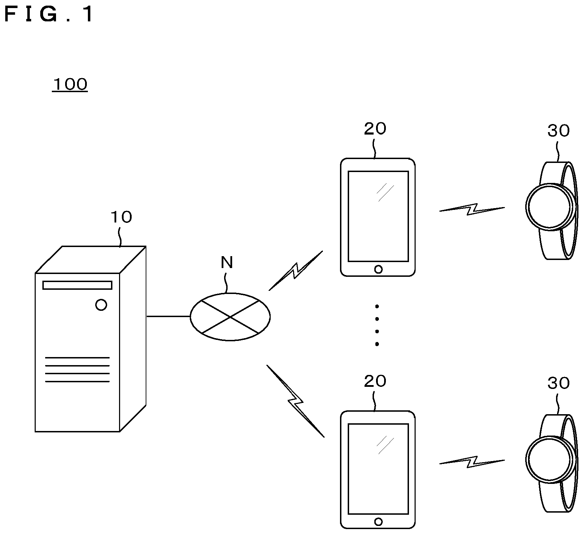

FIG. 1 is a schematic view showing a configuration example of an information processing system. An information processing system 100 according to the present embodiment includes a server 10, a plurality of user terminals 20, and the like, and the server 10 and the user terminals 20 are communicatively connected to each other via a network N such as the Internet. The server 10 is an information processing apparatus capable of performing various kinds of information processing and transmitting and receiving information and is, for example, a server computer, a personal computer, or the like. The server 10 may be provided in plurality, realized by a plurality of virtual machines provided inside a single server apparatus, or realized using a cloud server. Each of the user terminals 20 is a terminal of a user who purchases subscription foods which is an information processing apparatus (computer) such as a smartphone, a tablet terminal, or a personal computer and which may be constituted of a dedicated terminal. In addition, the information processing system 100 according to the present embodiment includes a wearable device 30 to be used by each user, and a corresponding user terminal 20 and wearable device 30 can communicate with each other in a wireless manner. The wearable device 30 is configured to be capable of measuring biological information of the user such as a body temperature, blood pressure, a heart rate, and a pulse, exercise information such as the number of steps, a travel distance, and a travel time, and sleep information such as a sleep time. The user terminal 20 of a communication partner is set to the wearable device 30 in advance and the wearable device 30 transmits various kinds of measured information to the user terminal 20 of the communication partner. The wearable device 30 may be configured as a wristwatch-type wearable device such as that shown in FIG. 1 or configured as an eyeglass-type wearable device, a ring-type wearable device, or the like. Alternatively, instead of including the server 10, the information processing system 100 according to the present embodiment may be constituted of a blockchain (distributed ledger technology or a distributed network) which uses the plurality of user terminals 20 as nodes.

In the information processing system 100 according to the present embodiment, the user terminal 20 performs various kinds of information processing such as processing of receiving various kinds of information related to the user having been input by the user and processing of transmitting the received information to the server 10. The server 10 performs various kinds of information processing such as processing of registering information related to the user having been received from the user terminal 20 and processing of providing each user with advice based on registered information. The server 10 may be equipped with a function of a web server and a food sales site (not illustrated) that accepts a purchase request and the like with respect to subscription foods via the network N may be disclosed on the network N.

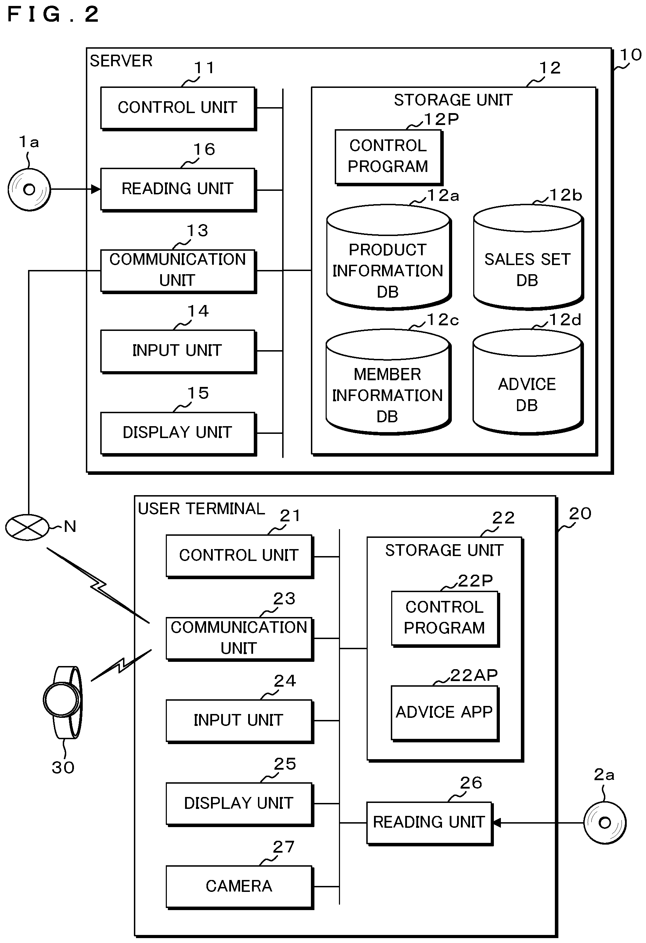

FIG. 2 is a block diagram showing a configuration example of the server 10 and the user terminal 20. The server 10 includes a control unit 11, a storage unit 12, a communication unit 13, an input unit 14, a display unit 15, and a reading unit 16 and the respective units are connected to each other via a bus. The control unit 11 includes one or a plurality of processors such as a CPU (Central Processing Unit), an MPU (Micro-Processing Unit), or a GPU (Graphics Processing Unit). The control unit 11 executes various kinds of information processing, control processing, and the like that the server 10 is expected to perform by appropriately executing a control program 12P that is stored in the storage unit 12.

The storage unit 12 includes a RAM (Random Access Memory), a flash memory, a hard disk, and an SSD (Solid State Drive). The storage unit 12 stores, in advance, the control program 12P to be executed by the control unit 11, various kinds of data necessary for executing the control program 12P, and the like. In addition, the storage unit 12 temporarily stores data and the like that are generated when the control unit 11 executes the control program 12P. Furthermore, the storage unit 12 stores a product information DB (database) 12a, a sales set DB 12b, a member information DB 12c, an advice DB 12d, and the like. The product information DB 12a, the sales set DB 12b, the member information DB 12c, and the advice DB 12d may be stored in another storage apparatus connected to the server 10 or may be stored in another storage apparatus which the server 10 can communicate with.

The communication unit 13 is an interface for connecting to the network N by wired communication or wireless communication and transmits and receives information to and from other apparatuses via the network N. The input unit 14 includes, for example, a mouse and a keyboard, accepts an operation input by a manager who manages the server 10, and sends a control signal corresponding to operation contents to the control unit 11. The display unit 15 is a liquid crystal display, an organic EL display, or the like and displays various kinds of information in accordance with an instruction from the control unit 11. The input unit 14 and the display unit 15 may be an integrally-configured touch panel.

The reading unit 16 reads information stored on a portable storage medium 1a including a CD (Compact Disc)-ROM, a DVD (Digital Versatile Disc)-ROM, a USB (Universal Serial Bus) memory, or an SD (Secure Digital) card. The control program 12P and various kinds of data stored in the storage unit 12 can be read by the control unit 11 from the portable storage medium 1a via the reading unit 16 and stored in the storage unit 12. Alternatively, the control program 12P and various kinds of data stored in the storage unit 12 can be downloaded by the control unit 11 from another apparatus via the communication unit 13 and stored in the storage unit 12.

The user terminal 20 includes a control unit 21, a storage unit 22, a communication unit 23, an input unit 24, a display unit 25, a reading unit 26, and a camera 27 and the respective units are connected to each other via a bus. Since the respective units 21 to 26 of the user terminal 20 are configured in a similar manner to the respective units 11 to 16 of the server 10, detailed descriptions of the configurations will be omitted. In addition to a control program 22P to be executed by the control unit 21, the storage unit 22 of the user terminal 20 stores an advice application program 22AP (hereinafter, referred to as an advice app 22AP) which is a program according to the present disclosure and which is used to perform processing of acquiring, from the server 10, appropriate advice in accordance with various kinds of information (user information) related to the diet, exercise, sleep, and the like of the user. Furthermore, the storage unit 22 may be configured to store various kinds of information (user information) related to the diet, exercise, sleep, and the like of the user having been input via, for example, the input unit 24. In addition to an interface for connecting to the network N, the communication unit 23 of the user terminal 20 includes an interface for communicating with the wearable device 30 in a wireless manner. Alternatively, the communication unit 23 may be configured to communicate with the wearable device 30 by wired communication via a cable.

The camera 27 has a lens, an imaging element, and the like and acquires image data by photoelectrically converting light incident via the lens with the imaging element. The camera 27 performs photography in accordance with an instruction from the control unit 21, sequentially sends acquired image data (photographed images) to the storage unit 22, and causes the storage unit 22 to store the image data (photographed images). Besides a configuration in which the camera 27 is a built-in camera, the user terminal 20 may be configured to include a camera connecting unit to which an external camera can be connected or configured to include a camera communication unit that communicates in a wireless manner to an external camera. In this case, the camera connecting unit or the camera communication unit accepts input of image data acquired by the external camera, sequentially sends the input image data to the storage unit 22, and causes the storage unit 22 to store the image data.

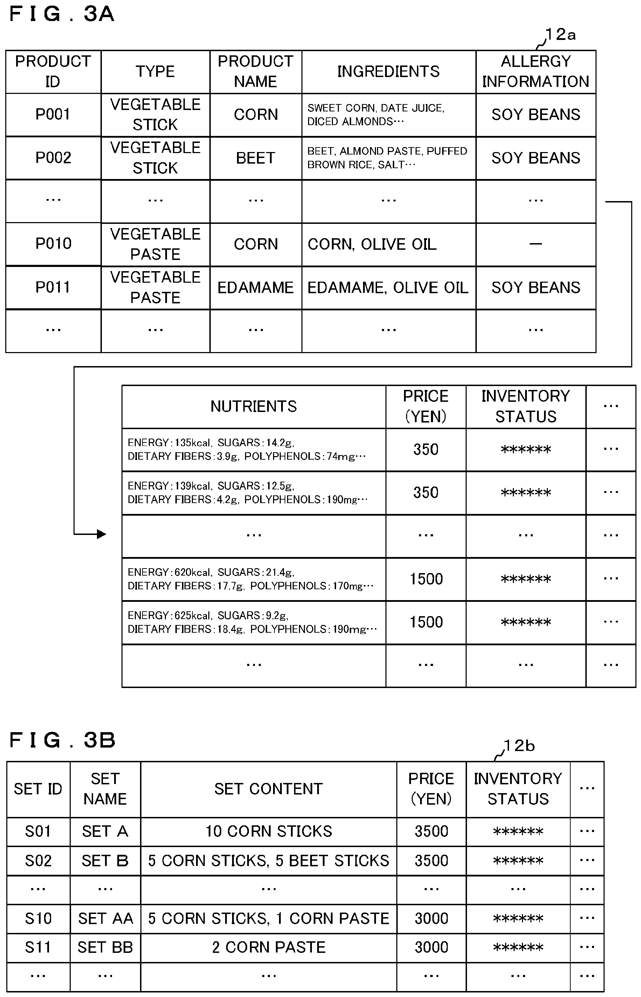

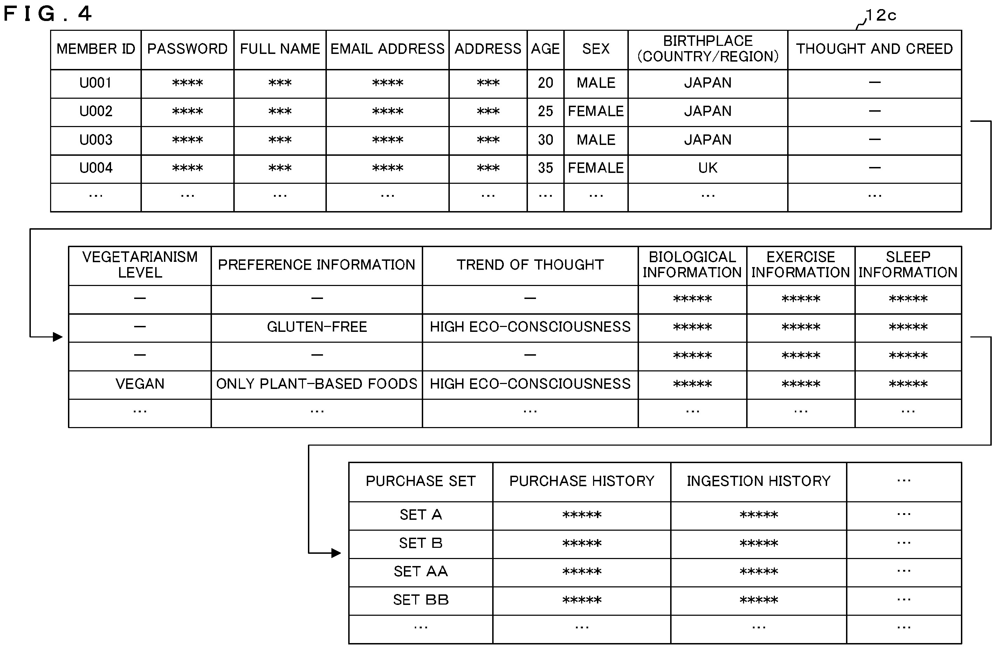

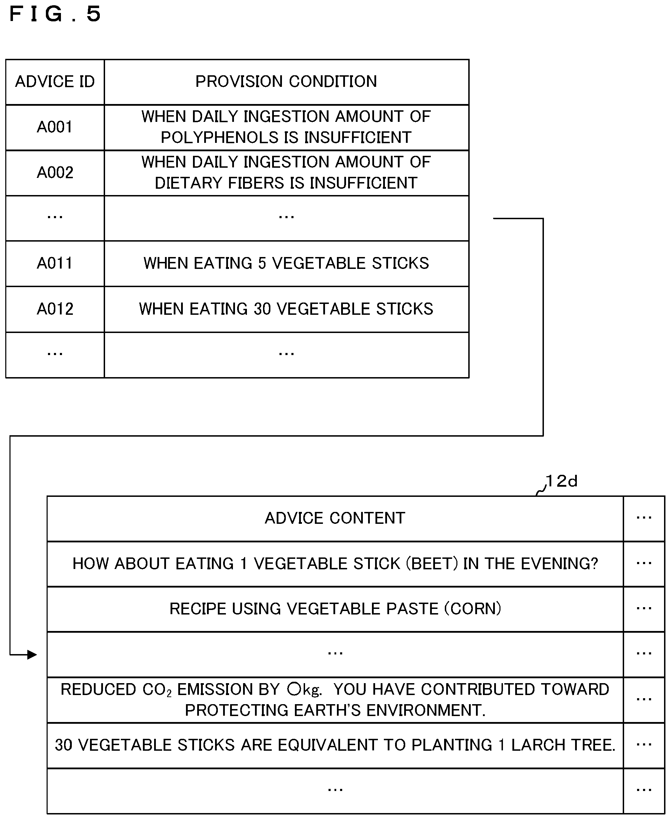

FIGS. 3A to 5 are schematic views showing configuration examples of the DBs 12a to 12d which are stored in the server 10. FIG. 3A shows the product information DB 12a, FIG. 3B shows the sales set DB 12b, FIG. 4 shows the member information DB 12c, and FIG. 5 shows the advice DB 12d. The product information DB 12a stores information related to products (subscription food) being sold by a subscription system. The product information DB 12a shown in FIG. 3A includes a product ID column, a type column, a product name column, an ingredients column, an allergy information column, a nutrient column, a price column, and an inventory status column and stores information on a product (subscription food) in association with the product ID. The product ID column stores identification information (a product ID) assigned to each article of subscription food that is a sales object. The type column stores a type into which products are classified according to forms, ingredients, manufacturing methods, or the like and, for example, a vegetable stick, a vegetable paste, a noodle, a fruit stick, or a fruit paste is stored. The product name column stores a name attached to the product, the ingredients column stores name of ingredients contained in the product, and the allergy information column stores names of allergy-provoking ingredients (ingredients that may possibly provoke allergy) that are contained in the product. The nutrient column stores, in association with each other, energy obtained by ingesting the product, a nutrient name, and an ingestion amount. As for the energy and the ingestion amount of the nutrient, an ingestion amount with respect to a product of a predetermined amount may be used such as using an ingestion amount per stick in the case of a stick-like food and using an ingestion amount per 100 g in the case of a paste-like food. The price column stores a price of the product and the inventory status column stores a quantity of inventory or the like of the product. The product ID stored in the product information DB 12a is issued by the control unit 11 and stored every time information of a new product that is a sale object is registered. Each piece of information representing the type, the product name, the ingredients, allergy information, nutrients, and the price stored in the product information DB 12a is stored by the control unit 11 when, for example, the control unit 11 acquires each piece of information of a new product that is a sale object via the communication unit 13 or the input unit 14. With the inventory status that is stored in the product information DB 12a, the number of manufactured products is added thereto by the control unit 11 when, for example, the control unit 11 accepts the number of manufactured products via the communication unit 13 or the input unit 14, and the number of sold products is subtracted therefrom by the control unit 11 when, for example, the control unit 11 accepts the number of sold products via the communication unit 13 or the input unit 14. The stored contents of the product information DB 12a are not limited to the example shown in FIG. 3A and various kinds of information related to subscription foods that are sales objects can be stored. For example, information and the like related to a storage method, a best-by date, a manufacturing company, and a manufacturing process of subscription foods may be stored in the product information DB 12a.

The sales set DB 12b stores information related to a product set that is a sales object. The sales set DB 12b shown in FIG. 3B includes a set ID column, a set name column, a set content column, a price column, and an inventory status column and stores information related to a product set in association with the set ID. The set ID column stores identification information (a set ID) assigned to each product set that is a sales object, and the set name column stores a name attached to the product set. The set content column stores a product name and the number of each product contained in the product set. The price column stores a price of the product set and the inventory status column stores a quantity of inventory or the like of the product set. The set ID stored in the sales set DB 12b is issued by the control unit 11 and stored every time information of a new product set that is a sale object is registered. Each piece of information representing the set name, the set content, and the price stored in the sales set DB 12b is stored by the control unit 11 when the control unit 11 acquires each piece of information of a new product set that is a sale object via, for example, the communication unit 13 or the input unit 14. With the inventory status that is stored in the sales set DB 12b, the number of manufactured product sets is added thereto by the control unit 11 when, for example, the control unit 11 accepts the number of manufactured product sets via the communication unit 13 or the input unit 14, and the number of sold product sets is subtracted therefrom by the control unit 11 when, for example, the control unit 11 accepts the number of sold product sets via the communication unit 13 or the input unit 14. The stored contents of the sales set DB 12b are not limited to the example shown in FIG. 3B and various kinds of information related to prepared product sets can be stored. As a product set, a set including a plurality of a same product, a set including different products of a same type, a set including products of different types, a trial set that is recommended for a first-time purchaser, a set of products that the seller wishes to introduce, or the like can be used.

The member information DB 12c stores information related to a user having registered as a member in order to get various kinds of advice from the server 10. The member information DB 12c shown in FIG. 4 includes a member ID column, a password column, a full name column, an email address column, an address column, an age column, a sex column, a birthplace column, a thought and creed column, a vegetarianism level column, a preference information column, a trend of thought column, a biological information column, an exercise information column, a sleep information column, a purchase set column, a purchase history column, and an ingestion history column, and stores information on the user who is a member in association with the member ID. The member ID column stores identification information (a member ID) assigned to each user having registered as a member, and the password column stores a password set by the user upon registering as a member or after registering as a member. The full name column, the email address column, the address column, the age column, the sex column, the birthplace column, and the thought and creed column respectively store a full name, an email address, an address, an age, a sex, a birthplace, and a thought and creed (such as a religion) input (designated) by the user upon registering as a member or after registering as a member. A date of birth may be stored instead of an age, a country or a region of birth, a country or a region where the user grew up, or the like can be used as a birthplace, and a religion which the user believes can be used as the thought and creed. The vegetarianism level column, the preference information column, the trend of thought column, the biological information column, the exercise information column, and the sleep information column respectively store a vegetarianism level, preference information, a trend of thought, biological information, exercise information, and sleep information designated by the user upon registering as a member or after registering as a member. The vegetarianism level is information indicating a level of vegetarianism of the user and examples thereof include veganism that refers to strict vegetarianism of refraining from consuming animal-source food, lacto-vegetarianism of consuming dairy products, and ovo-vegetarianism of consuming eggs. The preference information is information indicating a preference (likes and dislikes) of the user toward food, ingredients, and the like, and examples thereof include a preference type that refrains from consuming gluten (gluten-free), a preference type that refrains from consuming animal-source food (only plant-based food), and a preference type that refrains from consuming additives. The trend of thought is information indicating a trend of thought of the user in daily life or in lifestyle habits and examples include a type who is conscious about reducing garbage such as food waste (a high eco-consciousness), a type who wishes to purchase items with high quality even if doing so means paying a higher price, and a type who prioritizes price. The biological information includes various kinds of information related to a physical state of the user and examples thereof include a height, a weight, a body-fat percentage, a body temperature, blood pressure, a heart rate, and a pulse. In addition, the biological information may include a result of a medical checkup undergone by the user, a result of an inspection undergone by the user at a medical institution, and the like. The exercise information is information related to exercise performed by the user and includes, for example, a type (content) of the exercise and an exercise time. The sleep information is information related to sleep of the user and includes, for example, information such as a sleep time, a bedtime (a sleep start time), a wake-up time (a sleep end time), and pulse during sleep. The purchase set column stores a set name of a product set registered to be purchased by the user by a subscription system, and the purchase history column stores purchase information in which information on a product or a product set having been purchased by the user and a purchase date are associated with each other. The ingestion history column stores ingestion information in which information on an eaten product among products purchased by the user and a date and time on which the product had been eaten (ingestion date and time) are associated with each other. Instead of information on the product eaten by the user, the ingestion information may include information on energy and nutrients ingested by the user by eating the product. For example, in addition to nutrient information stored in the product information DB 12a, examples of ingestion information include information obtained from websites or the like provided by a company selling the product, information based on "Standards Tables of Food Composition in Japan 2015 - (Seventh Revised Version)", and information based on "Dietary Reference Intakes for Japanese (2015)".

The member ID stored in the member information DB 12c is issued by the control unit 11 and stored every time information of a user to newly register as a member is registered. Other information stored in the member information DB 12c is stored by the control unit 11 when the control unit 11 acquires each piece of information on the user via, for example, the communication unit 13 or the input unit 14 and changed by the control unit 11 when the control unit 11 acquires a change instruction via the communication unit 13 or the input unit 14. Each of biological information, exercise information, sleep information, a purchase history, and an ingestion history stored in the member information DB 12c is accumulated (additionally stored) in the member information DB 12c by the control unit 11 every time each piece of information is acquired by the control unit 11 from the user terminal 20 via the communication unit 13. The stored contents of the member information DB 12c are not limited to the example shown in FIG. 4 and various kinds of information related to the user having registered as a member can be stored for each user. For example, a family structure of the user, information on food or ingredients that the user is allergic to, hobbies, a direction in daily life, and the like may be stored in the member information DB 12c. In addition, the biological information, the exercise information, the sleep information, the ingestion history, and the like may be stored in a predetermined region of the storage unit 12 or in another storage apparatus in addition to being stored in the member information DB 12c. In such a case, each of the biological information column, the exercise information column, the sleep information column, and the ingestion history column stores information for reading each piece of information (for example, a file name indicating a storage location of data). The member information DB 12c is not limited to a single-DB configuration and may be configured to store each piece of information by dividing the information among a plurality of DBs.

The advice DB 12d stores information related to advice to be provided to the user (the user terminal 20). The advice DB 12d shown in FIG. 5 includes an advice ID column, a provision condition column, and an advice content column, and stores a provision condition of an advice and contents of the advice in association with the advice ID. The advice ID column stores identification information (an advice ID) assigned to each advice. The provision condition column stores a provision condition under which an advice is to be provided and stores, for example, a condition related to an excess or a deficiency of nutrients, exercise, sleep, or the like, a condition related to a physical state, and a condition related to a timing (a season, a time slot, or the like) of providing the advice. Accordingly, different advice contents can be registered in the advice DB 12d in accordance with the daily life of the user including eating habits and a physical state of the user or in accordance with a timing of providing advices. The advice content column stores an advice message to be proposed to the user with respect to lifestyle habits such as diet, exercise, and sleep, a message to be notified to the user with respect to a contribution that can be made to the earth's environment by eating subscription food, and the like. Advice related to food includes, for example, advice on an ingestion amount of various nutrients such as dietary fibers and proteins, advice on an ingestion amount of food with a low GI value (glycemic index), and advice for proposing a bad combination of food to be avoided, an eating order, meal times, or the like in order to suppress the GI value. Advice related to exercise includes an advice for proposing an exercise type and an exercise time and a message that generates the user's motivation. Advice related to sleep includes advice for proposing a sleep time, a bedtime, a wake-up time, an action prior to sleep, an action after waking up, and the like. In addition, messages related to a contribution toward the earth's environment include a message related to the earth's environment that can be protected in accordance with an ingestion amount (eaten amount) of subscription food such as a message notifying an amount of reduction of emission of CO₂ that can be reduced in accordance with an ingestion amount of subscription food. Note that advice messages include various kinds of advice related to lifestyle habits including diet, exercise, sleep, and the like by experts such as physicians, nurses, pharmacists, nutritionists, athletic trainers, and researchers (including advice related to an effect anticipated with an improvement in lifestyle habits such as psychosomatic (stress, work efficiency, and bodily functions) advice. More specifically, advice related to lifestyle habits including diet includes advice related to an improvement in an intestinal environment, stress reduction, an improvement in work efficiency, an improvement in bodily functions, or an improvement in cognitive functions which is anticipated with an improvement in lifestyle habits. The advice ID stored in the advice DB 12d is issued by the control unit 11 and stored every time a new advice is registered. Other information stored in the advice DB 12d is stored by the control unit 11 when the control unit 11 acquires each piece of information of a new advice via, for example, the communication unit 13 or the input unit 14. The stored contents of the advice DB 12d are not limited to the example shown in FIG. 5 and advice messages with various contents may be stored, and various kinds of information related to advices can also be stored.

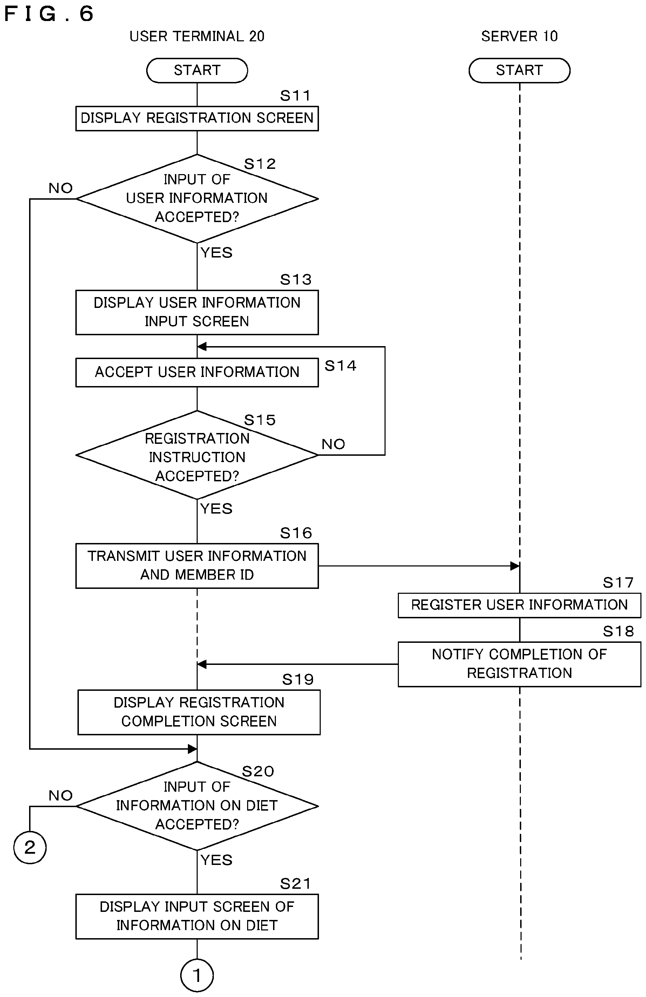

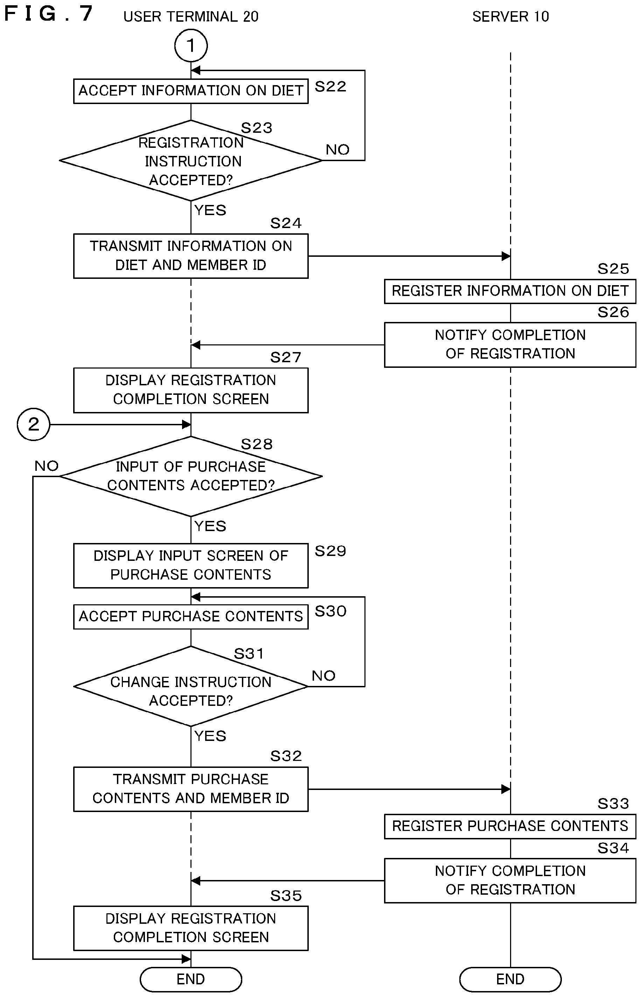

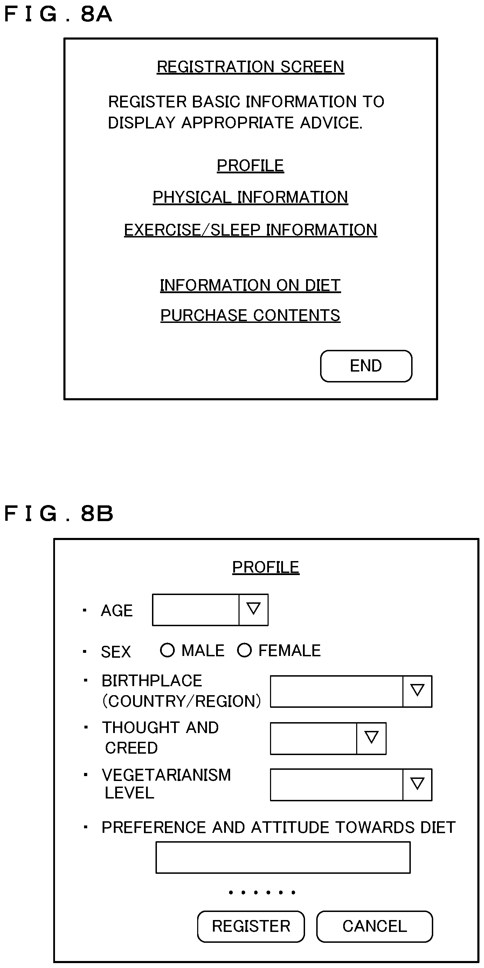

Hereinafter, processing performed by each apparatus when the user registers information (user information) related to the user in the server 10 using the user terminal 20 in the information processing system 100 according to the present embodiment will be described. FIGS. 6 and 7 are flow charts showing an example of a registration processing procedure of user information, and FIGS. 8A to 10B are schematic views showing screen examples of the user terminal 20. In FIGS. 6 and 7, processing performed by the user terminal 20 is shown on a left-hand side and processing performed by the server 10 is shown on a right-hand side. The processing described below is executed by the control unit 21 in accordance with the control program 22P and the advice app 22AP stored in the storage unit 22 of the user terminal 20 and executed by the control unit 11 in accordance with the control program 12P stored in the storage unit 12 of the server 10. A part of the processing described below may be realized by an exclusive hardware circuit. In the processing described below, it is assumed that the user is a user having registered as a member in advance in order to get advice from the server 10 and has performed login processing with respect to the server 10 using the user's own member ID and password. Upon registration by the user as a member, the control unit 11 (registering unit) of the server 10 acquires personal information necessary for member registration such as a full name, a password, an email address, and an address of the user from the user terminal 20 and registers each piece of acquired information in the member information DB 12c. In doing so, the control unit 11 issues a member ID and stores each piece of information in association with the member ID.

In the information processing system 100 according to the present embodiment, when the user wishes to register or change his/her own information (user information), the user inputs the user information using the user terminal 20 to transmit the user information to the server 10 and causes the server 10 to register the user information. The user causes an input screen (registration screen) of user information to be displayed on the user terminal 20 by having the user terminal 20 open the advice app 22AP and inputs user information via the input screen. In addition, when the user terminal 20 stores a web browser for viewing websites via the network N in the storage unit 22, the user may access the server 10 by having the user terminal 20 open the browser and acquire an input screen (registration screen) of user information from the server 10.

When the control unit 21 of the user terminal 20 accepts a start instruction of the advice app 22AP from the user via the input unit 24, the control unit 21 opens the advice app 22AP. Subsequently, when the control unit 21 accepts an instruction to register or change user information via the input unit 24, the control unit 21 displays a registration screen such as that shown in FIG. 8A on the display unit 25 (S11). FIG. 8A shows a first screen example of an input screen (registration screen) for inputting user information and the screen shown in FIG. 8A is a selection screen that accepts a selection of a type of user information to be input (registered). The screen shown in FIG. 8A is configured such that any of a profile, physical information, information on exercise/sleep, information on diet, and purchase contents of the user can be selected as user information to be input (registered). When any of the profile, the physical information, the information on exercise/sleep, the information on diet, and the purchase contents is selected on the screen shown in FIG. 8A via the input unit 24, the control unit 21 accepts an input instruction of the selected user information.

First, the control unit 21 determines whether or not an input instruction with respect to any piece of user information among the profile, the physical information, and the information on exercise/sleep has been accepted on the screen shown in FIG. 8A (S12). When the control unit 21 determines that an input instruction with respect to any of the pieces of user information has been accepted (S12: YES), the control unit 21 displays an input screen of the selected user information on the display unit 25 (S13). When the profile is selected on the screen shown in FIG. 8A, the control unit 21 displays an input screen of a profile such as that shown in FIG. 8B. The input screen shown in FIG. 8B has input fields for respectively inputting pieces of profile information including attribute information including an age and sex of the user, a birthplace of the user, thought and creed, a vegetarianism level, preference (palate) with respect to diet (food and ingredients), and principles and attitudes (thought). The "age" input field is provided with a pull-down menu that enables any one age or any one age group to be selected from predetermined ages or predetermined age groups, and any age or any age group can be input using the pull-down menu. The "sex" input field is provided with a radio button that enables male or female to be selected and sex can be input using the radio button. The "birthplace" input field is provided with a pull-down menu that enables any one birthplace (a country or a region) to be selected from predetermined birthplaces (countries or regions), and any birthplace can be input using the pull-down menu. The "thought and creed" input field is provided with a pull-down menu that enables any one thought and creed (for example, a religion) to be selected from predetermined thoughts and creeds, and any thought and creed can be input using the pull-down menu. The "vegetarianism level" input field is provided with a pull-down menu that enables any one vegetarianism level to be selected from predetermined information (veganism, lacto-vegetarianism, and the like) indicating a vegetarianism level, and any vegetarianism level can be input using the pull-down menu. Any comment can be input to the input field of "preference and attitude toward diet" via the input unit 24. Alternatively, the control unit 21 may perform processing of extracting the user's preference and attitude toward diet from a viewing history of websites (for example, recipe sites) that the user has viewed using the user terminal 20 via the network N. In this case, information extracted by the control unit 21 from the viewing history is input to the input field of "preference and attitude toward diet". The profile input screen is not limited to the configuration shown in FIG. 8B and each input field may be configured so that any information can be input via the input unit 24 or provided with a pull-down menu to enable any one option to be selected from predetermined options.

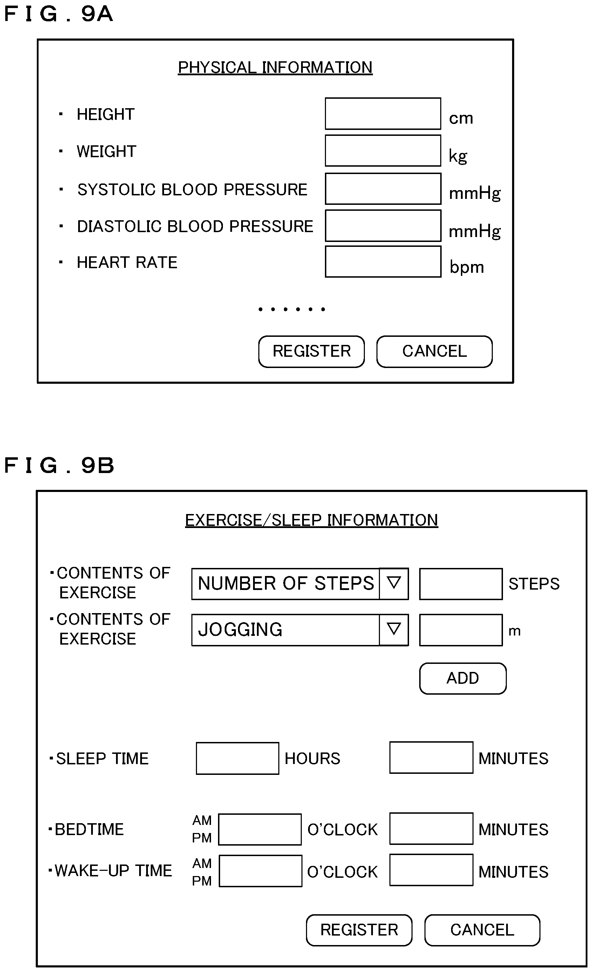

In addition, when the physical information is selected on the screen shown in FIG. 8A, the control unit 21 displays an input screen of physical information such as that shown in FIG. 9A. The input screen shown in FIG. 9A has input fields for inputting respective pieces of biological information (information related to a physical state) of the user including a height, a weight, blood pressure (systolic blood pressure and diastolic blood pressure), a heart rate, a body-fat percentage, a pulse, a body temperature, a frequency of bowel movement, and a blood glucose level. While any numerical value can be input via the input unit 24 to the input field of each piece of biological information, each input field may be provided with a pull-down menu that enables any one option to be selected from predetermined options. In addition, the user terminal 20 can acquire, from the wearable device 30, a measured value of biological information that is measurable by the wearable device 30, and measured data of biological information acquired from the wearable device 30 is input to each input field of biological information on the input screen shown in FIG. 9A. Specifically, measured values of a body temperature, blood pressure, a heart rate, and a pulse that can be measured by the wearable device 30 are input to each input field. The user terminal 20 may be configured to acquire a measured value by a measuring instrument other than the wearable device 30 and, in this case, a measured value acquired by the measuring instrument is displayed in each corresponding input field. As the measuring instrument, a stadiometer that measures a height, a scale that measures a weight and a body-fat percentage, a pulse monitor that measures a pulse, a blood sugar measuring instrument that measures a blood glucose level, and the like can be used. The input screen of physical information is not limited to the configuration shown in FIG. 9A and may have input fields for types and amounts of drugs currently being taken, past medical history (history of illness), frequency and amount of smoking and drinking, test results of various tests including a blood test and a urinalysis performed at a medical institution or the like, test results of various tests performed during a medical checkup or health screening, and the like. A configuration may be adopted in which data of a test result is acquired by taking a photograph of a sheet of paper describing the test result with the camera 27. In this case, the user terminal 20 photographs the sheet of paper describing the test result with the camera 27 and reads the test result by generating text data from the obtained photographed image by OCR (Optical Character Recognition). In addition, the user terminal 20 can acquire biological information of the user by extracting various kinds of data from the read test result. Alternatively, a configuration may be adopted in which processing of generating text data by OCR from a photographed image obtained by photographing a sheet of paper describing the test result is performed by an apparatus (such as the server 10) other than the user terminal 20.

In addition, when the information on exercise/sleep is selected on the screen shown in FIG. 8A, the control unit 21 displays an input screen of information on exercise/sleep such as that shown in FIG. 9B. The input screen shown in FIG. 9B has an input field for inputting contents of exercise (exercise information) including a type of exercise and an amount of exercise or exercise time and an input field for inputting sleep information including a sleep time, a bedtime, and a wake-up time. The "exercise type" input field is provided with a pull-down menu that enables any type to be selected from predetermined types, and any exercise type can be input using the pull-down menu. Any numerical value can be input to the input field of "exercise amount" or "exercise time" via the input unit 24. In addition, the user terminal 20 can acquire, from the wearable device 30, a measured value related to exercise information that is measurable by the wearable device 30, and a measured value related to exercise information acquired from the wearable device 30 is input to each input field on the input screen shown in FIG. 9B. Furthermore, the screen shown in FIG. 9B is provided with an add button for adding an input field of exercise information and, when the add button is operated, a new input field of exercise contents is displayed. While any numerical value can be input via the input unit 24 to each input field of sleep information, each input field may be provided with a pull-down menu that enables any one numerical value to be selected from predetermined numerical values. In addition, a measured value related to sleep information acquired by the user terminal 20 from the wearable device 30 may also be input to each input field of sleep information. The input screen of information on exercise/sleep is not limited to the configuration shown in FIG. 9B and, for example, an input field to which any comment can be input via the input unit 24 may be provided as exercise information and sleep information. Furthermore, the input screen of information on exercise/sleep may be configured so that, in addition to information on exercise and sleep, information (action information) related to various actions by the user in daily life is input.

The user inputs each piece of information that can be input to each input field on input screens such as those shown in FIGS. 8B to 9B. The control unit 21 of the user terminal 20 accepts, via the input unit 24, each piece of information (user information) input by the user (S14), and displays each piece of accepted information in a corresponding input field. The user terminal 20 may store previously-input information in the storage unit 22 and, in this case, when displaying input screens such as those shown in FIGS. 8B to 9B on the display unit 25, the control unit 21 displays information already input in respectively corresponding input fields. On the input screen displaying already-input information, each piece of information displayed in the input fields can be modified via the input unit 24 and the user can not only input information in each input field but can also appropriately modify information that has already been input. In addition, with respect to information that can be acquired from a measuring instrument on input screens such as those shown in FIGS. 8B to 9B, the control unit 21 acquires each piece of information from a measuring instrument regularly or when displaying the input screens on the display unit 25 and displays each piece of acquired information in respectively corresponding input fields.

The input screens shown in FIGS. 8B to 9B include a register button for instructing execution of processing of registering each piece of input information (user information) in the server 10 and a cancel button for instructing termination (stoppage) of the registration processing. The control unit 21 determines whether or not an execution instruction (registration instruction) for registration processing of user information has been accepted in accordance with whether or not the register button has been operated via the input unit 24 (S15), and when the control unit 21 determines that a registration instruction has not been accepted (S15: NO), the control unit 21 repeats the processing of step S14. When the control unit 21 determines that a registration instruction has been accepted (S15: YES), the control unit 21 associates each piece of information (user information) input via an input screen and the member ID of the user with each other and transmits the associated information to the server 10 (S16), and instructs the server 10 to register the user information. Specifically, when profile information (attribute information, preference information, or a trend of thought of the user) is input via the input screen shown in FIG. 8B, the profile information and the member ID are transmitted to the server 10. In addition, when physical information is input via the input screen shown in FIG. 9A, the physical information (biological information) and the member ID are transmitted to the server 10, and when information on exercise/sleep is input via the input screen shown in FIG. 9B, the information on exercise/sleep and the member ID are transmitted to the server 10. The member ID of the user is set in, for example, the advice app 22AP.

The control unit 11 of the server 10 acquires the user information transmitted by the user terminal 20 and registers the acquired user information in the member information DB 12c (S17). Specifically, the control unit 11 acquires the member ID and the user information from the user terminal 20 and stores, in association with the acquired member ID, each piece of information of the acquired user information in the member information DB 12c. With information already stored in the member information DB 12c, each piece of information newly acquired from the user terminal 20 may be added and stored or may be stored by overwriting the stored information. In addition, each piece of information included in user information may be stored in association with a current date and time (date and time of update). When each piece of information acquired from the user terminal 20 is sequentially added and stored, the server 10 can acquire user information of a time series from the user terminal 20 and accumulate time-series user information. Furthermore, when each piece of information is to be stored by overwriting stored information, the server 10 can hold latest user information.

After storing user information acquired from the user terminal 20 in the member information DB 12c, the control unit 11 notifies the user terminal 20 of completion of registration of the user information (S18). When notified of completion of registration by the server 10, the control unit 21 of the user terminal 20 displays a screen showing completion of registration of the user information on the display unit 25 (S19). Instead of displaying a registration completion screen of user information, the control unit 21 may return display to an initial screen of the input screen (registration screen) by causing the display unit 25 to display the screen shown in FIG. 8A. In step S12, when the control unit 21 determines that an input instruction with respect to any piece of user information is not accepted on the screen shown in FIG. 8A (S12: NO), the control unit 21 skips processing of steps S13 to S19.

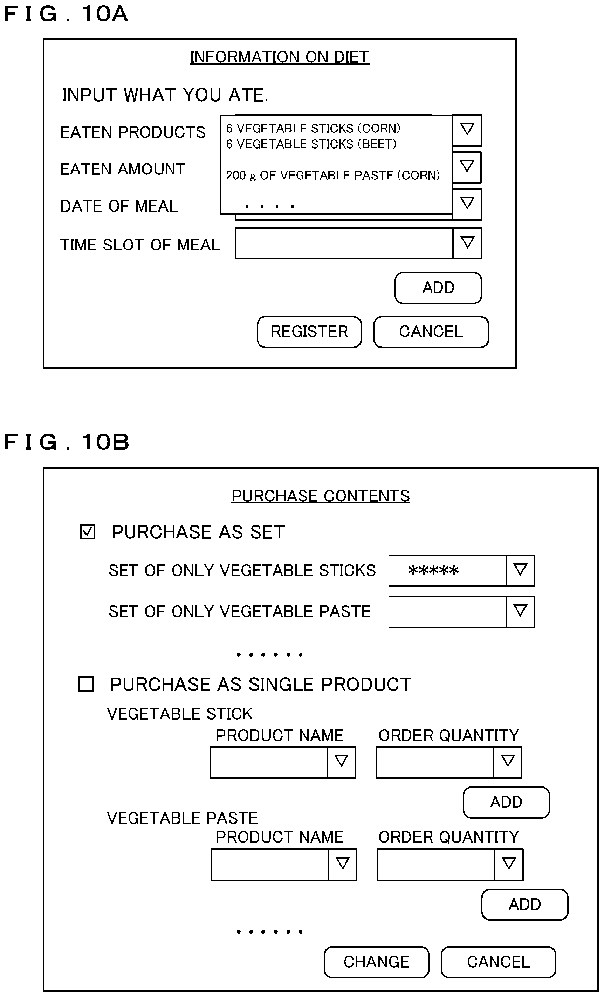

Next, the control unit 21 determines whether or not an input instruction with respect to information on diet (user information) has been accepted on the screen shown in FIG. 8A (S20). When the control unit 21 determines that an input instruction with respect to information on diet has been accepted (S20: YES), the control unit 21 displays an input screen of information on diet such as that shown in FIG. 10A on the display unit 25 (S21). The input screen of the information on diet shown in FIG. 10A has an input field for inputting information (user ingestion information) of articles of subscription food eaten (ingested) by the user among the articles of subscription food purchased by the user. Specifically, the input screen of the information on diet has input fields for inputting a product name of a product (subscription food) eaten by the user, an eaten amount (an ingestion amount), a date on which the product had been eaten, and a time slot during which the product had been eaten. The input field for a product name of the eaten subscription food is provided with a pull-down menu that enables any product or article of subscription food to be selected from products that are sales objects or articles of subscription food purchased by the user, and any product name can be input using the pull-down menu. For example, when a list of product names of articles of subscription food that are sales objects or a list of product names of articles of subscription food purchased by the user is stored in the storage unit 22, the control unit 21 can read the list of product names from the storage unit 22 and provide the product name input field with a pull-down menu displaying the read list of product names. When a list of product names such as that described above is not stored in the storage unit 22, the control unit 21 may acquire the list of product names described above from the server 10 and provide the product name input field with a pull-down menu displaying the acquired list of product names. In addition, as shown in FIG. 10A, a list of product names of articles of subscription food having been purchased but not yet eaten by the user may be displayed on the pull-down menu provided in the product name input field. In this case, when a purchase history and an ingestion history of subscription food by the user are stored in the storage unit 22, the control unit 21 may specify articles of unconsumed (not-yet-eaten) subscription food based on the purchase history and the ingestion history, generate a list of product names of unconsumed subscription food, and display the list on a pull-down menu. At this point, the control unit 21 may specify a remaining amount of articles of unconsumed subscription food based on the purchase history and the ingestion history and display a remaining amount of each article of subscription food together with a list of product names of unconsumed subscription food on a pull-down menu. In addition, when a purchase history and an ingestion history of subscription food by the user are not stored in the storage unit 22, the control unit 21 may acquire the purchase history and the ingestion history from the member information DB 12c of the server 10, generate a list of product names of unconsumed subscription food based on the acquired purchase history and ingestion history, and display the list on a pull-down menu. Alternatively, the control unit 11 of the server 10 may generate a list of product names of unconsumed subscription food based on the purchase history and the ingestion history stored in the member information DB 12c and specify a remaining amount of each article of subscription food. In this case, the control unit 21 may display the list of product names and the remaining amount of each article of subscription food acquired from the server 10 on a pull-down menu. Product names of subscription food already eaten by the user or subscription food not purchased by the user may be displayed on a pull-down menu so as to be unselectable or may not be displayed on a pull-down menu. According to the configuration described above, the user can readily select a product eaten by himself/herself and input operations are simplified. In addition, when a remaining amount of each article of subscription food is displayed as shown in FIG. 10A, the user can also readily comprehend the remaining amount of subscription food.

The "eaten amount" input field is provided with a pull-down menu that enables any numerical value to be selected from predetermined numerical values, and any numerical value can be input using the pull-down menu. When an article of food with a paste-like form is input as eaten food, the pull-down menu is configured so that grams (weight) can be selected, and when an article of food with a stick shape is input, the pull-down menu is configured so that the number of sticks can be selected. The input fields of the date on which a product had been eaten and the time slot during which the product had been eaten (ingestion timing) are respectively provided with a pull-down menu that enables any date and any time slot to be selected from a plurality of dates and a plurality of time slots, and any date and any time slot can be input using the pull-down menus. The input screen for information on diet is not limited to the configuration shown in FIG. 10A and each input field may be configured so that any information can be input via the input unit 24.

The user inputs each piece of information of a product name of an article of subscription food that the user had eaten, an amount eaten by the user, and a date and time (date, time slot) at which the user had eaten the article of subscription food to each input field on an input screen such as that shown in FIG. 10A. The control unit 21 of the user terminal 20 accepts, via the input unit 24, each piece of information (information on diet) input by the user (S22), and displays each piece of accepted information in a corresponding input field. The input screen shown in FIG. 10A includes a register button for instructing execution of processing of registering each piece of input information (information on diet) in the server 10 and a cancel button for instructing termination (stoppage) of the registration processing. The control unit 21 determines whether or not an execution instruction (registration instruction) for registration processing of information on diet has been accepted in accordance with whether or not the register button has been operated via the input unit 24 (S23), and when the control unit 21 determines that a registration instruction has not been accepted (S23: NO), the control unit 21 repeats the processing of step S22. When the control unit 21 determines that a registration instruction has been accepted (S23: YES), the control unit 21 associates each piece of information (information on diet, user ingestion information) input via an input screen and the member ID of the user with each other and transmits the associated information to the server 10 (S24), and instructs the server 10 to register the information on diet (user information).

The control unit 11 (acquiring unit) of the server 10 acquires the information on diet (user ingestion information) transmitted by the user terminal 20 and registers the acquired information on diet in the member information DB 12c (S25). Specifically, the control unit 11 acquires the member ID and the information on diet from the user terminal 20 and stores the acquired information on diet in an ingestion history stored in the member information DB 12c in association with the acquired member ID. In this case, the control unit 11 causes the information on diet acquired from the user terminal 20 to be stored by being added to the ingestion history already stored in the member information DB 12c. After storing the information on diet acquired from the user terminal 20 in the member information DB 12c, the control unit 11 notifies the user terminal 20 of completion of registration of the information on diet (S26). When notified of completion of registration by the server 10, the control unit 21 of the user terminal 20 displays a screen showing completion of registration of the information on diet on the display unit 25 (S27). Even in this case, instead of displaying a registration completion screen of information on diet, the control unit 21 may return display to an initial screen of the registration screen by causing the display unit 25 to display the screen shown in FIG. 8A. In step S20, when the control unit 21 determines that an input instruction with respect to information on diet is not accepted on the screen shown in FIG. 8A (S20: NO), the control unit 21 skips processing of steps S21 to S27.

Furthermore, the control unit 21 determines whether or not an input instruction (change instruction) with respect to purchase contents has been accepted on the screen shown in FIG. 8A (S28). When the control unit 21 determines that an input instruction with respect to purchase contents has been accepted (S28: YES), the control unit 21 displays an input screen of purchase contents such as that shown in FIG. 10B on the display unit 25 (S29). The input screen of purchase contents shown in FIG. 10B has an input field for inputting purchase contents of articles of subscription food registered by the user. Specifically, the input screen of purchase contents has an input field for selecting purchase of a product set (purchase as a set) prepared by a vendor in advance and an input field to be used by the user to select any product (purchase as a single item). The input field for selecting a product set is provided with a pull-down menu that enables any one product set to be selected from predetermined product sets, and any product set can be input using the pull-down menu. The input field for selecting a single product has input fields for inputting a product type (a product name) and an order quantity. The input field of the product type is provided with a pull-down menu that enables any one product to be selected from products that are sales objects, and the input field of the order quantity is provided with a pull-down menu that enables any numerical value to be selected from a plurality of numerical values. In addition, the screen shown in FIG. 10B is provided with an add button for adding an input field of a single product and, when the add button is operated, a new input field is displayed. When displayed, the input screen of purchase contents displays purchase contents that have already been registered and the displayed purchase contents can be changed (modified) via the input unit 24. The input screen of purchase contents is not limited to the configuration shown in FIG. 10B and each input field may be configured so that any information can be input via the input unit 24.

When the user wishes to change a product set or a product to be purchased, the user inputs or changes each piece of information displayed in each input field to purchase contents that the user wishes to change on an input screen such as that shown in FIG. 10B. The control unit 21 of the user terminal 20 accepts, via the input unit 24, purchase contents after the change input by the user (S30), and displays accepted purchase contents in each corresponding input field. The input screen shown in FIG. 10B includes a change button for instructing the server 10 to make a change to input purchase contents and a cancel button for instructing termination (stoppage) of the change processing. The control unit 21 determines whether or not an execution instruction (change instruction) for change processing of purchase contents has been accepted in accordance with whether or not the change button has been operated via the input unit 24 (S31), and when the control unit 21 determines that a change instruction has not been accepted (S31: NO), the control unit 21 repeats the processing of step S30. When the control unit 21 determines that a change instruction has been accepted (S31: YES), the control unit 21 associates purchase contents after the change having been input via an input screen and the member ID of the user with each other and transmits the associated information to the server 10 (S32), and instructs the server 10 to change the purchase contents.

The control unit 11 of the server 10 acquires the purchase contents transmitted by the user terminal 20 and registers the acquired purchase contents in the member information DB 12c (S33). Specifically, the control unit 11 acquires the member ID and the purchase contents from the user terminal 20 and stores the acquired purchase contents in a purchased set stored in the member information DB 12c in association with the acquired member ID. In this case, the control unit 11 may store the purchase contents newly acquired from the user terminal 20 by overwriting the purchased set already stored in the member information DB 12c with the newly acquired purchase contents. After storing the purchase contents acquired from the user terminal 20 in the member information DB 12c, the control unit 11 notifies the user terminal 20 of completion of registration of the purchase contents (S34). When notified of completion of registration by the server 10, the control unit 21 of the user terminal 20 displays a screen showing completion of registration (change) of the purchase contents on the display unit 25 (S35). Even in this case, instead of displaying a registration completion screen of purchase contents, the control unit 21 may return display to an initial screen of the registration screen by causing the display unit 25 to display the screen shown in FIG. 8A. In step S28, when the control unit 21 determines that an input instruction with respect to purchase contents is not accepted on the screen shown in FIG. 8A (S28: NO), the control unit 21 skips processing of steps S29 to S35 and ends processing.

Due to the processing described above, in the information processing system 100 according to the present embodiment, user information such as attribute information, profile information, information related to a physical state (biological information), information related to exercise, and information related to sleep of the user, a type, an amount (ingestion amount), and an ingestion timing of articles of food eaten by the user, and the like are input using the user terminal 20 and registered in the server 10. It should be noted that user information includes a vegetarianism level of the user, information (preference information) related to preferences and principles of the user with respect to diet, a trend of thought of the user in daily life (what the user is conscious of), and the like. In addition, the user information may include information (action information) related to lifestyle habits (actions that have become habits) in daily life including exercise and sleep and information on bodily functions (pain, numbness, a range of joint motion, flexibility of body, posture, muscle strength, capacity of balance, locomotive function, and oral function) in daily life. Moreover, the user information may include comments sent by the user with respect to a change in a physical condition or an emotional change caused by eating subscription food, comments with respect to stress in daily life, comments with respect to work efficiency of studying, working, or the like, comments with respect to cognition such as memory or forgetfulness, or information (shared information) with respect to subscription food, recipes of dishes using subscription food, or the like which the user wishes to recommend to other users, and the like. Furthermore, due to the processing described above, in the information processing system 100 according to the present embodiment, purchase contents (order contents) of products that the user wishes to purchase can be changed using the user terminal 20. In addition to information related to an ingestion amount and an ingestion timing of food ingested by the user with respect to subscription food, combining the input information (in particular, action information and/or biological information) described earlier enables more appropriate advice (such as advice in accordance with a present or future disease risk or advice in accordance with a score based on various criteria to be described later) to be provided to the user. Furthermore, when subscription food is an article of food containing an effective amount or more of any nutrient to be described later or an article of food containing a nutrient and a useful component, advice can be more preferably be given based on health functionality that can be expected by the nutrient, by the useful component, or by both the nutrient and the useful component.

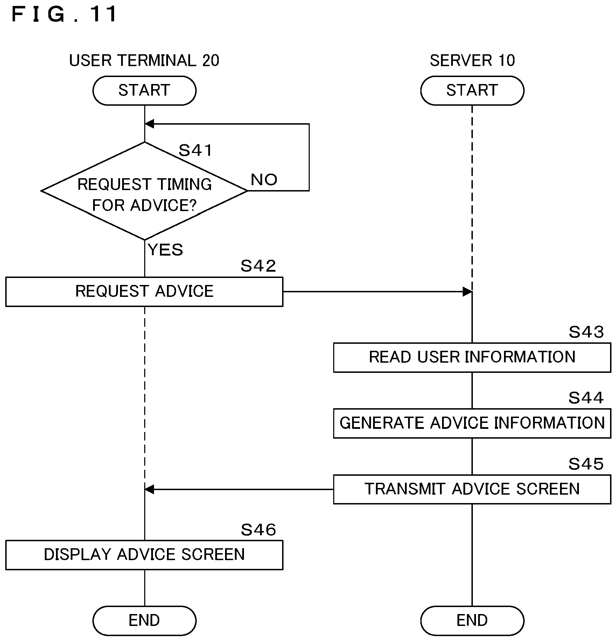

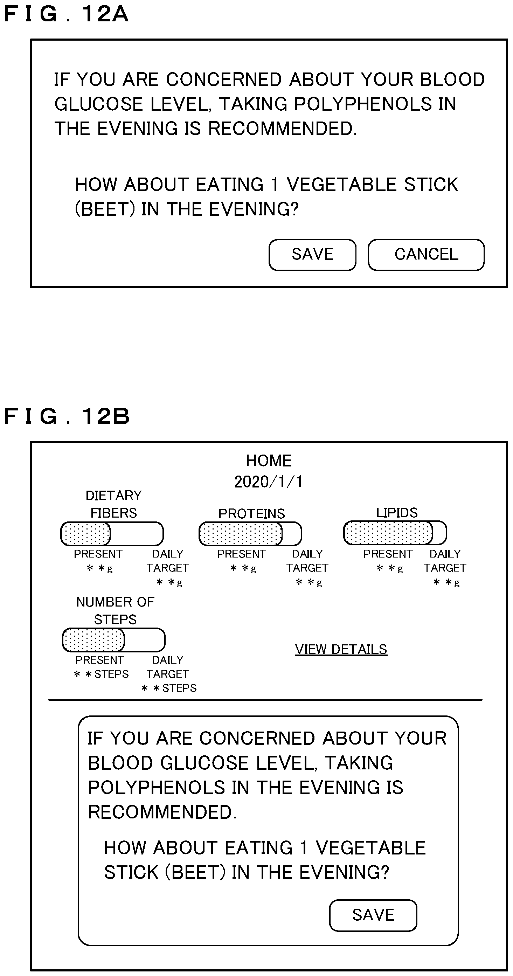

As described above, the server 10 sequentially registers various kinds of information with respect to the user and provides the user with appropriate advice in accordance with the registered information. Next, processing performed by each apparatus when the server 10 provides advice in accordance with user information of each user in the information processing system 100 will be described. FIG. 11 is a flow chart showing an example of a provision processing procedure of an advice, and FIGS. 12A and 12B are schematic views showing screen examples of the user terminal 20. In FIG. 11, processing performed by the user terminal 20 is shown on a left-hand side and processing performed by the server 10 is shown on a right-hand side. The processing described below is executed by the control unit 21 in accordance with the control program 22P and the advice app 22AP stored in the storage unit 22 of the user terminal 20 and executed by the control unit 11 in accordance with the control program 12P stored in the storage unit 12 of the server 10. A part of the processing described below may be realized by an exclusive hardware circuit.

In the information processing system 100, when the user wishes to receive advice from the server 10, the user causes the user terminal 20 to open the advice app 22AP and makes a request for advice to the server 10 via the advice app 22AP. The user can cause a home screen (start screen) to be displayed on the user terminal 20 by having the user terminal 20 open the advice app 22AP and can make a request for advice via the home screen. When the control unit 21 of the user terminal 20 accepts a start instruction of the advice app 22AP from the user via the input unit 24, the control unit 21 opens the advice app 22AP and displays the home screen on the display unit 25. Subsequently, when the control unit 21 accepts a request instruction of an advice via the input unit 24, the control unit 21 makes a request for advice to the server 10. Alternatively, the control unit 21 may make a request for advice to the server 10 once the advice app 22AP is started without accepting a request instruction from the user. In this case, the user need only perform an operation of starting the advice app 22AP and need not perform an operation with respect to a request instruction of an advice. In addition, after starting the advice app 22AP (in other words, when the advice app 22AP is running), the control unit 21 may make a request for advice to the server 10 on a regular basis or every time a time of day set in advance arrives.

When the control unit 21 of the user terminal 20 starts the advice app 22AP, the control unit 21 determines whether or not a timing of requesting the server 10 for advice has arrived (S41). For example, when the control unit 21 accepts a request instruction for an advice via the home screen, the control unit 21 determines that a request timing for an advice has arrived. In addition, the control unit 21 may determine that a request timing for an advice has arrived when performing start processing of the advice app 22AP or determine that a request timing for an advice has arrived when a predetermined period of time elapses or when a time of day set in advance arrives. When the control unit 21 determines that a request timing for an advice has not arrived (S41: NO), the control unit 21 stands by while performing other kinds of processing.

When the control unit 21 determines that a request timing for an advice has arrived (S41: YES), the control unit 21 makes a request for an advice to the server 10 (S42). Specifically, the control unit 21 transmits the member ID of the user and a request signal for requesting an advice to the server 10. When the control unit 11 of the server 10 accepts a request for an advice from the user terminal 20, the control unit 11 reads user information stored in the member information DB 12c in association with the member ID received from the user terminal 20 (S43). In addition, the control unit 11 (information processing unit) generates appropriate advice information with respect to the read user information based on stored contents of the advice DB 12d (S44). The control unit 11 generates advice information to be provided in consideration of at least a part of the user information stored in the member information DB 12c, a time slot (time of day) in which the request for an advice had been made, and the like. For example, based on ingestion history of the user read from the member information DB 12c, the control unit 11 may compare an ingestion amount of a nutrient ingested by the user with a recommended target ingestion amount, read advice contents stored in the advice DB 12d in association with a result of the comparison (a state of excess or deficiency with respect to a nutrient), and generate advice information to be provided using the read advice contents. The target ingestion amount of a nutrient may be acquired from an external apparatus and set or the user himself/herself may set the target ingestion amount of a nutrient. In addition, the comparison with the target ingestion amount of a nutrient may be performed using, for example, a daily ingestion amount or an ingestion amount for every predetermined time such as every 12 hours, every 6 hours, every 3 hours, or every hour. At this point, when deficient nutrients can be replenished by subscription food purchased by the user, the control unit 11 may generate advice information related to eating the purchased subscription food and a timing of eating the purchased subscription food (ingestion method) and a recipe of a dish using the purchased subscription food (cooking method). Therefore, by generating advice information in accordance with a state of excess or deficiency of a nutrient based on an ingestion amount in a predetermined time, an advice that enables the user to readily remedy the state of excess or deficiency of the nutrient can be provided. In addition, when there is subscription food that can replenish a deficient nutrient, the control unit 11 may generate advice information that recommends purchasing the subscription food. In this case, promotion of sales of the subscription food can be expected. Furthermore, advice information related to ingredients, articles of food, and recipes of dishes that enable the deficient nutrient to be replenished including ingredients and articles of food other than subscription food may be generated.

In addition, the control unit 11 may generate advice information using advice contents registered in the advice DB 12d in association with the age, sex, birthplace, thought and creed, or the like of the user or may generate advice information using advice contents registered in the advice DB 12d in association with a vegetarianism level, preference information, a trend of thought, or the like of the user. In this case, advice information can be generated which takes into consideration an attribute of the user, preferences with respect to ingredients and articles of food, principles, attitude, and the like. Furthermore, the control unit 11 may generate advice information using advice contents registered in the advice DB 12d in association with a physical state indicated by biological information, an amount of exercise indicated by exercise information, an amount of sleep indicated by sleep information, and the like of the user. In this case, advice information can be generated which takes into consideration a physical state, an amount of exercise, an amount of sleep, and the like of the user. In addition, when using time-series information such as an ingestion history, biological information, exercise information, and sleep information, the control unit 11 may generate advice information in accordance with a physical state, an amount of exercise, an amount of sleep, and the like of the user as specified from information at any time point or during any period. Furthermore, with respect to a user who continuously consumes subscription food, the control unit 11 can generate advice information in accordance with a trend of consumption of subscription food by the user based on ingestion history of subscription food of the user. In this manner, based on user information, the control unit 11 generates advice related to actions (lifestyle habits) in the daily life of the user such as diet, exercise, and sleep and generates advice information related to a psychosomatic state (stress, work efficiency, or bodily functions) in the daily life of the user. In this case, advice information can be generated based on comments sent by the user with respect to a change in a physical condition or an emotional change caused by eating subscription food, comments with respect to stress in daily life, comments with respect to work efficiency of studying, working, or the like, comments with respect to cognition such as memory or forgetfulness, and the like.

Note that subscription food according to the present embodiment includes articles of food having been manufactured so as to include not only an edible part of an ingredient but also an inedible part thereof. An "inedible part" of an ingredient according to the present disclosure refers to a portion that is usually not suitable for eating and a portion that is discarded in the usual dietary habit and specifically, for example, refers to a refuse portion as described in "Standards Tables of Food Composition in Japan - 2015- (Seventh Revised Edition) " (a food composition table set by The Ministry of Health, Labor and Welfare). In addition, while ingredients used in subscription food according to the present embodiment may be any kind of food served for human consumption (food described in "Standards Tables of Food Composition in Japan - 2015- (Seventh Revised Edition)"), the ingredients are preferably plants or, in other words, edible plants, and among the food groups described in "Standards Tables of Food Composition in Japan -2015-(Seventh Revised Edition)", pulses, nuts and seeds, vegetables, cereals, fruits, potatoes, mushrooms, and algae can be used as plants. An "edible part" of an ingredient refers to a portion obtained by subtracting the refused portion (inedible part) from the entire ingredient. The inedible part of an ingredient has poor compatibility with ingestion characteristics or other ingredients and, generally, the inedible part is often discarded without being eaten. However, when subscription food according to the present embodiment contains an inedible part of an ingredient, a discarded amount of the ingredient can be reduced. Therefore, by eating subscription food according to the present embodiment, a load of discard processing of the ingredient (inedible part) on earth's environment can be reduced and a contribution can be made to protecting earth's environment. Accordingly, in addition to generating an advice related to diet and nutrients based on the ingestion history of the user, the control unit 11 may also generate advice information related to a contribution toward protecting earth's environment. Even in this case, the control unit 11 can generate advice information related to a contribution toward protecting earth's environment using advice contents registered in the advice DB 12d in association with each article of subscription food and an ingestion amount of the subscription food. Furthermore, subscription food according to the present embodiment preferably contains both an edible part and an inedible part of ingredients, more preferably contains both an edible part and an inedible part of a same type of ingredients, and most preferably contains both an edible part and an inedible part of a same individual ingredient of a same type.

As described above, in the present embodiment, advice information to be provided to the user is generated in accordance with at least a part of user information. When a plurality of pieces of advice information are generated based on stored contents of the advice DB 12d, the control unit 11 may adopt all of the pieces of generated advice information as advice information to be provided or adopt one piece of randomly selected advice information as advice information to be provided. In addition, by setting a priority to each advice stored in the advice DB 12d, when a plurality of pieces of advice information are generated, advice information with a high priority may be adopted as advice information to be provided.

A content of any nutrient in subscription food according to the present embodiment is preferably equal to or larger than an effective amount (for example, equal to or larger than a criterion value that enables a nutrition claim to be made according to the Food Labeling Act). In addition, subscription food preferably contains one or two or more useful components. A useful component as described above refers to a component other than the nutrients defined in Appendix No. 9 of Food Labeling Standards (Cabinet Office Order No. 10 of 2015) which is useful in maintaining or improving health, motor function, beauty care, and the like, and specific examples include polyphenols, carotenoids, chlorophyll, and vitamin U. Specifically, a content of a nutrient is preferably an amount that enables making a claim that supplementation is possible, a claim that appropriate ingestion is possible, a claim regarding non-addition, an absolute claim (a "high" claim or a "source" claim), an absolute claim (a "free" claim or a "low" claim), a relative claim (an "increased" claim or a "reduced" claim), a non-addition claim (sugars, sodium salts, and the like), and the like. Furthermore, specifically, subscription food has a dietary fiber content (particularly, an insoluble dietary fiber content) of preferably 3 percent by mass or more, more preferably 4 percent by mass or more, more preferably 5 percent by mass or more, more preferably 6 percent by mass or more, more preferably 7 percent by mass or more, more preferably 8 percent by mass or more, and even more preferably 9 percent by mass or more, and an amount of dietary fiber (particularly, an amount of insoluble dietary fiber) contained in one serving size (which corresponds to an amount in which the subscription food is eaten in one sitting, and in the case of a product that comes in a single-serving size, one product) is 1 g or more, more preferably 2 g or more, and more preferably 3 g or more. Subscription food containing such a nutrient is preferable since an ingestion amount of the nutrient contained in the food by the user in a specific period can be collected from the user together with a date and time of consumption, an effective ingestion timing and ingestion method of the nutrient which were unknowable in the past can now be known and, by extension, a more effective ingestion timing and ingestion method than in the past can be proposed to the user. In addition, subscription food according to the present embodiment is preferably a useful ingredient containing the nutrient or the useful component described above, more preferably an article of food (dietary fiber-containing food) that is manufactured using an ingredient containing dietary fiber (particularly, preferably derived from a localized site of dietary fiber and more preferably derived from an inedible part of the ingredient), and more preferably an article of food (insoluble dietary fiber-containing food) that is manufactured using an ingredient containing insoluble dietary fiber (particularly, preferably derived from a localized site of insoluble dietary fiber and more preferably derived from an inedible part of the ingredient). A localized site of insoluble dietary fiber is a site where insoluble dietary fiber is localized among an entire ingredient and, specifically, refers to a site with a higher insoluble dietary fiber content ratio than an insoluble dietary fiber content ratio in an edible part. A localized site of insoluble dietary fiber according to the present disclosure represents a site where insoluble dietary fiber is localized among an entire ingredient and, specifically, represents a site with a higher insoluble dietary fiber content ratio than that of an edible part in the ingredient, and represents a site having an insoluble dietary fiber content ratio in a dry state that is more preferably 1.1 times or more than that of the edible part, further preferably 1.2 times or more, further preferably 1.3 times or more, further preferably 1.4 times or more, further preferably 1.5 times or more, further preferably 1.6 times or more, further preferably 1.7 times or more, further preferably 1.8 times or more, further preferably 1.9 times or more, and most preferably 2.0 times or more. In addition, an insoluble dietary fiber content ratio in a localized site of insoluble dietary fiber (particularly, an inedible part) in terms of dry mass is preferably more than 10 percent by mass, more preferably more than 11 percent by mass, more preferably more than 12 percent by mass, more preferably more than 13 percent by mass, more preferably more than 14 percent by mass, more preferably more than 15 percent by mass, more preferably more than 16 percent by mass, more preferably more than 17 percent by mass, more preferably more than 18 percent by mass, more preferably more than 19 percent by mass, and even more preferably more than 20 percent by mass. Furthermore, a similar description applies to a localized site of dietary fiber. "In terms of dry mass" as used in the present disclosure indicates a dry mass value when there is 0 percent by mass of moisture. An amount of moisture in a sample can be measured according to Standards Tables of Food Composition in Japan -2015- (Seventh Revised Edition). While a localized site of dietary fiber or a localized site of insoluble dietary fiber according to the present disclosure may be a part of an "edible part" (for example, a seed coat portion of vegetables, cereals, pulses, or fruits and, particularly, the seed coat portion of pulses) or an "inedible part" of an ingredient described earlier, a localized site of insoluble dietary fiber is preferably an "inedible part". In addition, a content of a localized site of dietary fiber or a localized site of insoluble dietary fiber (particularly, an inedible part) with respect to a total mass of all articles of food purchased by the subscription system according to the present disclosure is preferably 1 percent by mass or more, more preferably 3 percent by mass or more, and even more preferably 5 percent by mass or more. On the other hand, while an upper limit is not usually set, the upper limit may be preferably set to 70 percent by mass or less, more preferably set to 60 percent by mass or less, and even more preferably set to 50 percent by mass or less. Furthermore, a content of all ingredients including a localized site of dietary fiber or a localized site of insoluble dietary fiber (particularly, an inedible part) with respect to a total mass of all articles of food purchased by the subscription system according to the present disclosure is preferably 3 percent by mass or more, more preferably 5 percent by mass or more, and even more preferably 9 percent by mass or more. On the other hand, while an upper limit is not usually set, the upper limit may be preferably set to 70 percent by mass or less, more preferably set to 60 percent by mass or less, and even more preferably set to 50 percent by mass or less. Generally, since an inedible part of an ingredient contains a large amount of dietary fiber or insoluble dietary fiber, manufacturing an article of food so as to include not only an edible part of an ingredient but also an inedible part of the ingredient as in the case of subscription food according to the present embodiment is preferable because food enabling nutrients of ingredients to be ingested without waste can be provided. Therefore, subscription food according to the present embodiment is food which enables nutrients such as dietary fiber to be ingested in an efficient manner and which enables a discarded amount to be reduced.

The control unit 11 of the server 10 may generate advice information according to user information of each user using a learned model having been trained by machine learning or deep learning. In this case, for example, a learned model can be used which has been trained to receive at least a part of the user information stored in the member information DB 12c, a time slot (time of day) in which a request for an advice had been made, and the like as input, compute optimal advice information based on the input information, and output a result of the computation (advice information). Such a learned model can be constructed with, for example, a CNN (Convolution Neural Network), an RNN (Recurrent Neural Network), or an LSTM (Long Short-Term Memory).

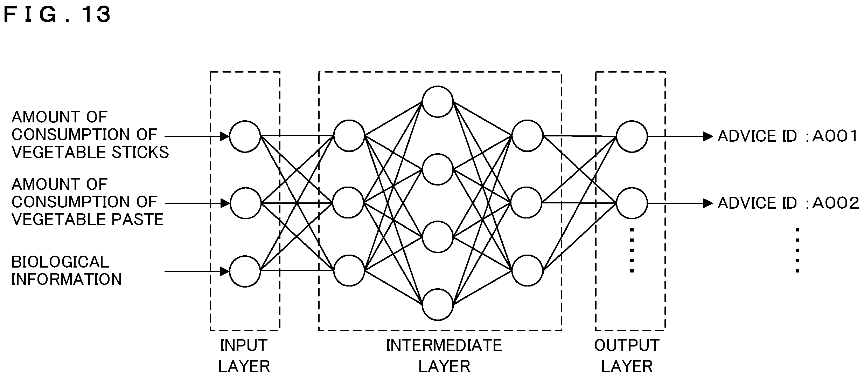

FIG. 13 is a schematic view showing a configuration example of a learning model. The learning model shown in FIG. 13 is a learning model which receives ingestion information of subscription food among user information (specifically, an amount of consumption of vegetable sticks and vegetable pastes) and biological information as input and which is trained to specify advice to be provided when the ingestion information of subscription food and biological information are input. The learning model shown in FIG. 13 is constructed of an input layer, an intermediate layer, and an output layer, the input layer has three input nodes, and an amount of consumption of vegetable sticks and an amount of consumption of vegetable pastes as ingestion information of subscription food and biological information of the user are input to each input node. While FIG. 13 shows a configuration in which biological information is input to one input node, when adopting a configuration in which a plurality of kinds of biological information such as a body-fat percentage and blood pressure are input, an input node to which each piece of biological information is input is provided. In addition, each piece of information (each numerical value) input to the input node may be a day's worth of information or time-series information over a predetermined period such as a week. For example, a configuration may be adopted in which an ingestion history of subscription food over a week, a change in body-fat percentage over a week, or the like is input and, in such a case, an advice that takes into consideration various kinds of history and changes during a predetermined period can be specified.

Each piece of data input to the input node is input to the intermediate layer. The intermediate layer has a plurality of (in FIG. 13, three) fully-connected layers, and a node of each layer calculates an output value based on input data using a weighting coefficient or a function between layers and inputs a calculated output value to a node of a subsequent layer. By sequentially inputting an output value of a node of each layer into a node of a subsequent layer, the intermediate layer finally supplies each output node of the output layer with each output value. The output layer has a plurality of output nodes and each output node outputs a discrimination probability with respect to each advice stored in the advice DB 12d. For example, a first output node outputs a discrimination probability with respect to an advice which is stored in the advice DB 12d and of which an advice ID is A001, and a second output node outputs a discrimination probability with respect to an advice of which an advice ID is A002. The discrimination probability output by each output node indicates a possibility that an advice with an advice ID associated with each output node is appropriate with respect to data input to the input layer. The output value of each output node in the output layer is, for example, a value ranging from 0 to 1.0 and a sum of all discrimination probabilities output from all output nodes is 1.0. The number of input nodes in the input layer, the number of layers in the intermediate layer, and the number of output nodes in the output layer are not limited to the example shown in FIG. 13. The learning model is not limited to a neural network (deep learning) in which the intermediate layer is configured as multiple layers as shown in FIG. 13, and learning models constructed using various machine learning algorithms may be used.

When generating advice information in accordance with user information such as the age, sex, the birthplace, or the thought and creed of the user, a learning model may be used which receives information on the age, sex, the birthplace, or the thought and creed of the user as input and which is trained to specify advice to be provided when these pieces of information are input. When generating advice information in accordance with user information such as the vegetarianism level, preference information, the trend of thought, or the like of the user, a learning model may be used which receives information on the vegetarianism level, preference information, or the trend of thought of the user as input and which is trained to specify advice to be provided when these pieces of information are input. Furthermore, when generating advice information in accordance with user information such as exercise information or sleep information of the user, a learning model may be used which receives information on the exercise information or the sleep information of the user as input and which is trained to specify advice to be provided when these pieces of information are input. In this manner, by training a learning model to receive user information to be taken into consideration when specifying advice information as input and to specify advice contents suitable for input user information, an optimal advice can be specified with respect to any piece of user information. Specifying an advice using such a learning model enables an advice that takes the user's physical state (physical condition) into consideration to be generated. In addition, when using a learning model, a different learning model may be used for each timing where an advice is provided. For example, advice in accordance with user information may be specified using a different learning model for each season, a different learning model for each time slot, and the like. In this case, an optimal advice that also takes a timing (a season, a time slot, or the like) of providing the advice into consideration can be generated.

A learning model such as that described above is trained using training data in which user information to be input and an advice ID of an advice corresponding to the user information is handled as a set. The learning model shown in FIG. 13 is trained using training data in which ingestion information of subscription food and biological information of the user and an advice ID corresponding to the ingestion information and the biological information are handled as a set. The advice used in training data can be advice recommended by an expert such as a physician with respect to corresponding user information, advice related to the user information that the user had felt to be effective, or the like. The learning model is trained so that, when user information (information to be input) included in training data is input to each of the input nodes, an output value of 1.0 is output from an output node corresponding to the advice ID included in the training data and an output value of 0.0 is output from other output nodes. Note that the learning model is trained so as to optimize a weighting coefficient and a function which connect the nodes of each layer in the intermediate layer. Training processing of the learning model may be performed by the server 10 or by another apparatus.

When the control unit 11 generates advice information in accordance with user information, the control unit 11 (output unit) transmits (outputs) an advice screen displaying the advice information as shown in FIG. 12A to the user terminal 20 (S45). When the control unit 21 of the user terminal 20 receives (acquires) an advice screen from the server 10, the control unit 21 displays the received advice screen on the display unit 25 (S46). The advice screen shown in FIG. 12A displays an advice message related to diet, and the advice message shown in FIG. 12A represents contents generated based on, for example, dietary contents (ingestion information) of the user. The advice message shown in FIG. 12A may be generated in consideration of information on a blood glucose level (biological information) of the user in addition to dietary contents of the user. The advice screen has a save button for issuing a save instruction for the displayed advice information and a cancel button for instructing termination of the display without saving the advice information. When the save button is operated via the input unit 24 on the advice screen, the control unit 21 stores the displayed advice information in the storage unit 22 in association with, for example, date and time information of the present time point. Accordingly, the user terminal 20 can accumulate advice provided by the server 10 in association with a date and time at which the advice had been provided.

Due to the processing described above, in the information processing system 100 according to the present embodiment, the server 10 can generate and provide appropriate advice to be provided to a user based on user information registered in the member information DB 12c. The advice provided by the server 10 may be contents generated based on at least a part of user information registered in the member information DB 12c and may be contents that differ in accordance with a timing (a time of day or a time slot) at which the advice is provided. Accordingly, based on information related to the user such as attribute information, profile information, physical information, exercise information, and sleep information of the user, information on subscription food eaten by the user, and the like, the server 10 can provide various kinds of advice related to lifestyle habits in actions of the user including diet, exercise, and sleep, various kinds of advice related to a psychosomatic state (stress, work efficiency, or bodily functions) in the daily life of the user, and advice with respect to a contribution toward protecting earth's environment. By appropriately setting a provision condition of advice in accordance with contents of the advice, an appropriate advice to be provided to the user in accordance with various kinds of information related to the user can be generated. In the information processing system 100 according to the present embodiment, the user terminal 20 can also be configured to generate advice information in accordance with user information. For example, when the user terminal 20 stores user information and the advice DB 12d in the storage unit 22, the control unit 21 can specify advice contents in accordance with the user information based on stored contents of the advice DB 12d. In addition, even when the user terminal 20 stores a learning model in the storage unit 22 in addition to user information and the advice DB 12d, the control unit 21 can specify advice contents in accordance with the user information using the learning model.

In the information processing system 100 according to the present embodiment, the server 10 is not limited to a configuration in which advice to be provided to the user is generated and provided in accordance with an advice request from the user terminal 20. For example, the server 10 may generate and provide advice to be provided to the user on a regular basis or when a time of day set in advance arrives. In such a configuration, for example, when a predetermined period elapses or when a set time of day arrives, the control unit 11 of the server 10 generates advice information to be provided to each user based on user information of each user registered in the member information DB 12c and stores the advice information in the storage unit 12 in association with the member ID of each user. Alternatively, the control unit 11 may store a date and time at which the advice information had been generated in the storage unit 12 in addition to the member ID and the advice information. On the other hand, the user terminal 20 may be configured to access the server 10 and acquire advice information upon starting the advice app 22AP and display the acquired advice information on the home screen (start screen). FIG. 12B shows an example of the home screen displaying advice information, and when advice information is displayed on the home screen, the user can check an advice on the home screen by merely opening the advice app 22AP on the user terminal 20. When the server 10 generates advice information to be provided to each user, the server 10 may push a notification of the generated advice information to the user terminal 20 instead of storing the generated advice information in the storage unit 12. In this case, since the user terminal 20 can display the fact that new advice information has been received on the display unit 25, the user can be notified of the presence of new advice information.

The home screen shown in FIG. 12B displays ingestion states related to dietary fibers, proteins, and lipids based on ingestion information of the user and an exercise state related to the number of steps based on exercise information. These pieces of information may be information acquired by the user terminal 20 from the server 10 when acquiring advice information from the server 10 or information stored in the storage unit 22 at a time point of a previous termination of the advice app 22AP. Target values with respect to an ingestion state and an exercise state may be target values set by the user or target values recommended for each piece of attribute information. In this manner, by displaying information on the daily life of the user such as diet, exercise, and sleep in addition to advice, the home screen can notify a present state of the user. In addition, displayed on the home screen is a "view details" button for instructing display of information indicating a state of the user including an ingestion state of other nutrients, an execution state of other exercises, or a sleep state. Although not illustrated, when the "view details" button is operated on the home screen, the control unit 21 displays an ingestion state of other nutrients, an execution state of other exercises, or a sleep state. Even in this case, each piece of information to be displayed may be information acquired by the user terminal 20 from the server 10 or information stored in the storage unit 22 at a time point of a previous termination of the advice app 22AP. Furthermore, the home screen is configured to accept a display instruction of detailed information related to an ingestion state of each nutrient, an execution state of each exercise, a sleep state, or the like. For example, when a display instruction of detailed information related to an ingestion state of dietary fiber is accepted, the control unit 21 acquires ingestion information related to dietary fiber from the server 10 and, based on the acquired ingestion information, generates and displays a graph showing, for example, a trend of a daily ingestion amount of dietary fiber. In addition, when a display instruction of detailed information related to an exercise state of the number of steps is accepted, the control unit 21 acquires exercise information related to the number of steps from the server 10 and, based on the acquired exercise information, generates and displays a graph showing, for example, a trend of the daily or weekly number of steps. Furthermore, when a display instruction of detailed information related to sleep time is accepted, the control unit 21 acquires sleep information related to the sleep time from the server 10 and, based on the acquired sleep information, generates and displays a graph showing, for example, a trend of a daily sleep time. When the server 10 receives a request for detailed information from the user terminal 20, the server 10 reads the requested nutrient, exercise information, or sleep information from the member information DB 12c and transmits the read information to the user terminal 20. Alternatively, the server 10 may transmit ingestion information of nutrients, exercise information, or sleep information during a predetermined period such as the last week or the last month to the user terminal 20.

In addition, the home screen may be configured to display detailed information related to biological information of the user. For example, when a display instruction of detailed information related to blood pressure of the user is accepted, the control unit 21 acquires biological information related to blood pressure from the server 10 and, based on the acquired biological information, displays a trend of daily blood pressure. In addition, when a display instruction of detailed information related to the weight of the user is accepted, the control unit 21 acquires biological information related to the weight from the server 10 and, based on the acquired biological information, generates and displays a graph indicating a trend of daily weight. By displaying a trend of biological information in this manner, the user can comprehend a change in his/her own physical state. The advice screen shown in FIG. 12A can also be configured so that ingestion information, exercise information, or sleep information of the user is displayed in a similar manner to the home screen shown in FIG. 12B. Even in this case, a configuration may be adopted in which detailed information related to an ingestion state of each nutrient, an execution state of each exercise, a sleep state, or the like is displayed via the advice screen shown in FIG. 12A.

In the information processing system 100 according to the present embodiment, the user terminal 20 may be configured to accept an evaluation by the user with respect to advice contents acquired from the server 10. According to such a configuration, an evaluation by the user can be fed back to advice contents, and by updating advice contents with a low evaluation, advice that is effective to the user can be prepared. For example, the screen shown in FIG. 12A or FIG. 12B may be provided with an evaluate button ("like" button) for inputting that a displayed advice has been useful and an advice of which contents are to be updated may be selected according to evaluation contents by the evaluate button. Alternatively, an evaluation with respect to advice may be accepted on a scale of one to five and contents of advice with a low evaluation may be updated. Contents of an evaluation by the user with respect to advice may be transmitted to the server 10 and stored in the member information DB 12c.

The information processing system 100 according to the present embodiment is not limited to a configuration in which advice to be provided to the user of the user terminal 20 is generated by the server 10 and, for example, advice may be generated by the user terminal 20 and displayed on the display unit 25. In this case, a configuration can be adopted in which the user terminal 20 stores various pieces of user information and the advice DB 12d input via the input unit 24 in the storage unit 22 and the control unit 21 (information processing unit) generates advice information in accordance with at least a part of the user information stored in the storage unit 22 based on stored contents of the advice DB 12d.

### (Second embodiment, which is not encompassed by the wording of the claims but is useful for understanding the invention)

An information processing system will be described in which not only subscription food but food other than subscription food (hereinafter, referred to as general food) is also considered an ingestion object (consumption object) and an ingestion history of each user registered in the server 10 also includes ingestion information of general food. Since the information processing system according to the present embodiment can be realized by a similar apparatus to that of the information processing system according to the first embodiment, a description of a configuration will be omitted. The ingestion history stored in the member information DB 12c in the server 10 according to the present embodiment includes not only information on subscription food eaten by the user and a date and time at which the subscription food had been eaten (date and time of ingestion) but also includes information on general food eaten by the user and a date and time at which the general food had been eaten. Instead of information on the subscription food and the general food eaten by the user, the ingestion history may store information on energy and nutrients ingested by the user by eating the subscription food and the general food.

In the information processing system 100 according to the present embodiment, the user terminal 20 not only performs processing of accepting ingestion information of food eaten by the user according to input from the input unit 24 but also performs processing of accepting ingestion information by photographing food eaten by the user with the camera 27. In addition, when acquiring a photographed image of food eaten by the user from the user terminal 20, the server 10 analyzes the photographed image and performs processing of specifying information on the food eaten by the user or specifying information on nutrients ingested through the food eaten by the user. Besides a configuration in which the server 10 performs processing of specifying information on food eaten by the user or information on nutrients ingested by the user from a photographed image, a configuration may be adopted in which the user terminal 20 performs the same processing. In this case, the user terminal 20 specifies information on food eaten by the user or information on nutrients ingested by the user by analyzing an image photographed by the camera 27 and transmits specified ingestion information to the server 10.

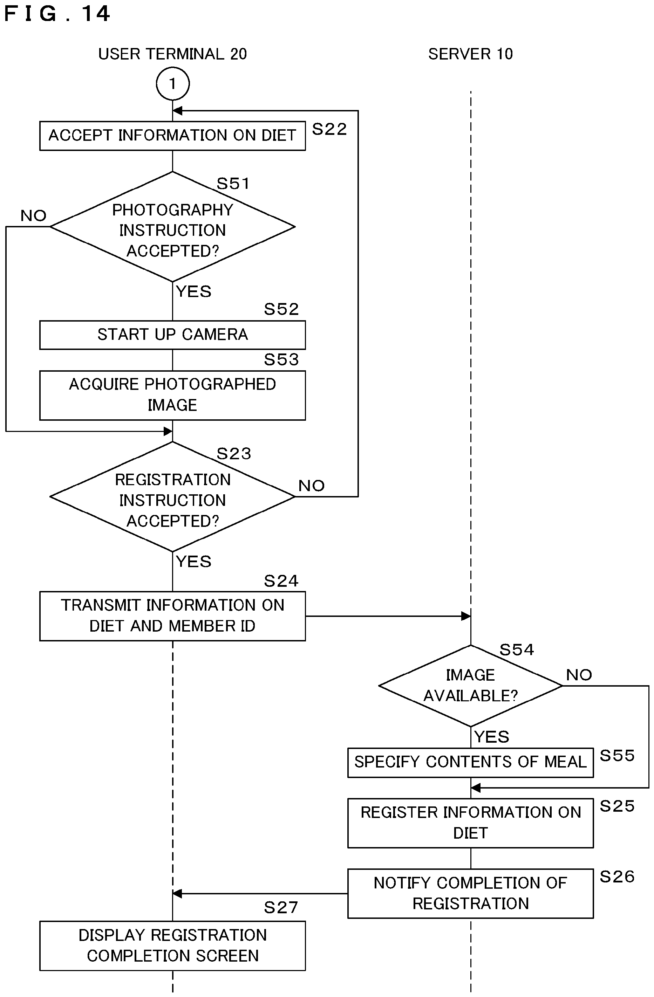

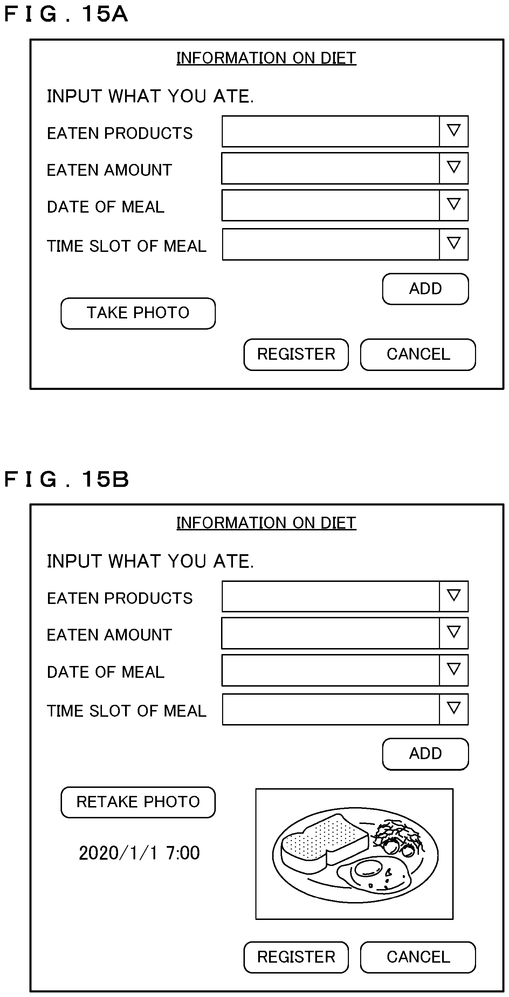

FIG. 14 is a flow chart showing an example of a registration processing procedure of user information according to a second embodiment, and FIGS. 15A and 15B are schematic views showing screen examples of the user terminal 20. The processing shown in FIG. 14 represents an addition of steps S51 to S53 between steps S22 and S23 and an addition of steps S54 to S55 before step S25 in the processing shown in FIGS. 6 and 7. Descriptions of same steps as in FIGS. 6 and 7 will be omitted. In addition, in FIG. 14, illustration of each step in FIG. 6 and steps S28 to S35 in FIG. 7 is omitted.

In the information processing system 100 according to the present embodiment, the user terminal 20 and the server 10 perform processing similar to steps S11 to S22 in FIGS. 6 and 7. Accordingly, when the user terminal 20 accepts information (profile information, physical information, information on exercise, and information on sleep) of the user input via the input screens shown in FIGS. 8B to 9B, the user terminal 20 transmits the accepted information to the server 10 and the server 10 registers the user information received from the user terminal 20 in the member information DB 12c.

In step S21, the user terminal 20 according to the present embodiment displays an input screen of information on diet such as that shown in FIG. 15A on the display unit 25. The input screen of information on diet shown in FIG. 15A has a similar configuration to the input screen shown in FIG. 10A and further has a "take photo" button for taking a photograph of a meal and articles of food eaten by the user with the camera 27. The input screen shown in FIG. 15A may have an input field for inputting contents of a meal eaten by the user or contents of nutrients ingested through the eaten meal in the form of, for example, text data. In this case, the control unit 21 can accept contents of the meal eaten by the user or contents of nutrients ingested by the user via the input field.

The user inputs each piece of information of subscription food that the user had eaten and a date and a time slot at which the user had eaten the subscription food to each input field on the input screen shown in FIG. 15A. Accordingly, the control unit 21 of the user terminal 20 accepts ingestion information of subscription food (information on diet) input by the user (S22). The control unit 21 displays each piece of accepted information in a corresponding input field. In addition, when the user wishes to photograph subscription food and general food (meal) eaten by the user himself/herself with the camera 27, the user selects and operates the "take photo" button via the input unit 24. The control unit 21 determines whether or not an execution instruction (photography instruction) for photographing a meal eaten by the user with the camera 27 has been accepted in accordance with whether or not the "take photo" button has been operated via the input unit 24 (S51). When the control unit 21 determines that a photography instruction has not been accepted (S51: NO), the control unit 21 makes a transition to the processing of step S23. When the control unit 21 determines that a photography instruction has been accepted (S51: YES), the control unit 21 starts up the camera 27 (S52), and performs photography in accordance with the photography instruction via the input unit 24 and acquires a photographed image (S53). When the control unit 21 acquires a photographed image of a meal, the control unit 21 displays the photographed image on the input screen of information on diet as shown in FIG. 15B. A "retake photo" button for retaking a photograph of the meal is displayed on the input screen shown in FIG. 15B. When the "retake photo" button is operated, the control unit 21 once again starts up the camera 27, once again acquires a photographed image in accordance with the photography instruction via the input unit 24, and displays the acquired photographed image on the input screen of information on diet. Besides general food eaten by the user himself/herself, the user can also photograph subscription food with the camera 27 instead of inputting ingestion information via the input fields. Therefore, besides general food eaten by the user, the control unit 21 may also accept ingestion information of subscription food eaten by the user by acquiring a photographed image of the subscription food. When the register button has been operated on the input screen shown in FIG. 15B or, in other words, when the control unit 21 accepts a registration instruction (S23: YES), the control unit 21 associates information on diet (ingestion information input via the input field and a photographed image) input via the input screen and the member ID of the user with each other and transmits the associated information to the server 10 (S24).

When the control unit 11 of the server 10 according to the present embodiment acquires information on diet from the user terminal 20, the control unit 11 determines whether or not the information includes a photographed image (S54). When it is determined that a photographed image is included (S54: YES), the control unit 11 analyzes the photographed image and specifies contents of the meal eaten by the user (S55). For example, the control unit 11 specifies a type of articles of food, ingredients, and dishes that appear in the photographed image by, for example, template matching. In this case, a template indicating an image feature amount extracted from a photographed image created by photographing articles of food, ingredients, and dishes is stored in the storage unit 12 in advance, and when the control unit 11 detects a region matching the template from the photographed image, the control unit 11 specifies that the detected region is a region of an article of food, an ingredient, or a dish corresponding to the template. The control unit 11 specifies all articles of food, ingredients, and dishes included in the photographed image. Instead of specifying the articles of food, ingredients, and dishes included in the photographed image, the control unit 11 may be configured to specify a type and an ingestion amount of nutrients that are ingested by eating the articles of food, ingredients, and dishes included in the photographed image. Alternatively, the control unit 11 may specify types of the articles of food, ingredients, and dishes included in the photographed image using a learned model having been trained in advance with respect to various articles of food, ingredients, dishes, and the like by machine learning or deep learning. In this case, a learning model may be used which has been trained to receive a photographed image as an input and to output a type and an amount of an article of food, an ingredient, or a dish in the input photographed image. Alternatively, the learning model may be a model trained to output, when a photographed image is input, a type and an ingestion amount of nutrients that are ingested by eating the articles of food, ingredients, and dishes that appear in the photographed image. Such a learned model can be constructed by, for example, a CNN.

When the control unit 11 determines that a photographed image is not included in the information on diet acquired from the user terminal 20 (S54: NO), the control unit 11 skips processing of step S55. Subsequently, the control unit 11 registers the information on diet acquired from the user terminal 20 and information on diet specified from a photographed image in the member information DB 12c (S25). Accordingly, besides ingestion information input via an input field on the input screen of the user terminal 20, contents of a meal (ingestion information) photographed by the camera 27 of the user terminal 20 is registered in the member information DB 12c. Subsequently, the control unit 11 of the server 10 and the control unit 21 of the user terminal 20 execute processing of step S26 and thereafter.

According to the processing described above, the user terminal 20 can transmit an image obtained by photographing a meal eaten by the user to the server 10, and the server 10 can acquire contents of the meal (articles of food, ingredients, and dishes) that appear in the photographed image received from the user terminal 20. Accordingly, even in the present embodiment, information (user information) such as attribute information, profile information, information related to a physical state (biological information), information related to exercise, and information related to sleep of the user, a type, an amount (ingestion amount), and an ingestion timing of articles of food eaten by the user, and the like can be input using the user terminal 20 and registered in the server 10. In addition, when a meal is to be photographed immediately before the user eats the meal, since a date and time of photography can be adopted as a date and time of ingestion of the meal, an ingestion timing (a date and time of ingestion) of the meal can be automatically acquired from the date and time of photography. In the present embodiment, types, ingestion amounts, and ingestion timings of articles of food and ingredients eaten by the user can be registered for not only subscription food but also general food.

The user terminal 20 and the server 10 according to the present embodiment can perform processing similar to that shown in FIG. 11. Therefore, by having the user terminal 20 and the server 10 perform similar processing, the user terminal 20 can be provided with advice information in accordance with user information registered in the server 10.

In the present embodiment, a similar advantageous effect to the first embodiment described above can be obtained. In addition, in the present embodiment, a user can input information on a meal (subscription food and general food) eaten by the user himself/herself on the user terminal 20 by not only inputting information on the meal eaten by the user himself/herself via the input unit 24 but also by taking a photograph using the camera 27. Furthermore, in the present embodiment, advice in accordance with ingestion information of not only subscription food but also general food eaten by the user can be provided. The modifications described above in the first embodiment can also be applied to the information processing system 100 according to the present embodiment.

### (Third embodiment, which is not encompassed by the wording of the claims but is useful for understanding the invention)

An information processing system which calculates a score based on various criteria using various kinds of information related to a user (user information) and which provides the user with the score will be described. Since the information processing system according to the present embodiment can be realized by a similar apparatus to that of the information processing system 100 according to the first embodiment, a description of a configuration will be omitted. The storage unit 12 of the server 10 according to the present embodiment stores a score calculation rule DB 12e in addition to the DBs 12a to 12d shown in FIGS. 3A to 5.

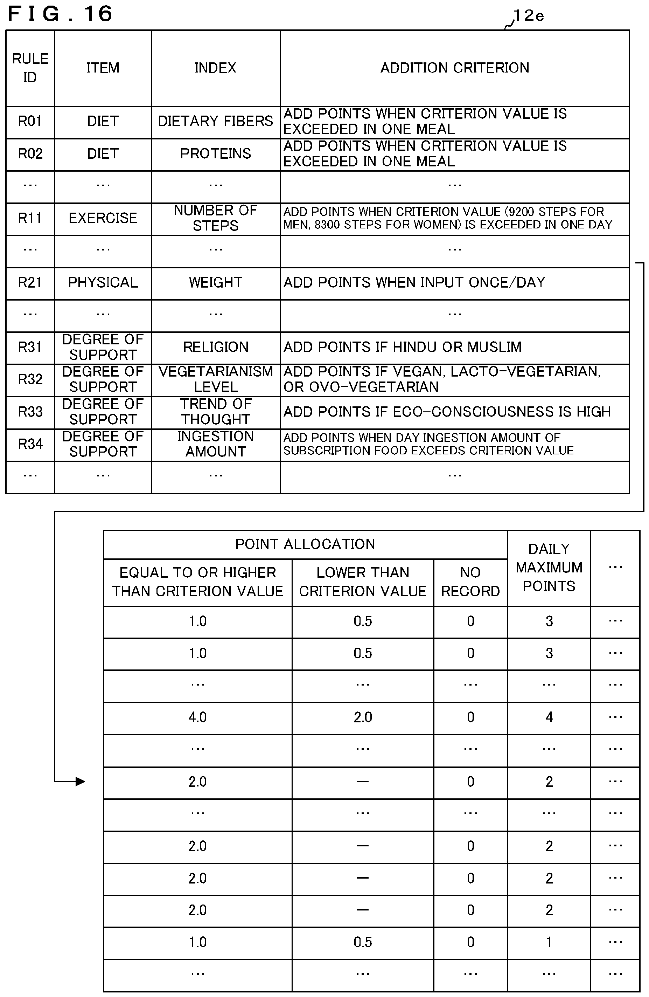

FIG. 16 is a schematic view showing a configuration example of the score calculation rule DB 12e. The score calculation rule DB 12e stores a rule applied when calculating a score to evaluate the user based on user information including a dietary state, a state of exercise, a physical state, and a state of sleep of the user. The score calculation rule DB 12e shown in FIG. 16 includes a rule ID column, an item column, an index column, an addition criterion column, a point allocation column, and a daily maximum points column and stores, in association with the rule ID, information on a rule that is applied when calculating a score. The rule ID column stores identification information (a rule ID) assigned to each rule. The item column stores an item of the rule and, for example, "diet" is stored in the case of a rule related to diet, "exercise" is stored in the case of a rule related to exercise, and "physical" is stored in the case of a rule related to a physical state. In addition, in the case of a rule related to a degree of support or a degree of empathy of the user with respect to subscription food according to the present embodiment, "degree of support" is stored in the item column. Subscription food according to the present embodiment is preferably manufactured using plant-based ingredients and manufactured so as to include not only an edible part but also an inedible part of the ingredients. In this case, the degree of support of the user with respect to subscription food according to the present embodiment can be represented by a score based on preference information, a trend of thought, a vegetarianism level, and the like of the user such as a preference for plant-based ingredients, a desire to contribute toward protecting earth's environment, and high eco-consciousness. The index column stores an index to be used as an addition object when calculating a score based on each item, the addition criterion column stores an addition criterion when calculating a score based on each index, and the point allocation column stores a point that is added in accordance with whether or not a criterion value indicated in the addition criterion is satisfied. The point allocation column shown in FIG. 16 respectively stores a point when the criterion value is satisfied (equal to or higher than the criterion value), a point when the criterion value is not satisfied (lower than the criterion value), and a point when there is no input by the user (no record). In the example shown in FIG. 16, a rule is stored which, for example, adds 1.0 points when an ingestion amount of dietary fiber that is ingested in one meal is equal to or larger than a criterion value in accordance with attributes of the user, adds 0.5 points when the ingestion amount is smaller than the criterion value, and adds no points when information related to diet has not been input (registered) (in other words, when there is no record). The criterion value in accordance with an attribute of the user may be stored in the score calculation rule DB 12e or stored in a predetermined region of the storage unit 12. The daily maximum points column stores points that can be added in a day with respect to each item. Besides points that can be added per day, the maximum points may be points that can be added per week or points that can be added per predetermined time such as 12 hours or six hours. The rule ID stored in the score calculation rule DB 12e is issued by the control unit 11 and stored every time a new rule is registered. Other information stored in the score calculation rule DB 12e is stored by the control unit 11 when the control unit 11 acquires each piece of information of a new rule via, for example, the communication unit 13 or the input unit 14. The stored contents of the score calculation rule DB 12e are not limited to the example shown in FIG. 16 and various kinds of information related to rules to be referred to when calculating a score can be stored.

In the information processing system 100 according to the present embodiment, the user terminal 20 and the server 10 perform registration processing of user information similar to the processing shown in FIGS. 6 and 7. Accordingly, the user terminal 20 can accept user information such as attribute information, profile information, biological information, exercise information, sleep information, and ingestion information (information on diet) of the user and the server 10 can register the user information input via the user terminal 20 in the member information DB 12c.

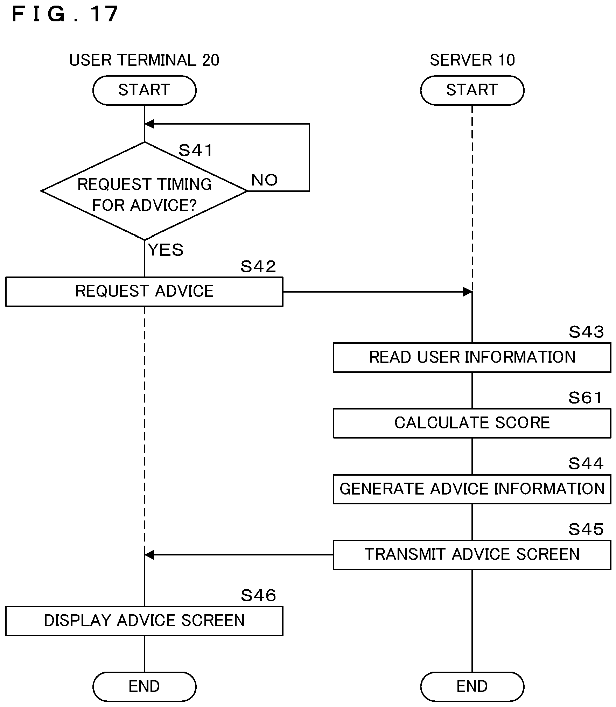

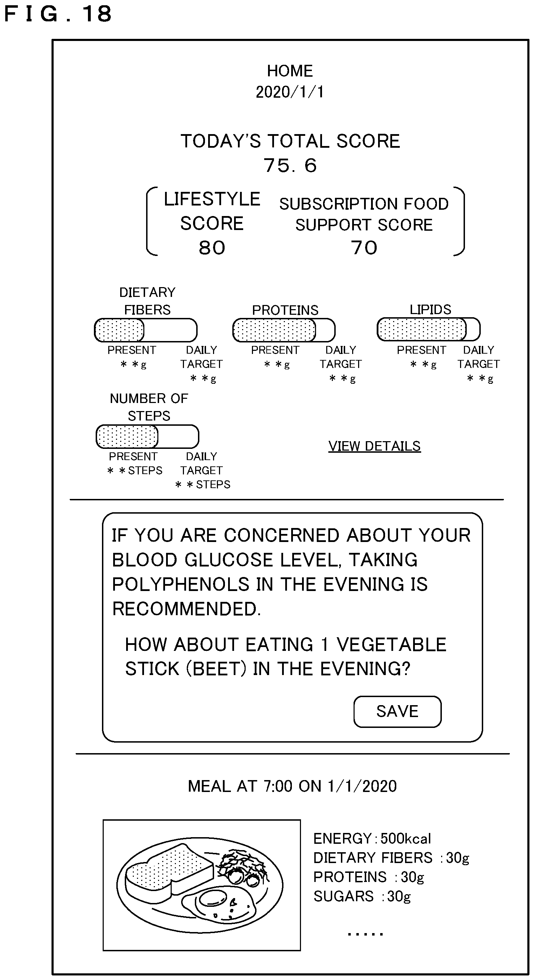

FIG. 17 is a flow chart showing an example of a provision processing procedure of an advice according to a third embodiment, and FIG. 18 is a schematic view showing a screen example of the user terminal 20. The processing shown in FIG. 17 represents an addition of step S61 between steps S43 and S44 in the processing shown in FIG. 11. Descriptions of same steps as in FIG. 11 will be omitted. In the information processing system 100 according to the present embodiment, the user terminal 20 and the server 10 perform processing similar to steps S41 to S43 in FIG. 11. Accordingly, when the server 10 accepts a request for an advice from the user terminal 20, the control unit 11 can read user information stored in the member information DB 12c in association with the member ID received from the user terminal 20.

In addition, the control unit 11 of the server 10 calculates a score in accordance with the read user information based on stored contents of the score calculation rule DB 12e (S61). Specifically, the control unit 11 extracts information related to an index to be used to calculate the score based on the score calculation rule DB 12e from the user information and calculates the score by performing addition in accordance with the information related to the index. For example, the control unit 11 extracts ingestion information related to dietary fiber from the user information and specifies points to be added based on the ingestion information of dietary fiber and an addition criterion and a point allocation related to dietary fiber. Accordingly, a score based on contents of a meal eaten by the user or contents of nutrients ingested through the meal is calculated. In addition, the control unit 11 extracts exercise information related to the number of steps from the user information and specifies points to be added based on the extracted number of steps and an addition criterion and a point allocation related to the number of steps. Accordingly, a score based on contents of exercise by the user is calculated. In addition, the control unit 11 extracts biological information from the user information and specifies points to be added based on whether or not an addition criterion is satisfied with respect to the extracted biological information. Accordingly, a score in accordance with a physical state (biological information) of the user is calculated. The control unit 11 calculates a score of the user by specifying points to be added and calculating total points with respect to each index stored in the score calculation rule DB 12e. In other words, the control unit 11 calculates a score based on information related to diet of the user, a score based on information related to actions in daily life such as exercise and sleep, a score based on information related to a physical state, and the like.

When subscription food according to the present embodiment is food including an inedible part of an ingredient containing a large amount of insoluble dietary fiber, dietary fiber can be ingested in an efficient manner and, at the same time, since only a small portion is discarded, a contribution can be made toward protecting earth's environment. Therefore, the control unit 11 can also calculate a score indicating a contribution made by the user by eating subscription food toward protecting earth's environment based on an ingestion state (an ingestion history) of subscription food of the user. For example, the control unit 11 extracts ingestion information related to subscription food from the user information and specifies points to be added based on the ingestion information of subscription food and an addition criterion and a point allocation in accordance with the ingestion amount. Accordingly, a score indicating a contribution toward protecting earth's environment based on ingestion amount of subscription food eaten by the user is calculated. Since it is conceivable that, the larger the amount of consumption of subscription food, the higher the degree of support of the user with respect to subscription food, a score indicating the degree of support of the user with respect to subscription food according to the present embodiment can also be calculated according to the amount of consumption of subscription food. In addition, the degree of support of the user with respect to subscription food according to the present embodiment can be indicated based on thought and creed, a vegetarianism level, preference information, a trend of thought, or the like of the user, and scores indicating such degrees of support of the user can also be calculated. For example, the control unit 11 extracts information such as thought and creed, a vegetarianism level, preference information, or a trend of thought of the user from the user information and specifies points to be added based on whether or not an addition criterion is satisfied with respect to the extracted information. Accordingly, a score indicating the degree of support of the user with respect to subscription food according to the present embodiment is calculated based on preference information of the user with respect to diet (articles of food or ingredients) or a trend of thought related to the user's attitude in daily life. The control unit 11 may calculate a comprehensive score by adding up added points based on all indexes stored in the score calculation rule DB 12e or calculate an item score for each item. In addition, the control unit 11 may calculate a lifestyle score by adding up added points based on indexes related to the items of diet, exercise, and sleep or calculate a subscription food support score by adding up added points based on indexes such as thought and creed, a vegetarianism level, preference information, a trend of thought, and an ingestion amount of subscription food of the user. The score indicating a contribution toward protecting earth's environment may be added to the lifestyle score, added to the subscription food support score, or used as an independent score.

The control unit 11 is not limited to a configuration of calculating a score when an advice is requested from the user terminal 20. For example, when the control unit 11 receives user information from the user terminal 20 and stores the user information in the member information DB 12c, the control unit 11 may update a score of the user based on the stored user information. Specifically, a determination may be made as to whether or not there are points to be added to the score in accordance with the user information stored in the member information DB 12c and, when it is determined that there are points to be added, the score of the user may be updated by adding the points to the score. In this case, the score of the user can be recalculated every time the user information stored in the member information DB 12c is updated. By storing a recalculated score in, for example, the member information DB 12c, when advice is requested by the user terminal 20, the control unit 11 may specify a score of the user by reading the score stored in the member information DB 12c.

In addition, the control unit 11 generates advice information with respect to the user information based on stored contents of the advice DB 12d (S44). In the present embodiment, the control unit 11 may generate advice information in accordance with the score calculated in step S61 in addition to user information. In this case, in addition to user information, the advice DB 12d stores, in association with a provision condition related to the score calculated based on the user information, advice contents to be provided under such a provision condition. Furthermore, the control unit 11 transmits an advice screen displaying the calculated score and the generated advice information to the user terminal 20 (S45). The control unit 21 of the user terminal 20 receives the advice screen from the server 10 and displays an advice screen such as that shown in FIG. 18 on the display unit 25 (S46). The advice screen shown in FIG. 18 has a similar configuration to the advice screen shown in FIG. 12B and further displays a score calculated based on user information. While the advice screen shown in FIG. 18 displays an overall score, a lifestyle score, and a support score with respect to one day, alternatively, only the overall score may be displayed or only the lifestyle score and the support score may be displayed. Each score may be a score with respect to a day or a score with respect to a week. In addition, the advice screen may display a result of a comparison with a score of the previous day or display a list of scores of the respective days of a last week. Furthermore, in the case of a configuration of registering an image obtained by photographing a meal with the camera 27 of the user terminal 20 as ingestion information in the server 10 as in the second embodiment described above, the server 10 may generate an advice screen displaying a photographed image of the meal as shown in FIG. 18 and provide the user terminal 20 with the advice screen.

Due to the processing described above, based on user information acquired from the user terminal 20 and stored in the member information DB 12c, the server 10 can calculate a score indicating an evaluation with respect to a physical state, a state of diet, a state of exercise, a state of sleep, and the like of the user and provide the user with the score. In addition, the user may be provided with a score indicating a degree of support with respect to subscription food based on preference information or a trend of thought of the user.

In the present embodiment, a similar advantageous effect to the respective embodiments described above can be obtained. In addition, in the present embodiment, based on user information input via the user terminal 20, not only an advice to be provided to the user can be specified but a score for evaluating a state of the user can be calculated. Therefore, the user can be provided with a score in accordance with user information in addition to an advice in accordance with user information. Since the user can comprehend his/her own state by means of a numerical value (score), the user can readily comprehend whether or not a state related to lifestyle habits such as diet, exercise, sleep, and the like is good. The configuration according to the present embodiment can also be applied to the information processing system 100 according to the second embodiment, and a similar advantageous effect is obtained even when the configuration according to the present embodiment is applied to the information processing system 100 according to the second embodiment. Furthermore, the modifications described above in the respective embodiments can also be applied to the information processing system 100 according to the present embodiment. The information processing system 100 according to the present embodiment is not limited to a configuration in which a score for evaluating a state of a user of the user terminal 20 is calculated by the server 10 and, for example, the score may be calculated by the user terminal 20. In this case, a configuration can be adopted in which the user terminal 20 stores various pieces of user information and the score calculation rule DB 12e input via the input unit 24 in the storage unit 22 and the control unit 21 (information processing unit) calculates a score in accordance with at least a part of the user information stored in the storage unit 22 based on stored contents of the score calculation rule DB 12e.

### (Fourth embodiment, which is not encompassed by the wording of the claims but is useful for understanding the invention)

An information processing system which classifies users into a plurality of classes (user classification) based on various kinds of information related to a user (user information) will be described. Since the information processing system according to the present embodiment can be realized by a similar apparatus to that of the information processing system 100 according to the first embodiment, a description of a configuration will be omitted. Note that the member information DB 12c stored in the storage unit 12 of the server 10 according to the present embodiment slightly differs from the configuration of the member information DB 12c shown in FIG. 4. In addition, the storage unit 12 of the server 10 according to the present embodiment stores a classification condition DB in addition to the DBs 12a to 12d shown in FIGS. 3A to 5.

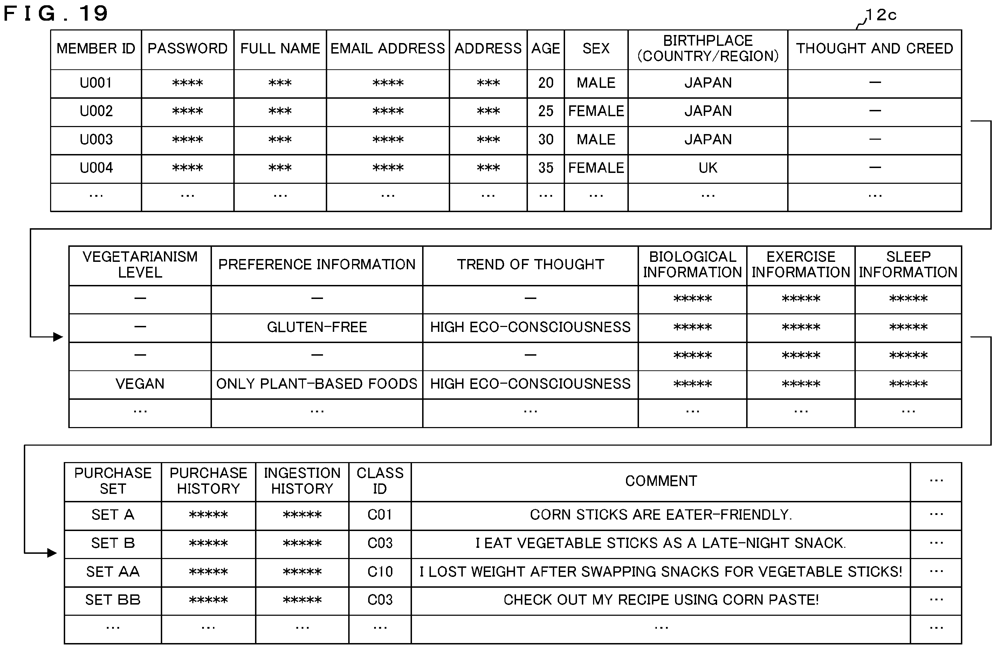

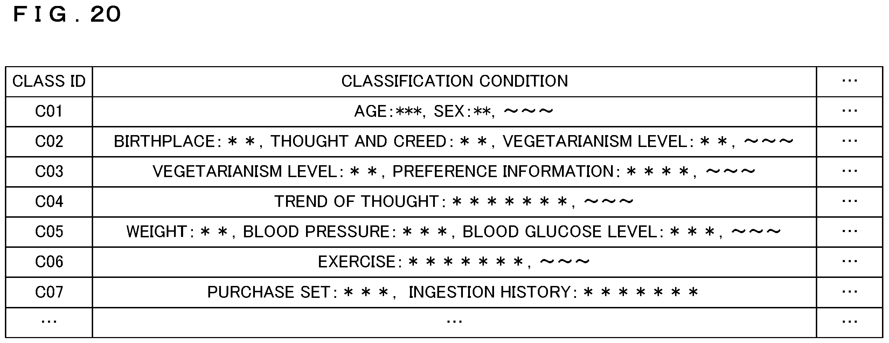

FIG. 19 is a schematic view showing a configuration example of the member information DB 12c according to a fourth embodiment, and FIG. 20 is a schematic view showing a configuration example of a classification condition DB. The member information DB 12c according to the present embodiment has a class ID column and a comment column in addition to the configuration of the member information DB 12c according to the first embodiment shown in FIG. 4. The class ID column stores identification information (class ID) indicating a class into which each user is classified based on user information, and when the control unit 11 classifies each user based on user information, a class ID of the classified class is stored by the control unit 11. The comment column stores a comment input by the user using the user terminal 20, and when the control unit 11 receives a comment from the user terminal 20 via the communication unit 13, the comment is stored by the control unit 11.

The classification condition DB stores a classification condition that applies when classifying users into a plurality of classes according to an attribute, preference information, a trend of thought, a physical state, a dietary state, a state of exercise, a state of sleep, or the like of the user. The classification condition DB shown in FIG. 20 includes a class ID column and a classification condition column, and stores, in association with the class ID, a classification condition that applies when classifying users into each class. The class ID column stores identification information (a class ID) assigned to each of a plurality of classes set in advance. The classification condition column stores a condition that applies when classifying each user into a class indicated by the class ID. The classification condition may be a classification condition according to attribute information such as age and sex, a classification condition according to profile information such as a birthplace, thought and creed, and a vegetarianism level, a classification condition according to biological information such as a weight, blood pressure, and a blood glucose level, a classification condition according to exercise information or sleep information, or a classification condition according to a purchased set or an ingestion history. In this manner, the classification condition may be a combination of conditions related to one or a plurality of pieces of information included in user information. The class ID stored in the classification condition DB is issued by the control unit 11 and stored every time a new class (classification condition) is registered. A classification condition stored in the classification condition DB is stored by the control unit 11 when the control unit 11 acquires a classification condition of a new class via, for example, the communication unit 13 or the input unit 14. The stored contents of the classification condition DB are not limited to the example shown in FIG. 20 and various kinds of information related to a class to which a user is classified can be stored. For example, a name (class name) attached to each class and a member ID of a user classified to each class may be stored in the classification condition DB.

In the information processing system 100 according to the present embodiment, the user terminal 20 and the server 10 perform registration processing of user information similar to the processing shown in FIGS. 6 and 7. Accordingly, the user terminal 20 can acquire user information such as attribute information, profile information, biological information, exercise information, sleep information, and ingestion information (information on diet) of the user via the input unit 24 and the server 10 can register the user information input via the user terminal 20 in the member information DB 12c. In addition, in the present embodiment, the user terminal 20 is configured to acquire any comment via the input unit 24 in addition to user information, and a comment input via the user terminal 20 is also stored in the member information DB 12c of the server 10. The comment input via the user terminal 20 may be any kind of comment such as a comment related to a change in a physical condition or an emotional change due to the user eating subscription food, subscription food or a recipe of a dish using subscription food which the user wishes to recommend to other users, and the like.

In addition, the user terminal 20 and the server 10 according to the present embodiment perform advice provision processing similar to the processing shown in FIG. 11. Accordingly, the server 10 can provide the user terminal 20 with advice in accordance with user information of each user acquired from the user terminal 20 and registered. In the present embodiment, since each user is classified into classes (user classification) based on user information, the advice DB 12d can be set for each class. In other words, the advice DB 12d in accordance with each class is stored in the storage unit 12 and, based on the advice DB 12d in accordance with a class of a user, the server 10 generates advice in accordance with user information of the user. In this manner, by providing the advice DB 12d for each class, advice that is more suitable for each user can be generated. Furthermore, in the case of a configuration in which the server 10 specifies advice in accordance with user information of each user using a learned model, a different learned model may be provided for each class. Even in this case, an appropriate advice that better reflects class contents can be generated.

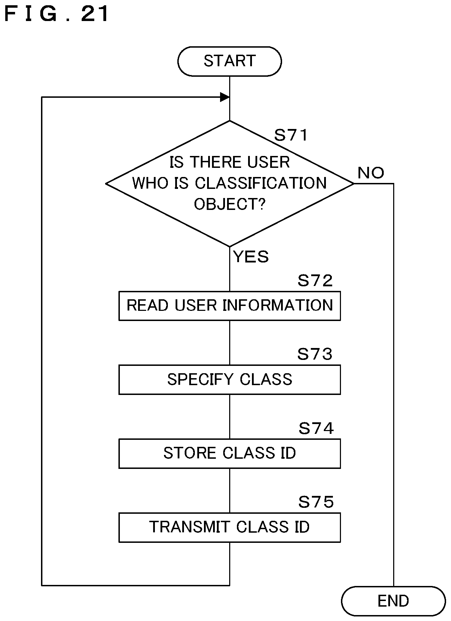

The server 10 according to the present embodiment performs processing of classifying each user into one of a plurality of classes based on each piece of user information acquired from the user terminal 20 and stored in the member information DB 12c. FIG. 21 is a flow chart showing an example of a user classification processing procedure. The following processing is executed by the control unit 11 in accordance with the control program 12P stored in the storage unit 12 of the server 10, and a part of the processing described below may be realized by an exclusive hardware circuit.

In the information processing system 100 according to the present embodiment, the control unit 11 of the server 10 determines whether or not there is a user who is a classification object to be classified into a class (S71). For example, when the control unit 11 acquires, via the communication unit 13 or the input unit 14, user information of a user to be newly registered as a member and stores the acquired user information in the member information DB 12c, the control unit 11 considers the user to be a classification object and determines that there is a user who is a classification object. In addition, when the control unit 11 acquires an execution request for user classification from the user terminal 20 of a user already registered as a member, the control unit 11 may consider the user to be a classification object and determine that there is a user who is a classification object. Furthermore, the control unit 11 may regularly re-classify classes of all users and, in this case, the control unit 11 may consider all users to be a classification object and determine that there is a user who is a classification object on a regular basis or when a time of day set in advance arrives. When the control unit 11 determines that there is no user who is a classification object (S71: NO), the control unit 11 ends the processing.

When the control unit 11 determines that there is a user who is a classification object (S71: YES), the control unit 11 reads user information of one classification object from the member information DB 12c (S72) and, from the read user information, specifies a class into which the user is to be classified based on stored contents of the classification condition DB (S73). For example, the control unit 11 determines whether or not each piece of information included in the user information read from the member information DB 12c matches a classification condition of each class stored in the classification condition DB, and when there is a class that matches a classification condition, the control unit 11 specifies the class as a class of the user who is the classification object. When each piece of information in the user information matches classification conditions of a plurality of classes, the control unit 11 may specify the plurality of classes. In addition, when each piece of information in the user information does not match a classification condition of any of the classes, the control unit 11 may specify a classification condition that is nearest to each piece of information in the user information and specify a class of the specified classification condition.

When the control unit 11 specifies a class of the user who is a classification object, the control unit 11 stores a class ID of the specified class in the member information DB 12c in association with a member ID of the user who is a classification object (S74). Accordingly, information on the class into which each user is classified can be managed by the member information DB 12c. In addition, the control unit 11 transmits the class ID of the specified class to the user terminal 20 of the user who is a classification object (S75). Accordingly, information on the class into which each user is classified can also be managed by the user terminal 20, and when the user terminal 20 displays the received class ID on the display unit 25, the user can be notified of the class into which the user has been classified. The server 10 may provide the user terminal 20 with information indicating a class, a name attached to the class, or the like in place of a class ID and, in such a case, the user can readily comprehend contents of the class into which the user has been classified. The control unit 11 need not provide the user terminal 20 of the user who is a classification object with the class ID of the class into which the user has been classified and a configuration may be adopted in which information on the class into which each user is classified is only managed by the server 10 (the member information DB 12c).

After the processing of step S75, the control unit 11 returns to the processing of step S71 to determine whether or not there is another user who is a classification object (S71), and when the control unit 11 determines that there is another user who is a classification object (S71: YES), the control unit 11 performs processing of steps S72 to S75 with respect to the user who is a classification object. Accordingly, the control unit 11 also performs class classification in accordance with user information with respect to other users who are classification objects and registers the class IDs of the classified classes in the member information DB 12c.

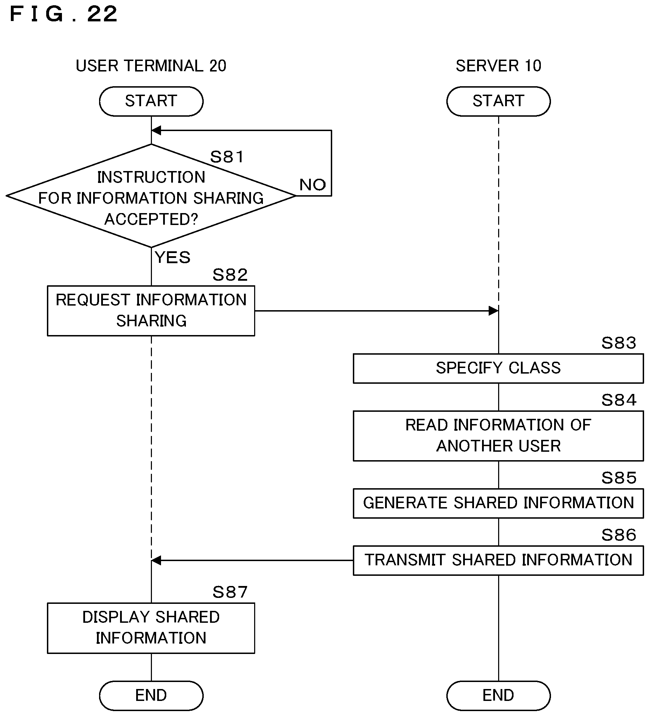

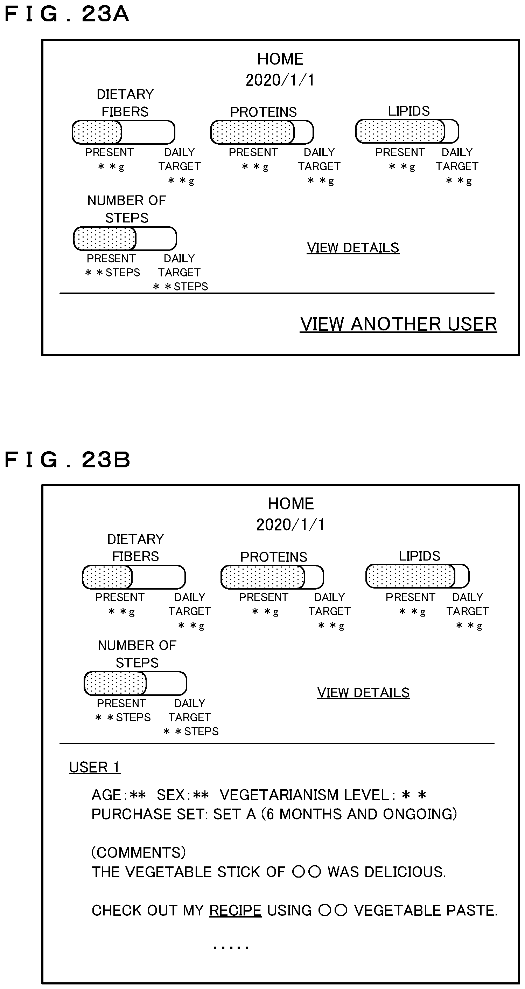

As described above, the server 10 classifies each user into a class based on user information and a configuration is adopted which enables information on each user to be shared among users of each class. FIG. 22 is a flow chart showing an example of a sharing processing procedure in a class, and FIGS. 23A and 23B are schematic views showing screen examples of the user terminal 20. In FIG. 22, processing performed by the user terminal 20 is shown on a left-hand side and processing performed by the server 10 is shown on a right-hand side. The processing described below is executed by the control unit 21 in accordance with the control program 22P and the advice app 22AP stored in the storage unit 22 of the user terminal 20 and executed by the control unit 11 in accordance with the control program 12P stored in the storage unit 12 of the server 10. A part of the processing described below may be realized by an exclusive hardware circuit.

In the information processing system 100 according to the present embodiment, when a user wishes to acquire information (shared information) related to another user classified into a same class as the user, the user requests the server 10 to share information related to the other user via the advice app 22AP. The user can make a request for information sharing via the home screen that is displayed when the user terminal 20 opens the advice app 22AP. For example, a home screen shown in FIG. 23A has a "view another user" button for requesting sharing of information related to another user. When the "view another user" button is operated on the home screen shown in FIG. 23A, the control unit 21 accepts a request instruction for information sharing. Therefore, after opening the advice app 22AP and displaying the home screen, the control unit 21 of the user terminal 20 determines whether or not a request instruction for information sharing has been accepted via the input unit 24 (S81). When the control unit 21 determines that a request instruction has not been accepted (S81: NO), the control unit 21 stands by while performing other kinds of processing.

When the control unit 21 determines that a request instruction for information sharing has been accepted (S81: YES), the control unit 21 makes a request for information sharing to the server 10 (S82). Specifically, the control unit 21 transmits the member ID of the user and a request signal for requesting information sharing to the server 10. Alternatively, the control unit 21 may make a request for information sharing to the server 10 once the advice app 22AP is started without accepting a request instruction for information sharing from the user. In addition, after starting the advice app 22AP (in other words, when the advice app 22AP is running), the control unit 21 may make a request for information sharing to the server 10 on a regular basis or every time a time of day set in advance arrives.

When the control unit 11 of the server 10 accepts a request for information sharing from the user terminal 20, the control unit 11 specifies a class into which the user of the user terminal 20 has been classified based on stored contents of the member information DB 12c (S83). Specifically, the control unit 11 specifies the class of the user of the user terminal 20 by reading the class ID stored in the member information DB 12c in association with the member ID acquired from the user terminal 20. In addition, the control unit 11 reads user information related to another user classified into the specified class from the member information DB 12c (S84). Specifically, the control unit 11 specifies a user for which the class ID of the specified class is stored in the member information DB 12c and reads the user information of the specified user from the member information DB 12c. When there are a plurality of users in a same class, user information of all of the users is read from the member information DB 12c. In addition, based on the read user information, the control unit 11 generates shared information to be shared between users classified into the same class (S85). For example, the control unit 11 generates shared information including attribute information, profile information, a purchase history of subscription food, information on diet including subscription food and general food, and information on actions in daily life such as exercise and sleep of the other user. Each user has set whether or not to disclose each piece of information included in user information registered in the server 10 to other users (disclosure or non-disclosure), and the control unit 11 generates shared information using only information set to disclosure. Accordingly, since information that a user does not wish to disclose to other users is not disclosed, personal information can be protected.

In addition, in the information processing system 100 according to the present embodiment, a configuration is adopted in which any comment can be input using the user terminal 20 and registered in the server 10 (member information DB 12c) in addition to various kinds of information related to a user. Therefore, the control unit 11 may generate shared information including a comment registered in the member information DB 12c. In addition, when contents of an evaluation made by the user with respect to advice acquired from the server 10 is stored in the member information DB 12c of the server 10, the control unit 11 may include the evaluation by the user with respect to the advice in the shared information. Using such shared information enables information regarding whether or not advice provided from the server 10 had been effective to be shared among users in the class. Due to the processing described above, the control unit 11 can generate shared information for each class (each user classification).

When the control unit 11 generates shared information in the class of the user having made a request for information sharing, the control unit 11 transmits the generated shared information to the user terminal 20 (S86). When the control unit 21 of the user terminal 20 receives the shared information transmitted by the server 10, as shown in FIG. 23B, the control unit 21 displays the received shared information on the home screen (S87). Compared to the home screen shown in FIG. 23A, the shared information received from the server 10 is additionally displayed on the home screen shown in FIG. 23B. The shared information shown in FIG. 23B includes an age, sex, a vegetarianism level, a purchase history of subscription food (a set name and a purchase period of a purchase set), and a comment of another user (user 1). By providing the user terminal 20 of users classified into each class with various kinds of information (shared information) of a user classified into each class according to the processing described above, the server 10 can share various kinds of information related to the user among users classified into the same class. Users classified into the same class are expected to have similarities with respect to eating habits, lifestyle habits, preference information, trends of thought, or the like. Therefore, by sharing information between similar users, it is anticipated that information beneficial for each user can be provided in an efficient manner.

In the information processing system 100 according to the present embodiment, the server 10 is not limited to a configuration in which the server 10 generates shared information to be provided to the user when information sharing is requested from the user terminal 20. For example, a configuration may be adopted in which the server 10 generates shared information in each class on a regular basis or when a time of day set in advance arrives. In this case, for example, when a predetermined period elapses or when a set time of day arrives, the control unit 11 of the server 10 generates, for each class, shared information to be shared between users of the class based on user information of users classified into the class, and stores the generated shared information in, for example, the classification condition DB in association with a class ID. In addition, when the server 10 is requested to share information from the user terminal 20, the server 10 reads shared information corresponding to the class of the user from the classification condition DB and provides the user terminal 20 with the read shared information. Even in this case, the shared information can be displayed on the home screen in a similar manner to FIG. 23B. Furthermore, when the server 10 generates shared information for each class, the server 10 may push a notification of the generated shared information to the user terminal 20 instead of storing the shared information in the classification condition DB. In this case, since the user terminal 20 can display the fact that new shared information has been received on the display unit 25, the user can be notified of the presence of new shared information. Therefore, a user can acquire shared information of the class into which the user has been classified without having to request information sharing and information that is highly likely to be of interest to the user can be obtained in an efficient manner.

In the present embodiment, each user is classified into a class based on user information according to the processing described above. When subscription food according to the present embodiment has been delivered to the user in advance (in particular, when the subscription food is articles of food corresponding to a certain period of time which are collectively delivered to the user or, in other words, when the subscription food is not "articles of food purchased by the user at each time of consumption"), the subscription food is food to be consumed according to a consumption desire of the user. Therefore, ingestion information of subscription food can be phrased as consumption information (ingestion information of food) that is faithful to the consumption desire of the user. Furthermore, including non-consumption information (non-ingestion information of subscription food) in addition to ingestion information enables even more unique and accurate analysis to be performed. In the present embodiment, since each user is classified into a class based on user information (particularly, preferably including non-ingestion information) including ingestion information (ingestion history) of subscription food, highly accurate class classification more suitable for the desire of the user can be performed. In addition, since each class into which a user is classified in this manner is a class that more appropriately reflects the desire of the user, providing advice in accordance with a class enables an optimal advice to be provided to the user. Furthermore, by sharing information between users classified into each class, information can be shared with a high degree of empathy.

In the present embodiment, a similar advantageous effect to the respective embodiments described above can be obtained. In addition, in the present embodiment, each user is classified into a class based on user information input via the user terminal 20 and an advice in accordance with the class can be provided. Therefore, advice contents in accordance with user information can be differentiated for each class and, at the same time, contents of various kinds of advice by an expert such as a physician in relation with lifestyle habits, stress, bodily functions, work efficiency, cognition, and the like can be differentiated for each class. Therefore, advice that is more suitable for the user can be specified and provided. The configuration according to the present embodiment can also be applied to the information processing system 100 according to the second and third embodiments, and a similar advantageous effect is obtained even when the configuration according to the present embodiment is applied to the information processing system 100 according to the second and third embodiments. Furthermore, the modifications described above in the respective embodiments can also be applied to the information processing system 100 according to the present embodiment.

### (Fifth embodiment, which is not encompassed by the wording of the claims but is useful for understanding the invention)

An information processing system in which the server 10 performs management (inventory management) of remaining subscription food that has not been eaten by the user among subscription food purchased by the user will be described. Since the information processing system according to the present embodiment can be realized by a similar apparatus to that of the information processing system 100 according to the first embodiment, a description of a configuration will be omitted. The server 10 according to the present embodiment manages types and purchase amounts of subscription food purchased by the user according to the purchase history stored in the member information DB 12c. In addition, the server 10 manages types and ingestion amounts (eaten amounts) of subscription food eaten by the user according to the ingestion history stored in the member information DB 12c. Therefore, since the server 10 can specify types and amounts of remaining subscription food for each user based on the purchase history and the ingestion history of each user, the server 10 can perform inventory management of subscription food for each user. When the server 10 according to the present embodiment acquires information on a meal (ingestion information) of a user from the user terminal 20, the server 10 updates a remaining amount of subscription food based on subscription food consumed in the meal. The member information DB 12c according to the present embodiment has a remaining amount column (not illustrated) that stores a remaining amount of subscription food in addition to the configuration of the member information DB 12c shown in FIG. 4, and when the server 10 specifies a remaining amount of subscription food of each user, the server 10 updates the remaining amount of subscription food that is stored in the member information DB 12c.

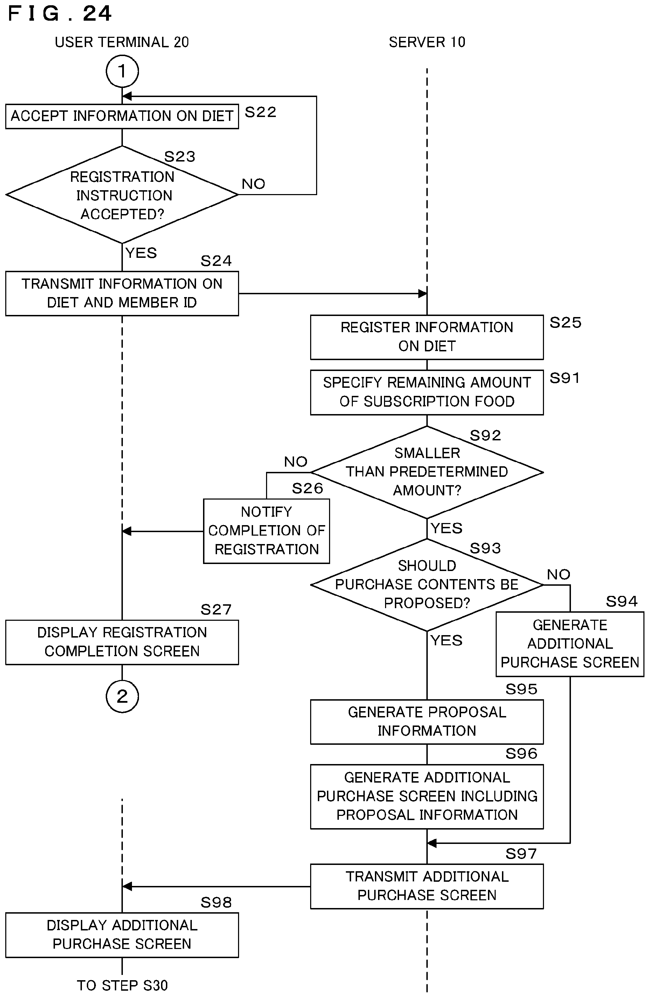

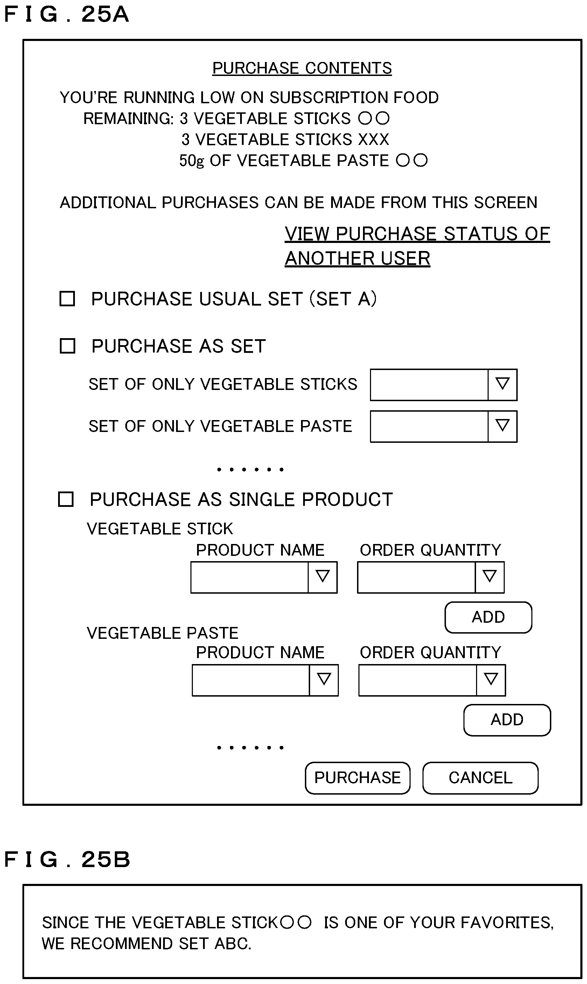

FIG. 24 is a flow chart showing an example of a registration processing procedure of user information according to a fifth embodiment, and FIGS. 25A and 25B are schematic views showing screen examples of the user terminal 20. The processing shown in FIG. 24 represents an addition of steps S91 to S98 between steps S25 and S26 in the processing shown in FIGS. 6 and 7. Descriptions of same steps as in FIGS. 6 and 7 will be omitted. In addition, in FIG. 24, illustration of each step in FIG. 6 and steps S28 to S35 in FIG. 7 is omitted.

In the information processing system 100 according to the present embodiment, the user terminal 20 and the server 10 perform processing similar to steps S11 to S25 in FIGS. 6 and 7. Accordingly, when the user terminal 20 accepts information (profile information, physical information, information on exercise, information on sleep, and information on diet) of the user input via the input screens shown in FIGS. 8B to 9B, the user terminal 20 transmits the accepted information to the server 10 and the server 10 registers the user information received from the user terminal 20 in the member information DB 12c. After the control unit 11 of the server 10 stores the information on diet acquired from the user terminal 20 in step S25 in the member information DB 12c, the control unit 11 specifies a remaining amount of subscription food at this point based on the information on diet that the control unit 11 stored in the member information DB 12c and purchase history already stored in the member information DB 12c (S91). For example, the control unit 11 specifies a remainder obtained by subtracting subscription food eaten (consumed) by the user from subscription food already purchased by the user as the remaining amount of subscription food. Therefore, the ingestion history stored in the member information DB 12c according to the present embodiment desirably represents types and amounts of consumption of subscription food eaten by the user. In addition, when a remaining amount of subscription food is stored in the member information DB 12c, an updated remaining amount of subscription food may be specified by subtracting the subscription food eaten by the user from the stored remaining amount.

The control unit 11 determines whether or not the specified remaining amount of subscription food is smaller than a predetermined amount (S92). For example, the control unit 11 may simply determine whether or not the remaining amount of subscription food is smaller than a predetermined amount set in advance, determine whether or not the remaining amount of subscription food is smaller than a predetermined ratio with respect to a purchased amount, or determine whether or not the remaining amount of subscription food is smaller than an amount (a predetermined amount) in accordance with the number of days until a scheduled date on which subscription food is to be next delivered to the user. The predetermined amount is desirably an amount that prevents the remaining amount of subscription food from becoming depleted before the next scheduled delivery date. In addition, the control unit 11 may determine whether or not the remaining amount of subscription food is an amount that does not become depleted before the next scheduled delivery date by also taking into consideration a pace of consumption of subscription food by the user. When the control unit 11 determines that the remaining amount of subscription food is larger than the predetermined amount (S92: NO), the control unit 11 makes a transition to the processing of step S26 and notifies the user terminal 20 of completion of registration of the information on diet (S26). Subsequently, the control unit 21 of the user terminal 20 performs processing of step S27 and thereafter.

When the control unit 11 determines that the remaining amount of subscription food is smaller than the predetermined amount (S92: YES), the control unit 11 provides the user terminal 20 with a screen for additionally purchasing subscription food. At this point, based on the purchase history and the ingestion history of the user, the control unit 11 determines whether or not a change to a product set (purchase contents) having been purchased by the user is to be proposed (S93). For example, when an amount of consumption of subscription food that is consumed by the user in a predetermined period is large with respect to an amount of products included in the product set purchased by the user (a purchased amount) and there is always a shortage of subscription food, the control unit 11 determines that a change to a product set with a larger number of products is to be proposed. Conversely, when an amount of consumption is small with respect to the product set purchased by the user and there is always a surplus of subscription food, the control unit 11 may determine that a change to a product set with a smaller number of products or a product set containing easy-to-eat products or products that can be readily used for cooking should be proposed.

When the control unit 11 determines that there is no need to propose a change to the purchase contents (S93: NO), the control unit 11 generates an additional purchase screen such as that shown in FIG. 25A (S94). The additional purchase screen shown in FIG. 25A has a similar configuration to the input screen of purchase contents shown in FIG. 10B and, further, has an input field (check box) for selecting an additional purchase (purchasing the usual set) of the product set currently being purchased. In addition, as shown in FIG. 25A, the additional purchase screen may display the remaining amount of subscription food specified in step S91. Accordingly, the user can readily comprehend the remaining articles of subscription food and readily determine whether or not an additional purchase is to be made. Furthermore, the additional purchase screen shown in FIG. 25A has a "view purchase status of another user" button for requesting sharing of information related to a product set purchased by another user. Note that the additional purchase screen is provided with a purchase button for requesting an additional purchase of subscription food according to input contents in place of the change button on the input screen shown in FIG. 10B. After generating the additional purchase screen, the control unit 11 makes a transition to processing of step S97.

On the other hand, when the control unit 11 determines that a change to the purchase contents should be proposed (S93: YES), the control unit 11 generates proposal information for proposing a change to the purchase contents (S95). For example, the control unit 11 specifies subscription food with a high consumption frequency by the user and generates proposal information for proposing a change to a product set containing the specified subscription food. In addition, the control unit 11 may specify subscription food that is likely to be preferred by the user based on the ingestion history of the user and may generate proposal information for proposing a change to a product set containing the specified subscription food. Accordingly, as shown in FIG. 25B, the control unit 11 generates a message recommending a product set that is highly likely to be purchased by the user. In addition, the control unit 11 generates an additional purchase screen including the generated proposal information (S96). In this case, the control unit 11 generates a screen in which proposal information such as that shown in FIG. 25B is displayed at, for example, the beginning of the additional purchase screen shown in FIG. 25A.

The control unit 11 transmits the additional purchase screen generated in step S94 or step S96 to the user terminal 20 (S97), and the control unit 21 of the user terminal 20 displays the additional purchase screen received from the server 10 on the display unit 25 (S98). When the server 10 determines that there is no need to propose purchase contents, an additional purchase screen such as that shown in FIG. 25A is displayed on the user terminal 20, but when the server 10 determines that purchase contents should be proposed, a screen created by displaying a message (proposal information) such as that shown in FIG. 25B on the additional purchase screen shown in FIG. 25A is displayed. After processing of step S98, the control unit 21 makes a transition to processing of step S30 and executes processing of step S30 and thereafter. In the present embodiment, in step S31, the control unit 21 determines whether or not an execution instruction (purchase instruction) of an additional purchase has been accepted based on whether or not the purchase button in the additional purchase screen has been operated via the input unit 24. In addition, when the control unit 21 determines that an execution instruction for an additional purchase has been accepted, the control unit 21 associates additional purchase contents having been input via the additional purchase screen and the member ID of the user with each other and transmits the associated information to the server 10 (S32), and requests the server 10 to execute the additional purchase. When the control unit 11 of the server 10 accepts an execution request for an additional purchase from the user terminal 20, the control unit 11 registers purchase contents of the additional purchase acquired from the user terminal 20 in the member information DB 12c as purchase history (S33), and further executes purchase processing related to the additional purchase. Although a detailed description of the purchase processing will not be given since purchase processing is processing related to a general purchasing procedure, for example, the purchase processing includes processing related to payment of a purchase price and processing related to arranging shipment of subscription food.

In addition, when the "view purchase status of another user" button is operated on the additional purchase screen, the control unit 21 accepts a sharing instruction of information related to a purchase status of another user and requests the server 10 to share information related to the purchase status. When the server 10 accepts a sharing request for information related to a purchase status from the user terminal 20, the server 10 reads information (purchase history) related to the purchase status of another user from the member information DB 12c and generates shared information related to the purchase status. The server 10 may specify a class into which a user having made the request is classified and generate shared information related to the purchase status from information (purchase history) related to a purchase status of another user classified into the specified class. In addition, by transmitting the generated shared information to the user terminal 20, the server 10 can provide a purchase status of another user.

In the present embodiment, a similar advantageous effect to the respective embodiments described above can be obtained. In addition, in the present embodiment, a remaining amount of subscription food purchased by each user can be managed and an additional purchase can be proposed to the user when a remaining amount decreases. Furthermore, in this case, by proposing a change to purchase contents with respect to a user whose purchase contents should be changed, sales promotion of subscription food can be achieved. The configuration according to the present embodiment can also be applied to the information processing system 100 according to the second to fourth embodiments, and a similar advantageous effect is obtained even when the configuration according to the present embodiment is applied to the information processing system 100 according to the second to fourth embodiments. Furthermore, the modifications described above in the respective embodiments can also be applied to the information processing system 100 according to the present embodiment.

### (Sixth embodiment, which is not encompassed by the wording of the claims but is useful for understanding the invention)

An information processing system in which the user terminal 20 performs management (inventory management) of a remaining amount of subscription food purchased by a user will be described. Since the information processing system according to the present embodiment can be realized by a similar apparatus to that of the information processing system 100 according to the first embodiment, a description of a configuration will be omitted. The user terminal 20 according to the present embodiment stores purchase history and ingestion history of subscription food in the storage unit 22. Accordingly, the user terminal 20 can manage types and purchase amounts of subscription food purchased by the user and types and ingestion amounts of subscription food eaten by the user. Therefore, the user terminal 20 according to the present embodiment can specify types and amounts of remaining subscription food based on the purchase history and the ingestion history of the user and perform inventory management of subscription food. The user terminal 20 according to the present embodiment acquires information on diet (ingestion information) of the user via the input unit 24 and, after registering the acquired ingestion information in the server 10, updates a remaining amount of subscription food.

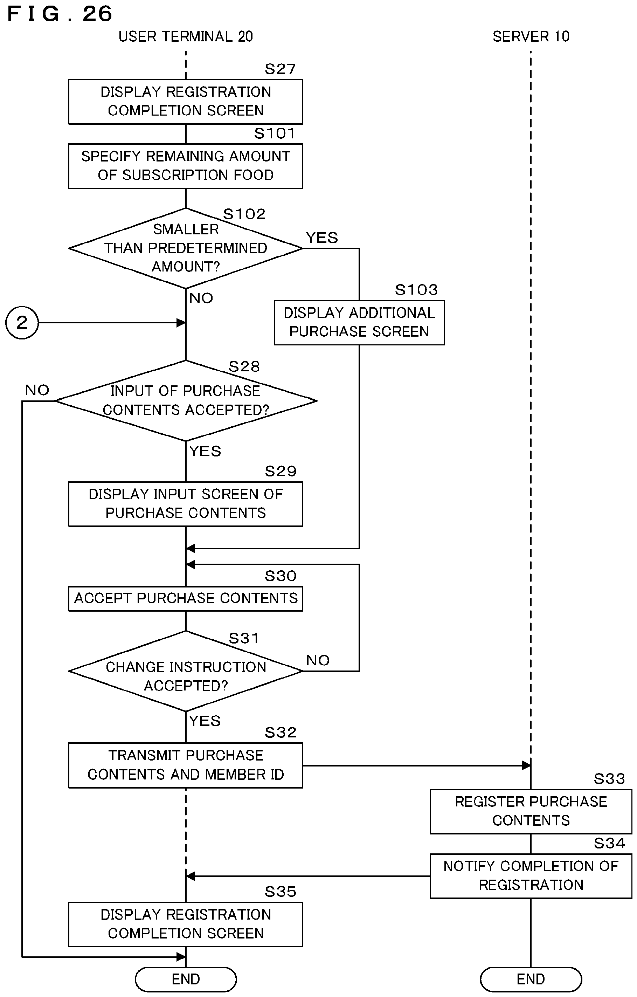

FIG. 26 is a flow chart showing an example of a registration processing procedure of user information according to a sixth embodiment. The processing shown in FIG. 26 represents an addition of steps S101 to S103 between steps S27 and S28 in the processing shown in FIGS. 6 and 7. Descriptions of same steps as in FIGS. 6 and 7 will be omitted. In addition, in FIG. 26, illustration of steps S11 to S26 in FIGS. 6 and 7 is omitted.

In the information processing system 100 according to the present embodiment, the user terminal 20 and the server 10 perform processing similar to steps S11 to S27 in FIGS. 6 and 7. Accordingly, when the user terminal 20 accepts information (profile information, physical information, information on exercise, information on sleep, and information on diet) of the user input via the input screens shown in FIGS. 8B to 9B, the user terminal 20 transmits the accepted information to the server 10. In addition, the server 10 registers the user information received from the user terminal 20 in the member information DB 12c. After displaying, in step S27, a screen indicating completion of registration of the information on diet notified from the server 10, the control unit 21 of the user terminal 20 specifies a remaining amount of subscription food at this point based on the purchase history and the ingestion history of subscription food stored in the storage unit 22 (S101). In this case, the control unit 21 can specify the remaining amount of subscription food by performing processing similar to step S91 in FIG. 24 that is performed by the control unit 11 of the server 10 in the fifth embodiment described above.

The control unit 21 determines whether or not the specified remaining amount of subscription food is smaller than a predetermined amount (S102). In this case, the control unit 21 determines whether or not the remaining amount of subscription food is smaller than a predetermined amount by performing processing similar to step S92 in FIG. 24 that is performed by the control unit 11 of the server 10 in the fifth embodiment described above. When the control unit 21 determines that the remaining amount of subscription food is larger than the predetermined amount (S102: NO), the control unit 21 makes a transition to the processing of step S28 and executes processing of step S28 and thereafter.

When the control unit 21 determines that the remaining amount of subscription food is smaller than the predetermined amount (S102: YES), the control unit 21 displays an additional purchase screen for additionally purchasing subscription food on the display unit 25 (S103). The additional purchase screen in this case has a similar configuration to the screen shown in, for example, FIG. 25A and notifies a remaining amount of subscription food and accepts a request for an additional purchase of subscription food. After displaying the additional purchase screen on the display unit 25, the control unit 21 makes a transition to processing of step S30 and executes processing of step S30 and thereafter. Accordingly, in the present embodiment, the control unit 21 accepts purchase contents or additional purchase contents via any of the input screen for purchase contents displayed in step S29 and the additional purchase screen displayed in step S103. In addition, the control unit 21 requests the server 10 to purchase subscription food in the accepted purchase contents or additional purchase contents and the server 10 executes purchase processing.

In the present embodiment, a similar advantageous effect to the respective embodiments described above can be obtained. In addition, in the present embodiment, a remaining amount of subscription food purchased by the user can be managed by the user terminal 20 and an additional purchase can be proposed to the user when a remaining amount decreases. By proposing an additional purchase of subscription food at a timing where the remaining amount has decreased, sales promotion of subscription food can be achieved. The configuration according to the present embodiment can also be applied to the information processing system 100 according to the second to fifth embodiments, and a similar advantageous effect is obtained even when the configuration according to the present embodiment is applied to the information processing system 100 according to the second to fifth embodiments. Furthermore, the modifications described above in the respective embodiments can also be applied to the information processing system 100 according to the present embodiment.

The information processing system 100 according to each of the embodiments described above may be provided with a component that automatically measures an amount of consumption (an ingestion amount) of subscription food by the user. For example, the information processing system 100 may be provided with a stocker that stores subscription food by type, a measuring instrument that measures a total weight of subscription food stored in each stocker, and a calculating unit which calculates, when the weight measured by the measuring instrument decreases, an amount of consumption of subscription food of this type in accordance with the decreased weight. For example, in the case of subscription food in the form of vegetable sticks, since a weight of one vegetable stick is known in advance, the number of consumed vegetable sticks can be calculated from the decreased weight. In addition, in the case of subscription food in the form of vegetable paste, the decreased weight is adopted as-is as an amount of consumption of the vegetable paste. Providing such components enables, when the user takes out subscription food from a stocker, a type and an amount (an amount of consumption) of the subscription food that had been taken out to be specified. Therefore, the user need not input ingestion information of subscription food via the user terminal 20 and an operation burden can be reduced. Alternatively, a measurement result by the measuring instrument can be transmitted to the user terminal 20 and the user terminal 20 may calculate an amount of consumption of subscription food based on the measurement result. In addition, the user terminal 20 may transmit a measurement result acquired from the measuring instrument to the server 10 and the server 10 may calculate an amount of consumption of subscription food based on the acquired measurement result and a previous measurement result. A configuration may be adopted in which a remaining amount of subscription food is calculated based on a weight measured by a measuring instrument instead of calculating an amount of consumption of subscription food. Even in this case, the amount of consumption of subscription food can be specified based on a change in the remaining amount of subscription food.

### (Seventh embodiment, which is not encompassed by the wording of the claims but is useful for understanding the invention)

An information processing system that proposes ingestion of subscription food to the user based on a target ingestion amount of the user with respect to a predetermined nutrient or a target contribution amount of the user toward earth's environment will be described. While subscription food to be proposed to the user is to be specified based on a target ingestion amount or a target contribution amount in a predetermined period such as a month in the present embodiment, the predetermined period is not limited to a month. In particular, in a case where a target value according to the present disclosure represents a target that is difficult to achieve (for example, a target related to a social problem that is difficult to solve unless public agencies take some kind of action and, as more specific examples, a target which is achievable by ingestion of food but which is difficult to achieve to such an extent that a public agency sets forth a numerical target to recommend or deter ingestion such as "a nutrient of which a deficiency affects maintaining and promoting health" or "a nutrient of which excessive ingestion affects maintaining and promoting health"), the present disclosure preferably adopts food suitable for achieving the target and a combination of the food, sets a plurality of intermediate targets, and enables a user to achieve such targets. In particular, given that eating is something that is done continuously every day, continuous consumption of any article of food is difficult due to a loss of interest unless sufficient variation is secured, there is a problem in that known articles of food (for example, supplements) with a high degree of contribution toward difficult-to-achieve targets do not offer much in terms of variation in taste and menus using such articles of food tend to be similar to each other. However, according to the present disclosure, articles of food which is highly relevant to an article of food that interests a consumer and which has a high degree of contribution toward a target can be adopted and an achievement method which is less likely to cause the consumer to lose interest and which has continuity can be provided. In particular, by outputting an achievement method including cooking methods and ingestion methods which focuses on adopting articles of food such as noodles, breads, and artificial rice which are used as staple foods, which has a high degree of contribution toward a target and which are, specifically, processed pulse products, an achievement method with continuity can be more preferably presented while securing diversity. Since the information processing system according to the present embodiment can be realized by a similar apparatus to that of the information processing system 100 according to the first embodiment, a description of a configuration will be omitted. The storage unit 12 of the server 10 according to the present embodiment stores a target amount DB 12f in addition to the DBs 12a to 12d shown in FIGS. 3A to 5.

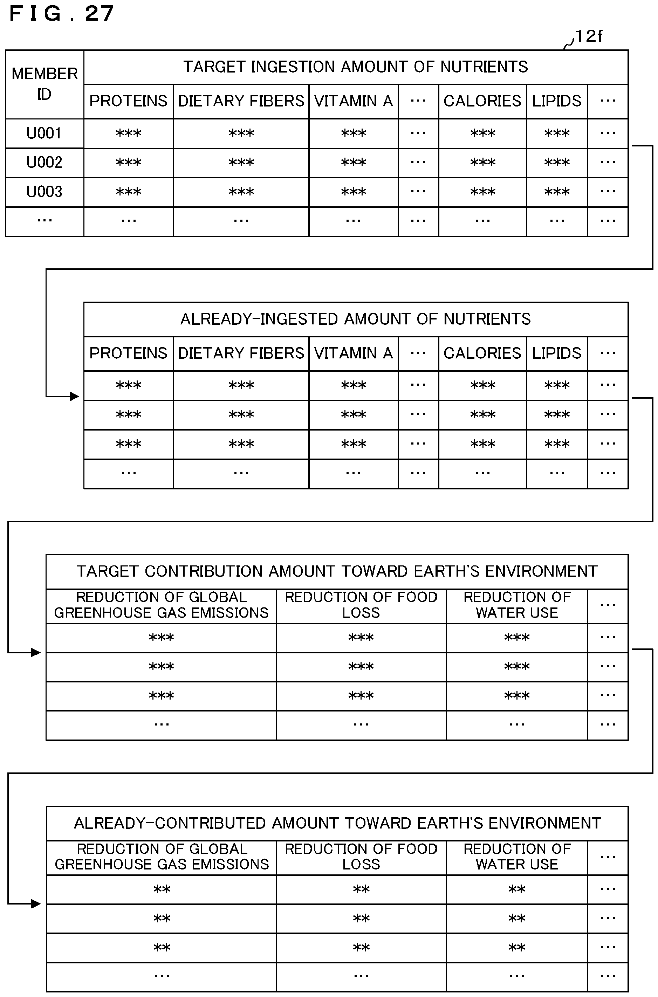

FIG. 27 is a schematic view showing a configuration example of the target amount DB 12f. The target amount DB 12f stores a target ingestion amount and an already-ingested amount of the user in a predetermined period with respect to a predetermined nutrient and a target contribution amount and an already-contributed amount of the user in a predetermined period with respect to earth's environment. The target amount DB 12f shown in FIG. 27 includes a member ID column, a target ingestion amount column and an already-ingested amount column of a nutrient, a target contribution amount column and an already-contributed amount column of earth's environment, and the like and stores, in association with a member ID, target amounts (a target ingestion amount and a target contribution amount) set by each user and achieved amounts (an already-ingested amount and an already-contributed amount) with respect to the target amounts. The member ID column stores a member ID of a user who is registered as a member. The target ingestion amount column of a nutrient stores a target ingestion amount and a target ingestion caloric amount (target ingestion calories) set by the user with respect to a predetermined nutrient. Examples of the predetermined nutrient include a nutrient of which a deficiency affects maintaining and promoting health and a nutrient of which excessive ingestion affects maintaining and promoting health. Examples of "a nutrient of which a deficiency affects maintaining and promoting health" (or "a nutrient of which excessive ingestion affects maintaining and promoting health") according to the present embodiment include nutrients that fall under the category of nutrients of which "a claim that supplementation is possible (emphasize that the amount of the nutrient is large)" or "a claim that appropriate ingestion is possible (emphasize that the amount of the nutrient or a calorific value of the nutrient is low)" can be made among nutrients defined as "nutrients and the like of which a deficiency or excessive ingestion affects maintaining and promoting public health" in The Nutrition Information Label Handbook (issued August 2019, Food Safety Control Section, Health and Safety Division, Bureau of Social Welfare and Public Health, Tokyo Metropolitan Government). Specifically, examples of "a nutrient of which a deficiency affects maintaining and promoting health" include proteins, dietary fiber, zinc, potassium, calcium, iron, copper, magnesium, niacin, pantothenic acid, biotin, vitamin A, vitamin B1, vitamin B2, vitamin B6, vitamin B12, vitamin C, vitamin D, vitamin E, vitamin K, and folic acid. In addition, examples of "a nutrient of which excessive ingestion affects maintaining and promoting health" include calories, lipids, saturated fatty acids, trans-fatty acids, cholesterol, sugars, and sodium. Therefore, the target ingestion amount column of a nutrient stores a target ingestion amount set by the user with respect to at least one of the nutrients described above and the already-ingested amount column of the nutrient stores an amount already ingested by the user with respect to each nutrient such as those described above.

The target contribution amount column of earth's environment stores a target contribution amount set by the user regarding a contribution toward earth's environment. The earth's environment according to the present disclosure refers to the totality of external things such as the home, society, and nature that surround human beings and animals on this Earth and is a concept of the environment in the broad sense of the term which not only includes the natural environment as exemplified by the marine environment and the atmospheric environment but also encompasses the social environment, and the term will be used in the same sense in the present embodiment. More specifically, representative examples of a target with respect to earth's environment according to the present disclosure include any one or a plurality of the 17 goals, 169 targets, and 232 indicators among the Sustainable Development Goals (SDGs) which are global goals to "achieve a better and more sustainable future for all" until 2030, described in "Transforming our world: the 2030 Agenda for Sustainable Development" adopted by the UN General Assembly in September, 2015. More specifically, examples include items such as a reduction of global greenhouse gas emissions (in particular, a reduction of carbon dioxide emission and, further, a reduction of a carbon footprint), a reduction of the amount of industrial waste (zero emission, improvements in recycling rates and resource recovery rates), a reduction of food loss (in particular, a reduction in discarded amounts and, further, a reduction in a discarded amount of inedible parts of food), and effective utilization of water resources (in particular, a reduction of water use or a reduction of drainage), in which case these goals can be used as contribution target items with respect to earth's environment in the present embodiment, and a target value set for each target item can be adopted as a target numerical value with respect to each target item in the present embodiment. Therefore, the column of a target contribution amount of earth's environment stores a target contribution amount set by the user with respect to at least one of the items described above and the column of an already-contributed amount of earth's environment stores an amount (reduction) already contributed by the user toward each of the items described above. As the member ID stored in the target amount DB 12f, for example, when a new member is registered in the member information DB 12c, the registered member ID is stored by the control unit 11. The target amounts (the target ingestion amount and the target contribution amount) stored in the target amount DB 12f are stored by the control unit 11 when the control unit 11 acquires each target amount via the communication unit 13. As the achieved amounts (the already-ingested amount and the already-contributed amount) stored in the target amount DB 12f, when the control unit 11 acquires information related to food (subscription food and general food) ingested by the user via the communication unit 13, the control unit 11 specifies an achieved amount due to food ingested by the user and stores the specified amount. Stored contents of the target amount DB 12f are not limited to the example shown in FIG. 27 and items related to nutrients and contributions toward earth's environment to be stored in the target amount DB 12f are not limited to the examples described above.

In the information processing system 100 according to the present embodiment, the user terminal 20 and the server 10 perform registration processing of user information similar to the processing shown in FIGS. 6 and 7. Accordingly, the user terminal 20 can accept user information such as attribute information, profile information, biological information, exercise information, sleep information, and ingestion information (information on diet) of the user and the server 10 can register the user information input via the user terminal 20 in the member information DB 12c. In the present embodiment, the user terminal 20 performs processing of accepting, in addition to the user information described above, the target ingestion amount of the user with respect to nutrients and the target contribution amount of the user with respect to earth's environment and registering the information in the server 10 (the target amount DB 12f). Therefore, in the present embodiment, an input screen (registration screen) of user information slightly differs from the configuration according to the first embodiment shown in FIG. 8A and registration processing of user information slightly differs from the processing shown in FIGS. 6 and 7.

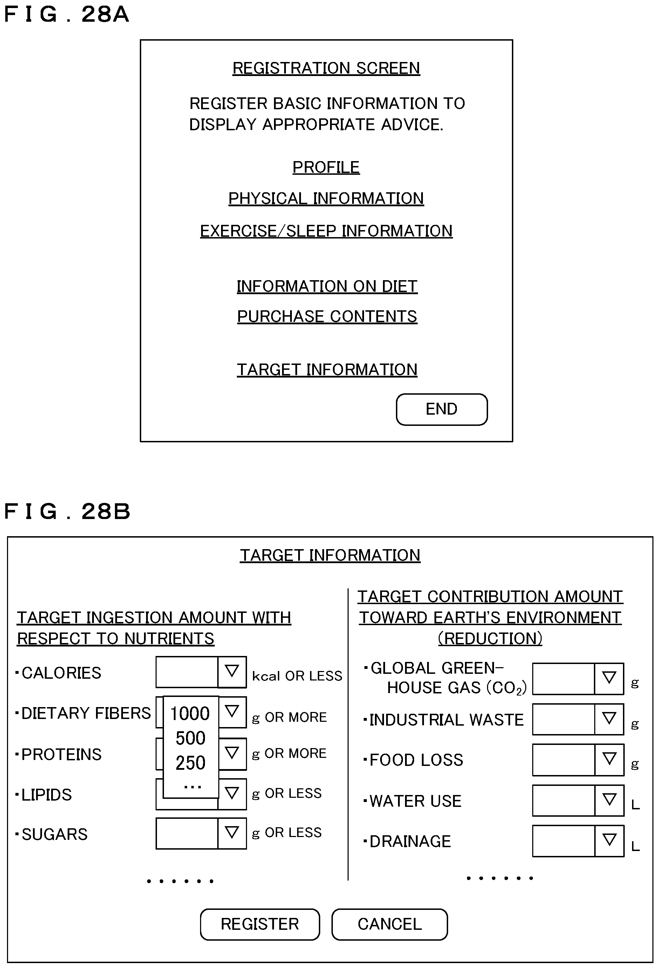

FIG. 28A is a schematic view showing a registration screen example according to the seventh embodiment, and FIG. 28B is a schematic view showing an input screen example of target information. The registration screen according to the present embodiment is configured such that target information of the user can be selected as user information to be input (registered) in addition to the configuration shown in FIG. 8A. When target information is selected on the screen shown in FIG. 28A, the control unit 21 of the user terminal 20 displays an input screen of target information such as that shown in FIG. 28B on the display unit 25. The input screen of target information shown in FIG. 28B has an input field for inputting a target ingestion amount with respect to a nutrient set in advance and an input field for inputting a target contribution amount with respect to earth's environment set in advance. The input field of the target ingestion amount and the input field of the target contribution amount are each provided with a pull-down menu that enables any of numerical values (amounts) set in advance with respect to each nutrient or each contribution item toward earth's environment to be selected. Each selectable numerical value may be determined from a dietary habit, lifestyle habits, and the like of the user. Alternatively, an article of subscription food can be selected and a selectable numerical value can be determined from a nutrient contained in the food. In addition, the input field of the target ingestion amount and the input field of the target contribution amount may be configured so that any numerical value can be input to the input fields. On the input screen of target information, registered contents may be displayed with respect to a target amount already registered by the user. In the case of such a configuration, the user can check the target amount already registered (or check a registration history in a time series) and, at the same time, the user can change the target amount already registered if the user wishes to do so. Alternatively, a set period for achieving a target amount can be selected or the target amount and the set period can be selected in plurality. Specifically, a daily target ingestion amount can be set, a target ingestion amount can be set for breakfast, lunch, dinner, or a between-meal snack, or a target ingestion amount can be set for each time slot. Alternatively, a weekly target ingestion amount can be set for each day of the week and the setting for each day of the week may be an iterative setting of the daily target ingestion amount. Furthermore, an individual setting can also be adopted such as setting a special day in conjunction with a calendar of the user or the like. On the input screen of target information shown in FIG. 28B, "or more" or "or less" is displayed in association with the input field of the target ingestion amount with respect to a nutrient. Alternatively, an achievement period of the target ingestion amount can be set, and the set period described above can be set in plurality and, for example, selected in stages. Specifically, "or less" is displayed with respect to calories, lipids, and sugars and "or more" is set with respect to dietary fiber and proteins. Accordingly, with respect to nutrients of which a deficiency affects maintaining and promoting health such as dietary fiber and proteins, a lower limit value is input to the input field and a value equal to or larger than the input value is accepted as the target ingestion amount. On the other hand, with respect to nutrients of which excessive ingestion affects maintaining and promoting health such as calories, lipids, and sugars, an upper limit value is input to the input field and a value equal to or smaller than the input value is accepted as the target ingestion amount. Note that "or more" or "or less" with respect to each nutrient can be changed. In addition, with respect to items of contributions toward earth's environment, a lower limit value is input to the input field and a value equal to or larger than the input value is accepted as a target contribution amount.

On the input screen shown in FIG. 28B, the user selects a nutrient or an item of a contribution toward earth's environment for which the user wishes to set a target and inputs a target amount in a corresponding input field. When the register button is operated on the input screen of target information, the control unit 21 of the user terminal 20 associates the target information (a target ingestion amount with respect to a nutrient or a target contribution amount with respect to a contribution item toward earth's environment) input via the input screen and the member ID of the user with each other and transmits the associated information to the server 10, and instructs the server 10 to register the target information. The control unit 11 (a target ingestion amount acquiring unit or a target contribution amount acquiring unit) of the server 10 acquires target information from the user terminal 20, associates the acquired target information with the member ID, and stores the associated information in the target amount DB 12f (a target ingestion amount storage unit or a target contribution amount storage unit). Accordingly, the target ingestion amount with respect to a nutrient or the target contribution amount with respect to a contribution item toward earth's environment having been input by the user via the user terminal 20 is registered in the server 10 (the target amount DB 12f).

In addition, in the present embodiment, when the server 10 receives ingestion information (information on diet) from the user terminal 20, the server 10 performs processing of specifying an ingestion amount (an already-ingested amount) of each nutrient due to food ingested by the user and a contribution amount (an already-contributed amount) with respect to earth's environment and registering the specified ingestion amount and the contribution amount in the target amount DB 12f. Specifically, after processing of step S25 in FIG. 7, based on information on diet acquired from the user terminal 20, the control unit 11 of the server 10 specifies a nutrient amount ingested by the user and a contribution amount (achievement amount) with respect to earth's environment due to the meal. For example, a nutrient amount and a contribution amount (achievement amount) with respect to earth's environment which can be achieved when eating each article of food (including subscription food and general food) are to be registered in the storage unit 12 or another server in advance. In addition, the control unit 11 extracts food included in a dietary content of the user and acquires a nutrient amount and a contribution amount toward earth's environment which correspond to each article of food from the storage unit 12 or another server. The control unit 11 stores the acquired achievement amount in the target amount DB 12f in association with the member ID. The control unit 11 adds the acquired achievement amount to the already-ingested amount and the already-contributed amount which are already stored in the target amount DB 12f and stores the obtained sums in the target amount DB 12f. Accordingly, the nutrient amount and the contribution amount toward earth's environment achieved by the user during a predetermined period (for example, a month) can be sequentially updated and stored.

As described above, in the present embodiment, a target ingestion amount with respect to nutrients and a target contribution amount with respect to earth's environment are set in the server 10. In addition, the ingestion amount of nutrients and a contribution amount toward earth's environment which are achieved by the user by eating meals are accumulated in the server 10 every time ingestion information (information on diet) of the user is transmitted from the user terminal 20 to the server 10. In the configuration described above, when the user selects subscription food displayed on a website provided by the server 10 while viewing the website, the server 10 according to the present embodiment performs processing of proposing (advising) subscription food that is more suitable for the user based on the selected subscription food and the target amounts set by the user.

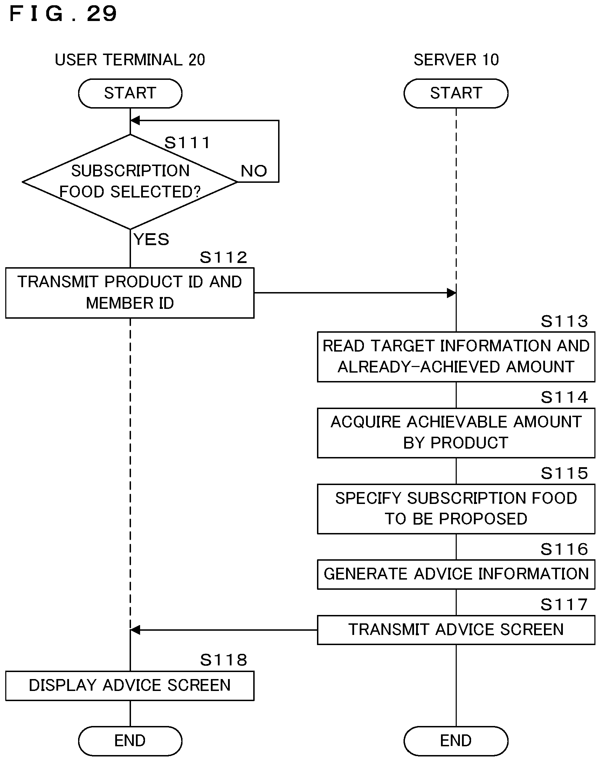

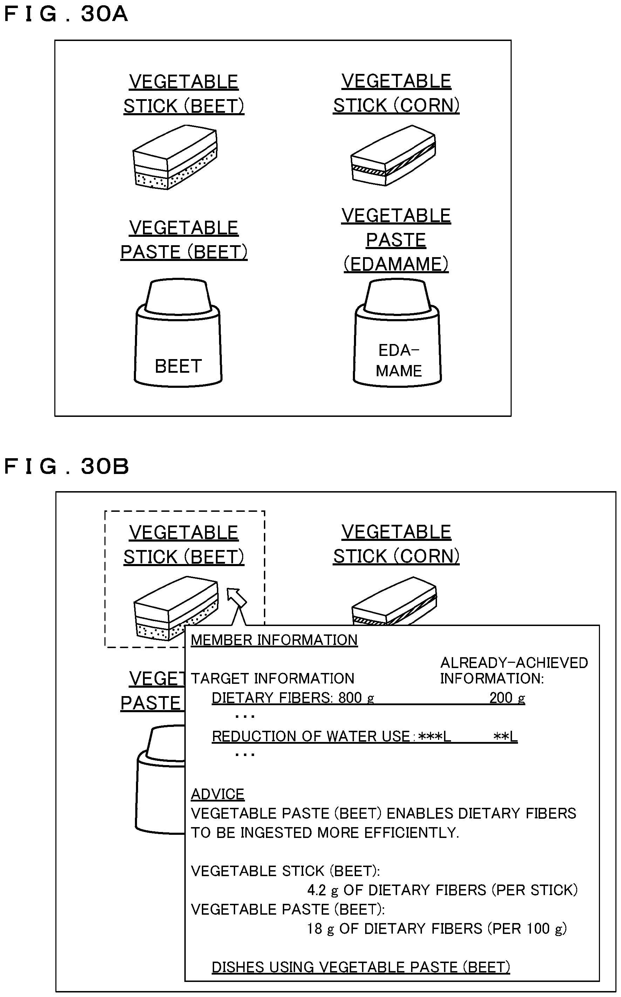

FIG. 29 is a flow chart showing an example of a provision processing procedure of an advice according to the seventh embodiment, and FIGS. 30A and 30B are schematic views showing a screen example of the user terminal 20. In FIG. 29, processing performed by the user terminal 20 is shown on a left-hand side and processing performed by the server 10 is shown on a right-hand side. The processing described below is executed by the control unit 21 in accordance with the control program 22P and the advice app 22AP stored in the storage unit 22 of the user terminal 20 and executed by the control unit 11 in accordance with the control program 12P stored in the storage unit 12 of the server 10. A part of the processing described below may be realized by an exclusive hardware circuit.

In the information processing system 100 according to the present embodiment, for example, while viewing a website made public by the server 10 via a network N using the user terminal 20, the user selects, using the input unit 24, an article of subscription food of interest among the articles of subscription food displayed on the website. For example, as shown in FIG. 30A, on the website displaying a plurality of types of subscription food, the user selects any article of food. The control unit 21 of the user terminal 20 has determined whether or not any of the articles of subscription food has been selected via the website being displayed (S111), and when the control unit 21 has determined that an article of subscription food has not been selected (S111: NO), the control unit 21 stands by while performing other processing. When the control unit 21 has determined that any of the articles of subscription food has been selected (S111: YES), the control unit 21 transmits a product ID of the selected subscription food and a member ID of the user to the server 10 (S112). The product ID is described in, for example, the website, and the member ID of the user is set in, for example, the advice app 22AP.

When the control unit 11 of the server 10 receives the product ID and the member ID from the user terminal 20, the control unit 11 reads target information (the target ingestion amount of a nutrient and a target contribution amount toward earth's environment) and already-achieved amounts (the already-ingested amount of the nutrient and the already-contributed amount toward earth's environment) which are stored in the target amount DB 12f in association with the member ID (S113). The control unit 11 need only read target amounts and already-achieved amounts with respect to a nutrient and a contribution item toward earth's environment of which a target amount (a target ingestion amount or a target contribution amount) is registered. Next, the control unit 11 acquires a nutrient amount and a contribution amount toward earth's environment (achievable amounts) which can be achieved by eating the subscription food selected by the user (S114). A nutrient amount and a contribution amount toward earth's environment which can be achieved by eating each article of subscription food are stored in advance in, for example, the product information DB 12a, and the control unit 11 reads achievable amounts corresponding to the product ID from the product information DB 12a. Alternatively, achievable amounts corresponding to each article of subscription food may be stored in another server and, in this case, the control unit 11 may acquire achievable amounts corresponding to each article of subscription food from the other server.

Next, the control unit 11 specifies subscription food to be proposed to the user based on the target information and the already-achieved amounts read in step S113 and the achievable amounts acquired in step S114 (S115). For example, with respect to each article of subscription food being a sales object, the control unit 11 (achieved ingestion amount acquiring unit and achieved contribution amount acquiring unit) acquires a nutrient amount and a contribution amount toward earth's environment (achievable amounts) which can be achieved by eating the subscription food. Even in this case, the control unit 11 acquires achievable amounts corresponding to each article of subscription food from the product information DB 12a or another server. In addition, the control unit 11 calculates a remaining target ingestion amount by subtracting the already-ingested amount of the user from the target ingestion amount of the user with respect to a nutrient and specifies subscription food that should be ingested in order to achieve (realize) the remaining target ingestion amount. For example, with respect to a nutrient for which a value of a predetermined amount or more is set as the target ingestion amount, the control unit 11 extracts subscription food with a larger achievable nutrient amount than the article of subscription food selected by the user among the articles of subscription food being a sales object and specifies the extracted article of subscription food as the article of subscription food to be proposed. At this point, when the remaining target ingestion amount is larger than a nutrient amount that is achievable by the subscription food selected by the user, an article of subscription food to be proposed may be specified. In addition, with respect to a nutrient for which a value of a predetermined amount or less is set as the target ingestion amount, the control unit 11 extracts subscription food with a smaller achievable nutrient amount than the article of subscription food selected by the user among the articles of subscription food being a sales object and specifies the extracted article of subscription food as the article of subscription food to be proposed. In this case, when the remaining target ingestion amount is smaller than a nutrient amount that is achievable by the subscription food selected by the user, an article of subscription food to be proposed may be specified. In a similar manner, the control unit 11 calculates a remaining target contribution amount by subtracting the already-contributed amount of the user from the target contribution amount of the user with respect to earth's environment and specifies subscription food that should be ingested in order to achieve (realize) the remaining target contribution amount. For example, the control unit 11 extracts subscription food with a larger achievable contribution amount than the article of subscription food selected by the user among the articles of subscription food being a sales object and specifies the extracted article of subscription food as the article of subscription food to be proposed. Even in this case, when the remaining target contribution amount is larger than a contribution amount that is achievable by the subscription food selected by the user, an article of subscription food to be proposed may be specified. When target amounts are set with respect to a plurality of nutrients or a plurality of contribution items toward earth's environment, the control unit 11 specifies an article of subscription food that enables each target amount to be approached to a greater extent as the subscription food to be proposed. In addition, the control unit 11 may specify subscription food suitable for the user's preference as subscription food to be proposed by also taking preference information (dietary preferences) of the user into consideration.

In addition, when a content of "a nutrient of which a deficiency affects maintaining and promoting health" in subscription food according to the present embodiment enables a "high" claim, a "source" claim, or an "increased" claim to be made and/or a content of "a nutrient of which excessive ingestion affects maintaining and promoting health" in subscription food according to the present embodiment enables a "low" claim, a "free" claim, or a "reduced" claim to be made, an article of subscription food that enables a nutrient with high user acceptability to be ingested in an appropriate amount can be specified. For example, when a high target ingestion amount is set with respect to proteins and/or dietary fiber and a low target ingestion amount is set with respect to calories, it is preferable to specify an article of food of which a content of proteins and/or dietary fiber enables a "high" claim, a "source" claim, or an "increased" claim to be made (more specifically, the article of food: preferably contains 8 percent by mass or more of dietary fiber; preferably has a dietary fiber content ratio relative to sugars of 0.15 or more; and preferably is an article of food of which a main raw ingredient is processed pulse products (in particular, noodles, breads, and artificial rice which are used as staple foods)) and of which a content of calories enables a "low" claim, a "free" claim, or a "reduced" claim to be made.

The control unit 11 (generating unit) generates advice information such as that shown in FIG. 30B based on the subscription food specified in step S115 (S116). FIG. 30B shows an advice screen example that is displayed when the vegetable stick "beet" is selected by the user on the screen shown in FIG. 30A. The advice screen shown in FIG. 30B displays a target ingestion amount of dietary fiber and a target reduction amount of water use as target information set by the user and an already-ingested amount of dietary fiber and a reduction amount of water use as already achieved information. In addition, the screen shown in FIG. 30B displays a message proposing a vegetable paste "beet" that enables dietary fiber to be more efficiently ingested than the selected vegetable stick "beet" and information that compares a dietary fiber amount due to the vegetable stick "beet" selected by the user and a dietary fiber amount due to the proposed vegetable paste "beet". When the proposed subscription food has a larger contribution amount toward earth's environment than the subscription food selected by the user, a message proposing the other subscription food with a larger contribution amount toward earth's environment than the selected subscription food is displayed on the screen shown in FIG. 30B. In doing so, information comparing the contribution amount toward earth's environment by the subscription food selected by the user and the contribution amount toward earth's environment by the proposed subscription food may be displayed. In addition, a link for displaying a menu (an ingestion method and a cooking method) using the proposed article of subscription food is set on the screen shown in FIG. 30B, and a menu using the proposed article of subscription food is provided via the link. Alternatively, a menu may be displayed using the proposed article of subscription food on the screen shown in FIG. 30B.

The control unit 11 transmits an advice screen displaying the generated advice information to the user terminal 20 (S117). The control unit 21 of the user terminal 20 receives the advice screen from the server 10 and displays an advice screen such as that shown in FIG. 30B on the display unit 25 (S118). While the user terminal 20 displays the advice screen received from the server 10 as shown in FIG. 30B by superimposing the advice screen on the display screen shown in FIG. 30A, this configuration is not restrictive. When the user selects any article of subscription food while viewing a website using the user terminal 20 according to the processing described above, the server 10 can specify an article of subscription food that can better achieve target information of the user and provide the user with an advice. Accordingly, the user can be provided with advice for achieving a target set by the user himself/herself.

In the present embodiment, a similar advantageous effect to the respective embodiments described above can be obtained. In addition, in the present embodiment, when the user selects any article of subscription food via a viewed image, the user can receive advice for achieving target information of the user. In doing so, an achievable amount with respect to a target of the user can be provided for the article of subscription food selected by the user and the proposed article of subscription food. Therefore, the user can take a degree of achievability with respect to the user's own target into consideration when evaluating purchase and ingestion of subscription food. Furthermore, a degree of achievement in an achievement plan during an arbitrary period set by the user can be checked and advice can be received and considered for the purpose of realizing the achievement plan. The configuration according to the present embodiment can also be applied to the information processing system 100 according to the second to sixth embodiments, and a similar advantageous effect is obtained even when the configuration according to the present embodiment is applied to the information processing system 100 according to the second to sixth embodiments. When the configuration according to the present embodiment is applied to the fourth embodiment described above, class classification according to a target ingestion amount with respect to a nutrient or a target contribution amount with respect to earth's environment by each user can be performed. Furthermore, the modifications described above in the respective embodiments can also be applied to the information processing system 100 according to the present embodiment.

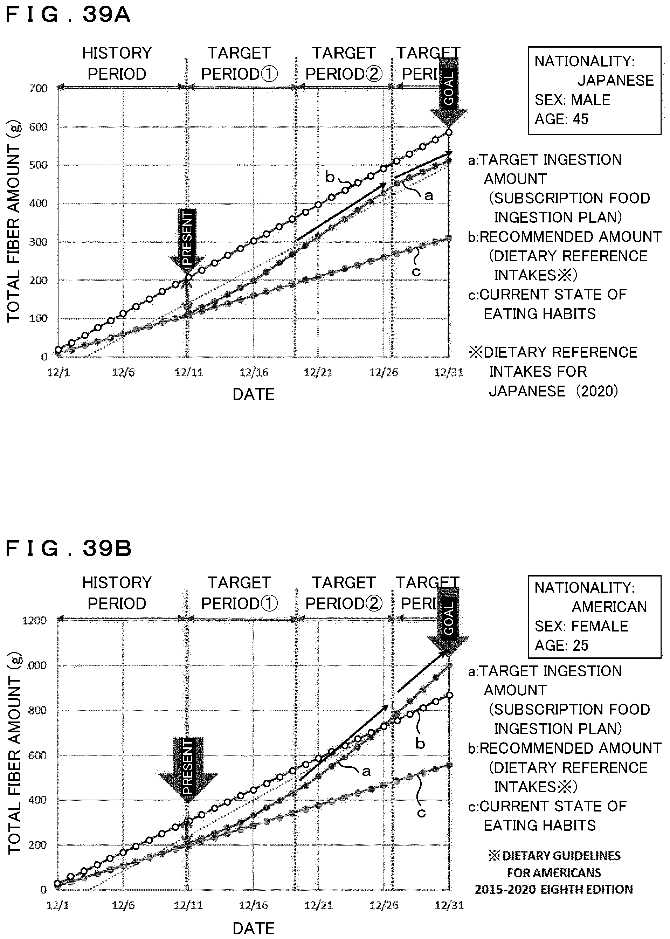

The present embodiment is not limited to a configuration in which the user himself/herself sets target information. For example, a target ingestion amount with respect to each nutrient and a target contribution amount with respect to a contribution item toward earth's environment can be registered in advance in the storage unit 12 or another server in accordance with information such as an age, sex, a birthplace (country or region), religion, a vegetarianism level, and a trend of preference related to diet (preference information). In this case, the control unit 11 of the server 10 may acquire a target amount in accordance with the information of the user described above from the storage unit 12 or another server and set the target amount as target information of the user. In addition, the control unit 11 may acquire target information (in particular, target information which is achievable by ingestion of food but which is difficult to achieve to such an extent that a public agency sets forth a numerical target to recommend or deter ingestion) in accordance with the user from a web server publishing such information and may set the target information. For example, as a target ingestion amount with respect to dietary fibers, ingestion amounts (medians) described in "Table 2: Values Referred to for Calculating DRIs for Dietary Fiber (g/day)" in "1-4 Carbohydrates, 4 Dietary Fiber" in "II Details, 1 Energy/Nutrients" in "Dietary Reference Intakes for Japanese (2020)" can be used. In addition, a target amount in accordance with a birthplace or a place of residence of the user may be acquired from an ingestion criterion of each nutrient disclosed in each country and used as target information. When such a configuration is adopted, a target amount in accordance with a state of the user can be automatically set. In addition, a target amount in accordance with a dietary habit or lifestyle habits of the user such as when the user is on a diet can be automatically set. Specifically, an ingestion criterion in accordance with a birthplace or a place of residence associated with a user ID can be selected by a system and an ingestion amount described in the ingestion criterion can be used. For example, as a target ingestion amount with respect to dietary fiber in the US, an ingestion amount described in "Dietary Fiber, g" that corresponds to an age and sex of the user from a list titled "Table A7-1. Daily Nutritional Goals for Age-Sex Groups Based on Dietary Reference Intakes & Dietary Guidelines Recommendations" in "Appendix 7. Nutritional Goals for Age-Sex Groups Based on Dietary Reference Intakes & Dietary Guidelines Recommendations" in "2015-2020 Dietary Guidelines for Americans" can be used. Alternatively, when the birthplace or the place of residence of the user is the UK, as a target ingestion amount with respect to dietary fiber, an ingestion amount described in "Age group" in accordance with an age of the user in a list titled "Dietary Fiber" in "Nutrition Requirements_Revised August 2019" can be selected and used. The ingestion criterion of each nutrient made public in each country is not limited to the above and the ingestion criterion can be acquired from data stored in the storage unit or information such as a website of administrative agencies or research institutions of each country being disclosed via the network N. Besides setting an ingestion criterion of the public agencies described earlier as a target ingestion amount, the ingestion criterion can be set as a recommended amount to be compared with a target ingestion amount of each user and the user can manually change a target value through a comparison with the recommended amount or the system can be configured to execute an advice with respect to a target value setting on the user based on a difference from the recommended amount. FIGS. 39A and 39B are schematic views showing a setting example of a target value. As shown in FIGS. 39A and 39B, when there is a difference between a recommended amount and a target ingestion amount of a user, the target ingestion amount may be changed to a value approaching the recommended amount based on the difference between the recommended amount and the target ingestion amount. By setting a target value based on a recommended amount with respect to the user, a target value suitable for the user can be set.

In addition, the server 10 may provide the user terminal 20 with target information acquired from another server or the like based on information on the user as described above and the user terminal 20 may display target information (each target amount) provided from the server 10 when displaying the input screen of target information shown in FIG. 28B. In this case, the user can comprehend general target information in the user's own state (an age, sex, a birthplace, religion, or the like) and appropriately set his/her own target information. Therefore, the user can set target information within an achievable range.

### (Eighth embodiment)

The seventh embodiment described above represents a configuration which, when a user selects an article of subscription food via a website being viewed by the user, proposes another article of subscription food. In the present embodiment, an information processing system will be described which, when a user selects an article of general food via a website, proposes an article of subscription food as a substitute ingredient. Since the information processing system according to the present embodiment can be realized by a similar apparatus to that of the information processing system 100 according to the seventh embodiment, a description of a configuration will be omitted. In the present embodiment, an ingestion history that is transmitted from the user terminal 20 to the server 10 in order to be stored in the member information DB 12c includes not only information on subscription food eaten by the user but also includes information on general food eaten by the user.

In the information processing system 100 according to the present embodiment, the user terminal 20 and the server 10 perform registration processing of user information similar to the processing shown in FIGS. 6, 7, and 14. Accordingly, the user terminal 20 can accept user information such as attribute information, profile information, biological information, exercise information, sleep information, and ingestion information (information on diet) of the user and the server 10 can register the user information input via the user terminal 20 in the member information DB 12c. In addition, the user terminal 20 and the server 10 according to the present embodiment perform registration processing of user information similar to that of the seventh embodiment described above and register a target ingestion amount of the user with respect to nutrients and a target contribution amount of the user with respect to earth's environment in the server 10 (the target amount DB 12f). Furthermore, in a similar manner to the seventh embodiment described above, when the server 10 according to the present embodiment receives ingestion information (information on diet) from the user terminal 20, the server 10 registers an ingestion amount (an already-ingested amount) of each nutrient due to food (subscription food and general food) ingested by the user and a contribution amount (an already-contributed amount) with respect to earth's environment in the target amount DB 12f.

Therefore, even in the present embodiment, a target ingestion amount with respect to nutrients and a target contribution amount with respect to earth's environment are set in the server 10 for each user and an ingestion amount of nutrients and a contribution amount toward earth's environment achieved by the user by eating meals are accumulated in the server 10. For example, when the user selects an article of food (general food) being displayed on any website while viewing the website or when information that enables an article of food to be directly or indirectly specified is displayed on a website and an article of food (general food) is selected by the system based on the displayed food information, the server 10 according to the present embodiment performs processing of advising subscription food suitable for the user based on general food selected by the user or general food selected by the system (both may be collectively referred to as "food selected by the user") and on a target amount set by the user.

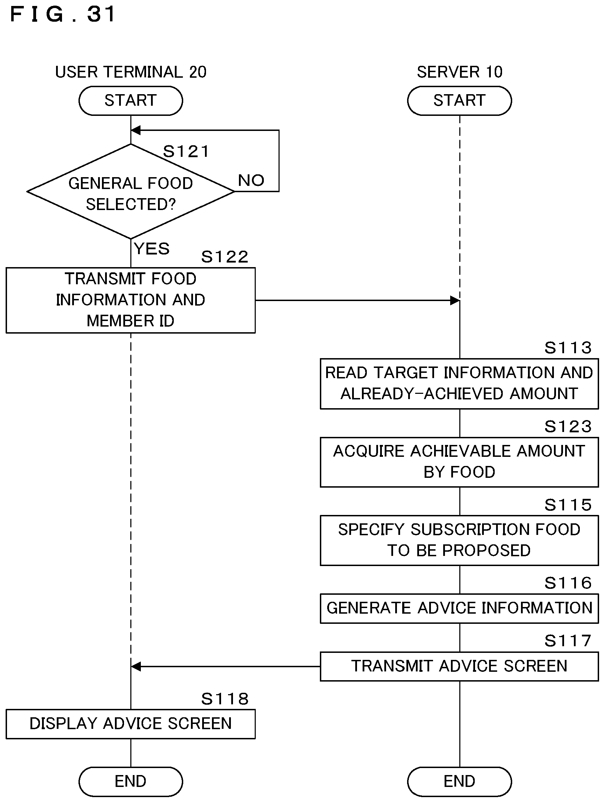

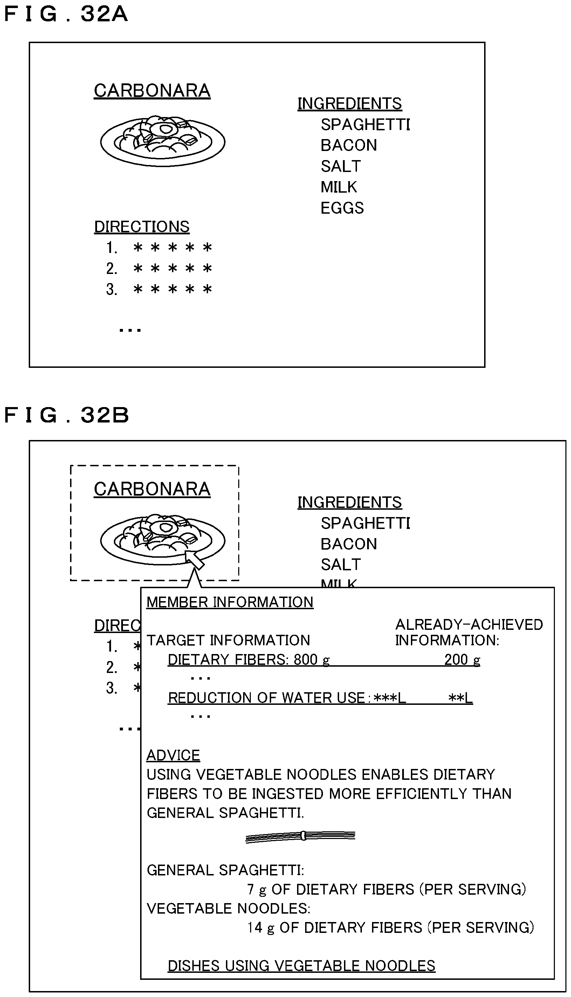

FIG. 31 is a flow chart showing an example of a provision processing procedure of an advice according to the eighth embodiment, and FIGS. 32A and 32B are schematic views showing a screen example of the user terminal 20. The processing shown in FIG. 31 represents an addition of steps S121 to S122 in place of steps S111 and S112 and an addition of step S123 in place of step S114 in the processing shown in FIG. 29. Descriptions of same steps as in FIG. 29 will be omitted. In the information processing system 100 according to the present embodiment, for example, while viewing any website made public via a network N using the user terminal 20, the user selects an article of general food of interest using the input unit 24. For example, on a website that discloses a recipe for carbonara as shown in FIG. 32A, the user selects a photograph of carbonara. Alternatively, the user may select an ingredient such as spaghetti, bacon, or the like from materials displayed on the website. The control unit 21 of the user terminal 20 has determined whether or not any of the articles of general food has been selected via the website being displayed (S121), and when the control unit 21 has determined that an article of general food has not been selected (S121: NO), the control unit 21 stands by while performing other processing.

When the control unit 21 has determined that any of the articles of general food has been selected (S121: YES), the control unit 21 transmits information on the selected article of food and a member ID of the user to the server 10 (S122). In this case, the control unit 21 specifies a type (menu), a dish name, or the like of the selected article of food and transmits the specified food information (specific information) to the server 10. As food information, for example, information that can be acquired from described contents on a website is used. Food information need only be information that enables an article of food to be directly or indirectly specified, and examples of food information include: literation of food (for example, a combination of characters "pa", "su", and "ta" (from which "pasta" is specified)); a photograph of food (for example, a photograph of pasta (from which "pasta" is specified)); vocalization of food (for example, a combination of sounds "pa", "su", and "ta" (from which "pasta" is specified)); an ingredient of food (for example, a flavor component characteristic of pasta (from which "pasta" is specified) or an amino acid composition characteristic of pasta (from which "pasta" is specified)); information that is generally highly relevant to food (for example, an image of a cow (from which "beef" and "milk", being articles of highly-relevant food, are specified) or a cat-type robot supposedly from the 22nd century (from which "Dorayaki", being an article of highly-relevant food, is specified); and information corresponding to an article of food in association information with the article of food set in advance (for example, a photograph of a gym (and association information created by the user or an AI (Artificial Intelligence), from which "low-sugar/high-protein food" is specified which is an article of corresponding food having been associated in advance). In addition, the food specified by specific information may be a specific article of food (for example, pasta), a group that is a superordinate concept to which the food belongs (for example, noodles), or food defined by characteristics related to target information of the food (for example, "low-sugar/high-protein food" or "food with reduced food loss". When the control unit 11 of the server 10 receives the food information and the member ID from the user terminal 20, the control unit 11 reads target information (the target ingestion amount of a nutrient and the target contribution amount toward earth's environment) and already-achieved amounts (the already-ingested amount of the nutrient and the already-contributed amount toward earth's environment) corresponding to the user from the target amount DB 12f (S113).

In the present embodiment, the control unit 11 acquires a nutrient amount and a contribution amount toward earth's environment (achievable amounts) which can be achieved by eating the article of general food selected by the user (S123). In this case, the control unit 11 specifies ingredients included in the general food and acquires an achievable amount by each ingredient. For example, when carbonara (an article of general food) is selected, the control unit 11 specifies ingredients such as spaghetti and bacon as ingredients included in carbonara and acquires an achievable amount by each ingredient. The achievable amount by each ingredient is stored in advance in, for example, the storage unit 12 or another server, and the control unit 11 acquires an achievable amount corresponding to each ingredient from the storage unit 12 or the other server. In addition, when there is a web server that discloses, for each menu (type of article of food), an achievable amount by each ingredient included in the menu, the control unit 11 may acquire the achievable amount from the web server. When the general food selected by the user is an ingredient such as spaghetti or bacon, the control unit 11 may acquire an achievable amount according to the selected ingredient.

Subsequently, the control unit 11 of the server 10 and the control unit 21 of the user terminal 20 execute processing of steps S115 to S118. Accordingly, based on the target information and an already-achieved amount of the user and an achievable amount by the article of general food selected by the user, the server 10 specifies an article of subscription food to be proposed to the user and provides the user terminal 20 with an advice screen for proposing the specified article of subscription food. In the present embodiment, in step S115, the control unit 11 specifies an article of subscription food that can be used as a substitute food of the article of general food selected by the user as the article of subscription food to be proposed to the user. Even in doing so, the control unit 11 specifies subscription food that should be ingested in order to achieve a remaining target ingestion amount that is calculated by subtracting the already-ingested amount of the user from the target ingestion amount of the user with respect to a nutrient. In addition, the control unit 11 specifies subscription food that should be ingested in order to achieve a remaining target contribution amount that is calculated by subtracting the already-contributed amount of the user from the target contribution amount of the user with respect to earth's environment. When target amounts are set with respect to a plurality of nutrients or a plurality of contribution items toward earth's environment, the control unit 11 specifies an article of subscription food that enables each target amount to be approached to a greater extent as the subscription food to be proposed. For example, when a low value is set as the target ingestion amount of sugars or lipids and a high value is set as the target ingestion amount of proteins, the control unit 11 specifies an article of subscription food of which an ingestion amount of sugars or lipids is low and an ingestion amount of proteins is high. Alternatively, during a period of achieving a target amount set by the user, an optimal amount of consumption of subscription food for achieving the target amount during the achievement period and a recipe for the consumption can be selected based on a degree of achievement calculated from an ingestion amount up to the point of input and the target ingestion amount. In addition, when a high value is set as a target reduction amount of food loss and a high value is set as the target ingestion amount of dietary fibers, the control unit 11 specifies an article of subscription food of which a reduction amount of food loss is high and an ingestion amount of dietary fibers is high. Furthermore, the control unit 11 may specify subscription food suitable for the user's preference by also taking preference information (dietary preferences) of the user into consideration.

FIG. 32B shows an advice screen example that is displayed when an image of carbonara is selected by the user on the screen shown in FIG. 32A. In a similar manner to FIG. 30B, the advice screen shown in FIG. 32B displays a target ingestion amount of dietary fiber and a target reduction amount of water use as target information set by the user and an already-ingested amount of dietary fiber and a reduction amount of water use as already achieved information. In addition, the screen shown in FIG. 32B displays, with respect to spaghetti included in the selected menu (in this case, carbonara), a message proposing vegetable noodles (an article of subscription food) that enables dietary fiber to be more efficiently ingested than commercially-available general spaghetti, and information that compares a dietary fiber amount due to general spaghetti and a dietary fiber amount due to the proposed vegetable noodles. When the proposed subscription food has a larger contribution amount toward earth's environment than the general food selected by the user, a message proposing the article of subscription food with a larger contribution amount toward earth's environment than the selected article of general food may be displayed on the screen shown in FIG. 32B. In doing so, information comparing the contribution amount toward earth's environment by the article of general food selected by the user and the contribution amount toward earth's environment by the proposed article of subscription food may be displayed. In addition, a link for displaying a menu (an ingestion method and a cooking method) using the proposed subscription food is set on the screen shown in FIG. 32B.

In the present embodiment, a similar advantageous effect to the respective embodiments described above can be obtained. In addition, in the present embodiment, when the user selects any article of food while viewing a website using the user terminal 20, the user can receive advice related to an article of subscription food for achieving target information of the user. In doing so, since an achievable amount with respect to a target of the user can be provided for the food selected by the user and the proposed subscription food, the user can evaluate purchasing and ingesting the subscription food by taking an achievable degree of the target set by the user himself/herself into consideration. The configuration according to the present embodiment can also be applied to the information processing system 100 according to the second to sixth embodiments, and a similar advantageous effect is obtained even when the configuration according to the present embodiment is applied to the information processing system 100 according to the second to sixth embodiments. Furthermore, the modifications described above in the respective embodiments can also be applied to the information processing system 100 according to the present embodiment.

The present embodiment is not limited to a configuration in which the target ingestion amount with respect to a nutrient and the target contribution amount with respect to earth's environment of the user are registered in the server 10 (the target amount DB 12f) in advance. For example, a configuration may be adopted in which, when the user selects articles of food (subscription food and general food) via a website being viewed by the user, an input screen of a target ingestion amount with respect to a nutrient and a target contribution amount with respect to earth's environment is displayed on the user terminal 20 and target amounts are acquired at this point. Even in this case, based on the target amount of the user and an achievable amount due to a selected article of food, advice related to an article of subscription food capable of better achieving the target amount can be provided. In such a configuration, even a user who is not registered as a member can receive a proposal of subscription food based on an input target amount.

### (Ninth embodiment, which is not encompassed by the wording of the claims but is useful for understanding the invention)

An information processing system that proposes purchasing subscription food to the user based on a desired purchase amount with respect to an article of subscription food or a desired ingestion amount with respect to any nutrient will be described. Since the information processing system according to the present embodiment can be realized by a similar apparatus to that of the information processing system 100 according to the first embodiment, a description of a configuration will be omitted. In the information processing system 100 according to the present embodiment, the user terminal 20 and the server 10 perform registration processing of user information similar to the processing shown in FIGS. 6 and 7. Accordingly, user information such as attribute information, profile information, biological information, exercise information, sleep information, and ingestion information of the user is input via the user terminal 20 and registered in the member information DB 12c of the server 10. In addition, in the present embodiment, the user terminal 20 and the server 10 perform advice provision processing similar to the processing shown in FIG. 11. Accordingly, the server 10 provides the user terminal 20 with advice in accordance with user information registered in the server 10. For example, while generating advice that encourages improvement from information indicating that an improvement in BMI in biological information is necessary as compared to a proper value, information indicating a deviation from a set target in ingestion information, and the like, noodles in accordance with preference information can be selected from articles of subscription foods based on information indicating a preference for noodles in preference information, and specific advice for proposing a method of use of subscription food which improves biological information and realizes a target value can be generated.

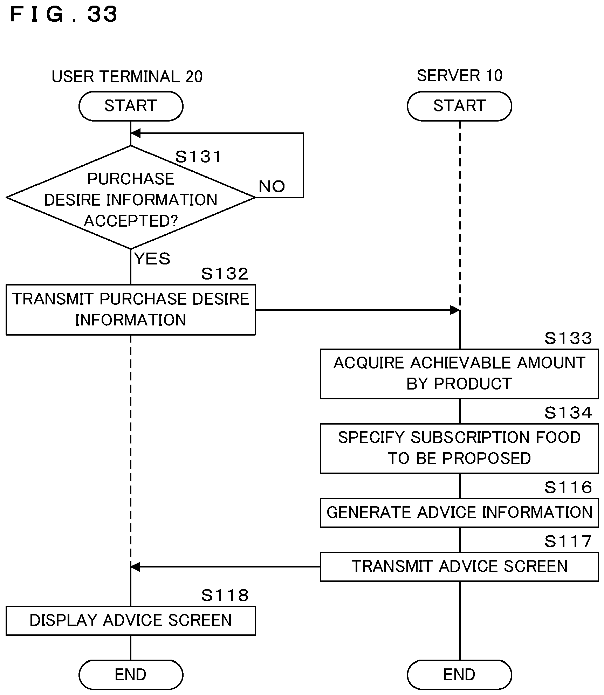

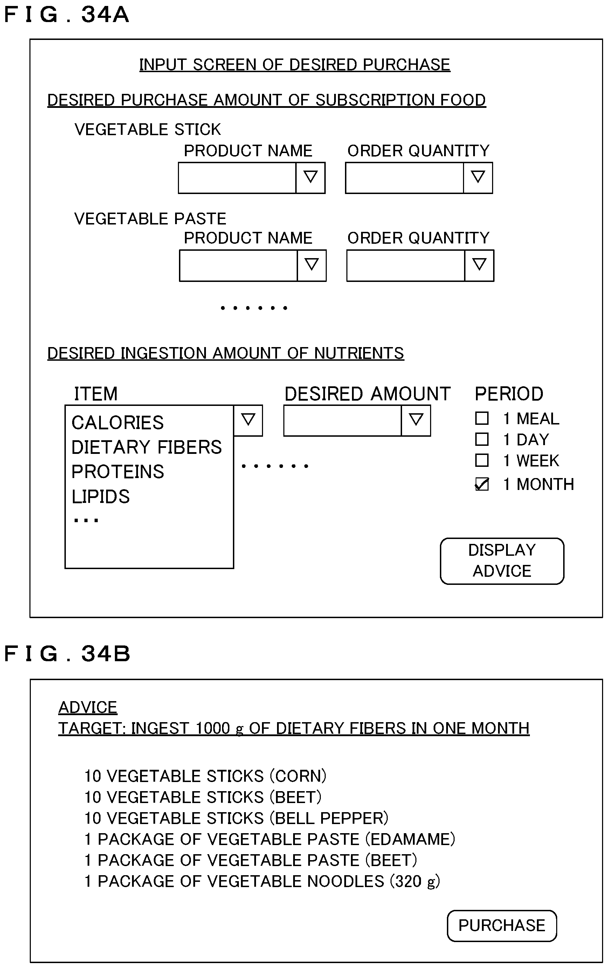

When the user designates a desired purchase amount with respect to subscription food or a desired ingestion amount with respect to any nutrient, the server 10 according to the present embodiment performs processing of advising subscription food for achieving desired contents. FIG. 33 is a flow chart showing an example of a provision processing procedure of an advice according to the ninth embodiment, and FIGS. 34A and 34B are schematic views showing a screen example of the user terminal 20. The processing shown in FIG. 33 represents an addition of steps S131 to S134 in place of steps S111 to S115 in the processing shown in FIG. 29. Descriptions of same steps as in FIG. 29 will be omitted.

In the information processing system 100 according to the present embodiment, the user causes the user terminal 20 to execute the advice app 22AP and display an input screen of purchase desire information. The control unit 21 of the user terminal 20 starts the advice app 22AP in accordance with an operation by the user and displays an input screen of purchase desire information such as that shown in FIG. 34A on the display unit 25. The screen shown in FIG. 34A has an input field for inputting a product name and an order quantity of a vegetable stick and an input field for inputting a product name and an order quantity of a vegetable paste as a desired purchase amount of subscription food. In addition, the screen shown in FIG. 34A has an input field for inputting an item and a desired amount of a nutrient as a desired ingestion amount of the nutrient. The input field of the item of a nutrient is provided with a pull-down menu that enables any one nutrient to be selected from a plurality of nutrients, and the input field of the desired amount is provided with a pull-down menu that enables any numerical value to be selected from a plurality of numerical values. A configuration may be adopted in which a desired ingestion amount with respect to a plurality of nutrients can be input as a desired ingestion amount of nutrients. Furthermore, the screen shown in FIG. 34A has a check box for selecting a period in which an input desired ingestion amount of a nutrient is to be achieved from a plurality of periods. The example shown in FIG. 34A is configured so that any one of a meal, a day, a week, and a month can be selected.

When an article of subscription food that the user wishes to purchase is determined, a product name and an order quantity of the subscription food are input to respective input fields on the input screen of purchase desire information. In addition, when an article of subscription food that the user wishes to purchase is not yet determined but a nutrient that the user wishes to ingest and an ingestion amount thereof are determined, an item and a desired amount of the nutrient are input to respective input fields on the input screen of purchase desire information. On the input screen of purchase desire information, after inputting the desired purchase amount of an article of subscription food or the desired ingestion amount of a nutrient, the user operates an advice display button. Alternatively, an item and a desired amount of the nutrient that the user desires to limit are input to respective input fields. On the input screen of purchase desire information, after operating the advice display button and referring to the desired amount and an advice in accordance with user information associated with the member ID, the user can input the desired purchase amount of the subscription food or the desired ingestion amount of the nutrient.

The control unit 21 of the user terminal 20 has determined whether or not a desired purchase amount of a subscription food or a desired ingestion amount of a nutrient (in other words, purchase desire information) has been accepted via the input screen of purchase desire information (S131), and when the control unit 21 determines that purchase desire information has not been accepted (S131: NO), the control unit 21 stands by while performing other processing. When the control unit 21 determines that purchase desire information has been accepted (S131: YES), the control unit 21 transmits the input purchase desire information to the server 10 after the advice display button is operated (S132).

When the control unit 11 of the server 10 receives purchase desire information from the user terminal 20, the control unit 11 acquires an achievable amount due to the subscription food that is a sales object (S133). When the purchase desire information is a product name and an order quantity of subscription food, the control unit 11 acquires an achievable amount due to the subscription food included in the purchase desire information, and when the purchase desire information is an item and a desired amount of a nutrient, the control unit 11 acquires an achievable amount due to all articles of subscription food. An achievable amount due to subscription food can be acquired from, for example, the product information DB 12a or another server. In addition, the control unit 11 specifies an article of subscription food to be proposed to the user based on the purchase desire information received from the user terminal 20 (S134). For example, when the purchase desire information is a product name and an order quantity of subscription food, the control unit 11 specifies subscription food that is similar to the subscription food included in the purchase desire information, subscription food with a larger achievable amount, and the like. In addition, when the purchase desire information is an item and a desired amount of a nutrient, the control unit 11 specifies an article of subscription food for achieving the desired amount of the nutrient.

In addition, the control unit 11 generates advice information for proposing the specified article of subscription food (S116). For example, when the purchase desire information is a product name and an order quantity of subscription food, the control unit 11 generates advice information that proposes purchase and ingestion of subscription food specified in step S134 in addition to the subscription food included in the purchase desire information. In addition, when the purchase desire information is an item and a desired amount of a nutrient, the control unit 11 generates advice information for achieving the desired amount of the nutrient due to the subscription food specified in step S134. FIG. 34B shows an advice screen example that is displayed when monthly ingestion of 1000 g of dietary fibers is input as a desired ingestion amount of a nutrient on the screen shown in FIG. 34A. On the advice screen shown in FIG. 34B, a set of articles of subscription food that enables 1000 g of dietary fibers to be ingested in a month is displayed.

The control unit 11 transmits the advice screen displaying the generated advice information to the user terminal 20 (S117), and the control unit 21 of the user terminal 20 displays the advice screen received from the server 10 on the display unit 25 (S118). When the user inputs purchase desire information using the user terminal 20 according to the processing described above, the server 10 can provide an advice on subscription food in line with the purchase desire information. Accordingly, the user can be provided with advice on subscription food to be purchased in order to achieve the purchase desire information input by the user himself/herself. Alternatively, menus that use each of the articles of subscription food may be displayed on the advice screen.

In the present embodiment, a similar advantageous effect to the respective embodiments described above can be obtained. In addition, in the present embodiment, by inputting purchase desire information using the user terminal 20, the user can receive advice related to an article of subscription food for achieving the purchase desire information. Therefore, when the user is undecided as to which subscription food he/she wants to purchase, by designating a nutrient that he/she wants to ingest, the user can be recommended of an article of subscription food which is capable of achieving an desired ingestion amount of the nutrient that the user wishes to ingest. Alternatively, by inputting purchase desire information by inputting an item and a desired amount of a nutrient that the user wishes to limit, the user can receive advice related to purchase information of an article of subscription food that is recommended for achieving the nutrient limitation desire information. Therefore, by taking a recommended product into consideration when evaluating the purchase and ingestion of subscription food, the user can use subscription food according to a subscription purchase method that enables the user to achieve a desired target. The configuration according to the present embodiment can also be applied to the information processing system 100 according to the second to eighth embodiments, and a similar advantageous effect is obtained even when the configuration according to the present embodiment is applied to the information processing system 100 according to the second to eighth embodiments. Furthermore, the modifications described above in the respective embodiments can also be applied to the information processing system 100 according to the present embodiment.

In the present embodiment, a configuration may be adopted in which recommends, based on a desired contribution amount toward earth's environment, an article of subscription food for achieving the desired contribution amount in addition to achieving a desired purchase amount with respect to subscription food or a desired ingestion amount with respect to any nutrient. In addition, in a case of a user already registered as a member, an article of subscription food to be recommended to the user may be specified based on various kinds of information included in user information registered in the member information DB 12c in addition to purchase desire information input via an input screen for purchase desire information. For example, an article of subscription food to be recommended to the user may be specified based on any or a plurality of pieces of information among purchase desire information, a purchase history, an ingestion history, exercise information, sleep information, biological information, preference information, psychosomatic information (stress, work efficiency, and bodily functions), and the like.

### (Tenth embodiment, which is not encompassed by the wording of the claims but is useful for understanding the invention)

While the seventh and eighth embodiments described above represent a configuration in which an article of subscription food suitable for a user is proposed when the user selects an article of food (subscription food or general food) via a website being viewed by the user, in the present embodiment, an information processing system will be described which proposes an article of food (including food other than subscription food) suitable for the user in accordance with contents of the website being viewed by the user. The food to be proposed to the user in the present embodiment is general food. Specifically, the food proposed to the user in the present embodiment may be food being sold in a subscription system (subscription food that is a sales object) or food being sold in a system other than subscription (for example, food being sold on a website or at a store). It its needless to say that users to be an object of providing the information processing system according to the present embodiment may be general users who have not purchased food by a subscription system or users who have already purchased food by a subscription system. Furthermore, users to be an object of providing the information processing system according to the present embodiment may be users who purchase food by a subscription service provided by a person who is the same or who is legally or economically equivalent to a person providing the information processing system according to the present embodiment or users who purchase food by a subscription service provided by another person. Since the information processing system according to the present embodiment can be realized by a similar apparatus to that of the information processing system 100 according to the seventh embodiment, a description of a configuration will be omitted. In addition, in the present embodiment, an ingestion history that is transmitted from the user terminal 20 to the server 10 in order to be stored in the member information DB 12c may include not only information on subscription food eaten by the user but may also include information on general food eaten by the user.

In the information processing system 100 according to the present embodiment, the user terminal 20 and the server 10 perform registration processing of user information similar to the processing shown in FIGS. 6, 7, and 14. Accordingly, the user terminal 20 can accept user information such as attribute information, profile information, biological information, exercise information, sleep information, and ingestion information (information on diet) of the user and the server 10 can register the user information input via the user terminal 20 in the member information DB 12c. In addition, in a similar manner to the seventh embodiment described above, the user terminal 20 according to the present embodiment registers a target ingestion amount of the user with respect to nutrients and a target contribution amount of the user with respect to earth's environment in the server 10 (the target amount DB 12f), and the server 10 registers an ingestion amount of each nutrient due to food ingested by the user and a contribution amount with respect to earth's environment in the target amount DB 12f based on ingestion information received from the user terminal 20.

Therefore, even in the present embodiment, a target ingestion amount with respect to nutrients and a target contribution amount with respect to earth's environment are set in the server 10 for each user and an ingestion amount of nutrients and a contribution amount toward earth's environment achieved by the user by eating meals are accumulated in the server 10. For example, when a user is viewing any website, the server 10 according to the present embodiment performs processing of advising food that is suitable for the user in accordance with contents displayed on the website.

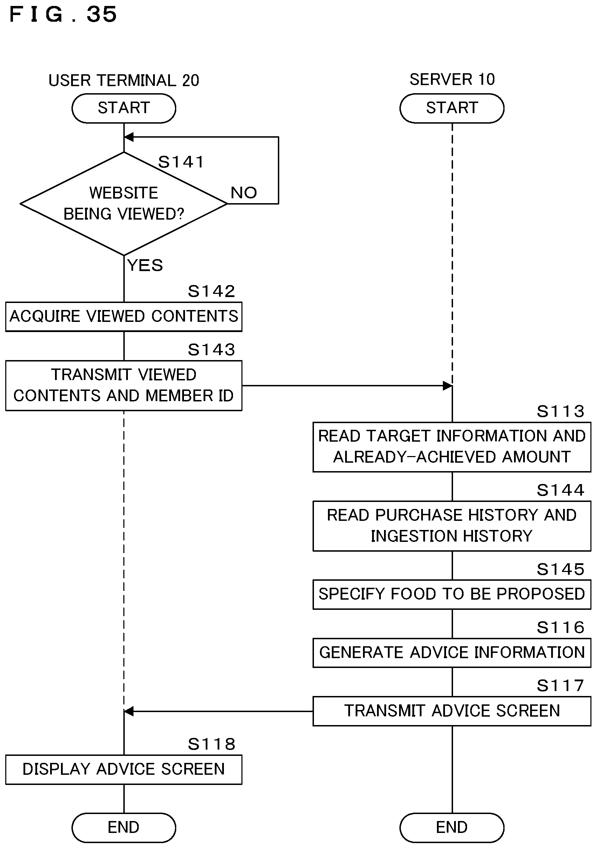

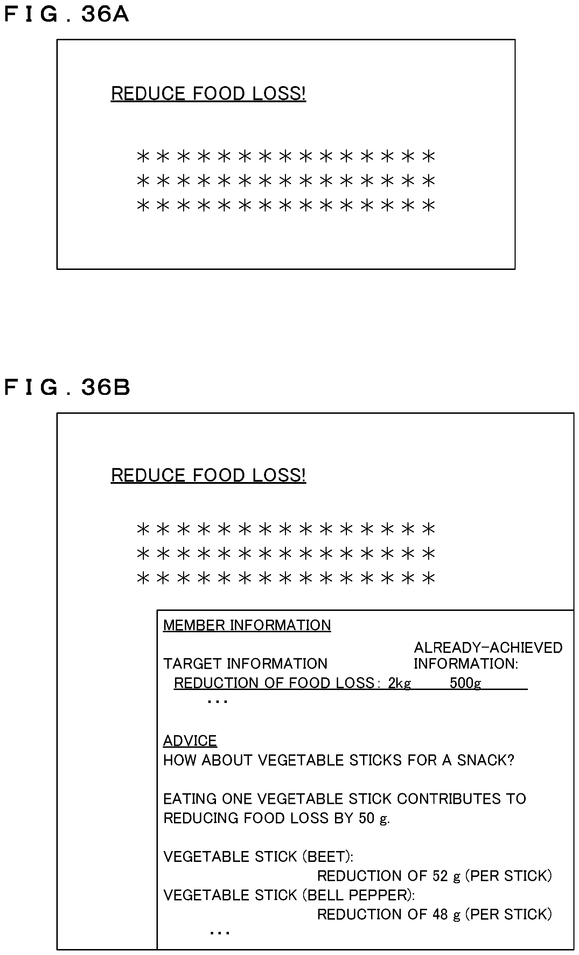

FIG. 35 is a flow chart showing an example of a provision processing procedure of an advice according to a tenth embodiment, and FIGS. 36A and 36B are schematic views showing a screen example of the user terminal 20. The processing shown in FIG. 35 represents an addition of steps S141 to S143 in place of steps S111 to S112 and an addition of steps S144 to S145 in place of steps S114 to S115 in the processing shown in FIG. 29. Descriptions of same steps as in FIG. 29 will be omitted. In the information processing system 100 according to the present embodiment, for example, when the user is viewing any website made public via a network N using the user terminal 20, the user terminal 20 and the server 10 perform the following processing. Let us assume that, for example, the user is viewing a website displaying information related to food loss as shown in FIG. 36A. The website to be viewed by the user is not limited to the configuration shown in FIG. 36A and may be websites displaying various kinds of information such as information related to various kinds of nutrients, information related to contributions toward earth's environment, and information related to diet, exercise, or sleep.

The control unit 21 of the user terminal 20 has determined whether or not the user is currently viewing the website (S141), and when the control unit 21 has determined that the user is not viewing the website (S141: NO), the control unit 21 stands by while performing other processing. When the control unit 21 determines that the user is viewing the website (S141: YES), the control unit 21 acquires displayed contents (viewed contents) of the website (S142) and transmits the acquired viewed contents and the member ID of the user to the server 10 (S143). For example, the control unit 21 may acquire contents of a title described on the website as viewed contents (specific information) or the control unit 21 may specify a word with a large number of occurrences on the website and acquire the specified word as viewed contents (specific information). The control unit 11 of the server 10 receives the viewed contents and the member ID from the user terminal 20. Accordingly, the control unit 11 (specific information acquiring unit) can acquire specific information that specifies display contents of the website being viewed by the user. Subsequently, the control unit 11 reads target information (the target ingestion amount of a nutrient and a target contribution amount toward earth's environment) and already-achieved amounts (the already-ingested amount of the nutrient and the already-contributed amount toward earth's environment) corresponding to the user from the target amount DB 12f (S113).

In the present embodiment, for example, the control unit 11 reads a purchase history and an ingestion history of the user from the member information DB 12c (S144) and, based on the viewed contents, target information and already-achieved amounts of the user, and the purchase history and the ingestion history of the user, specifies an article of food to be proposed to the user (S145). For example, an article of food to be proposed to the user may be registered in the storage unit 12 in advance in accordance with viewed contents of the user, and the control unit 11 specifies an article of food to be proposed to the user based on the viewed contents received from the user terminal 20. For example, in association with viewed contents related to earth's environment such as food loss, food with a large contribution toward earth's environment (for example, a large reduction amount in food loss) is registered in advance. In this case, the control unit 11 specifies an article of food with a large contribution toward earth's environment as the article of food to be provided to the user viewing the website shown in FIG. 36A. In addition, food with a large ingestion amount of proteins is registered in advance in association with viewed contents related to exercise, muscle training, or the like. In this case, the control unit 11 can specify an article of food with a large ingestion amount of proteins as the article of food to be provided to the user viewing a website related to exercise or muscle training. As described above, after specifying food in accordance with viewed contents of the user, an article of food for achieving target information is further specified from the specified food. Even in this case, the control unit 11 may specify an article of food for achieving a remaining target amount calculated by subtracting already-achieved amounts (an already-ingested amount of a nutrient and an already-contributed amount toward earth's environment) from target information (a target ingestion amount of the nutrient and a target contribution amount toward earth's environment). Specifically, the control unit 11 (target amount acquiring unit) acquires target information of the user from the target amount DB 12f. In addition, the control unit 11 (contribution amount acquiring unit) acquires an achievable nutrient amount and a contribution amount (achieved contribution amount) toward earth's environment with respect to, for example, each of food sold by a subscription system (subscription food that are sales objects) and various kinds of food sold via a website or a store. Furthermore, the control unit 11 specifies an article of food capable of further achieving target information (target amount) based on the achieved contribution amount (achievable amount) by each article of food. Note that the control unit 11 may specify an article of food with a high purchase frequency or a high ingestion frequency by the user or a low purchase frequency or a low ingestion frequency by the user in consideration of a purchase history and an ingestion history of the user. As described above, in the present embodiment, an article of food in accordance with viewed contents of the user and which achieves target information of the user is specified as an article of food to be proposed to the user. Among food in accordance with the viewed contents, an article of food to be proposed to the user may be food in accordance with the purchase history or the ingestion history of the user or food that conforms to preference information related to a preference or a principle regarding food of the user.

Subsequently, the control unit 11 of the server 10 and the control unit 21 of the user terminal 20 execute processing of steps S116 to S118. Accordingly, the server 10 can provide the user terminal 20 with an advice screen for proposing the article of food specified in step S145. FIG. 36B shows an advice screen example that is displayed when the website shown in FIG. 36A is being viewed. The advice screen shown in FIG. 36B displays target information set by the user and an already-achieved amount with respect to the target information in accordance with display contents of the website shown in FIG. 36A. The example shown in FIG. 36B displays a target reduction amount of food loss as target information set by the user and an achieved reduction amount of food loss. In addition, the screen shown in FIG. 36B displays a message for proposing an article of food for further achieving the target information of the user. Accordingly, advice related to an article of food for achieving target information set by the user can be displayed in accordance with contents of a website being viewed by the user. Alternatively, an ingestion method and a cooking method (menu) of the specified article of food may be displayed on the screen shown in FIG. 36B.

In the present embodiment, a similar advantageous effect to the respective embodiments described above can be obtained. In addition, in the present embodiment, when the user is viewing a website using the user terminal 20, the user can receive advice related to an article of food in accordance with viewed contents and which enables target information of the user to be achieved. Therefore, advice related to an article of food in accordance with viewed contents or, in other words, an article of food that is of interest to the user can be provided. In addition, in doing so, since an achieved contribution amount with respect to a target of the user can be provided for each article of food to be proposed, the user can evaluate purchasing and ingesting each article of food by taking an achievable degree of the target set by the user himself/herself into consideration. As described above, in the present embodiment, an article of food in accordance with viewed contents and which achieves target information set by the user can be proposed with viewing of a website as a trigger. The configuration according to the present embodiment can also be applied to the information processing system 100 according to the second to sixth and eighth to ninth embodiments, and a similar advantageous effect is obtained even when the configuration according to the present embodiment is applied to the information processing system 100 according to the second to sixth and eighth to ninth embodiments. In addition, in the information processing system 100 according to the present embodiment, a part of a configuration of modifications described from time to time in each of the embodiments described above or to be described later can be applied to the present embodiment by performing appropriate adjustments including changing object food from subscription food to general food (including food other than subscription food) as necessary and also changing users as objects to which the information processing system is to be provided to general users (including users who do not purchase food by a subscription system).

The present embodiment is not limited to a configuration in which processing of specifying food to be proposed to the user in accordance with contents of a website being viewed by the user is performed by the server 10. For example, by registering food to be proposed to the user in the user terminal 20 in advance in accordance with food information that can be directly or indirectly specified from contents of a website, the user terminal 20 can specify food in accordance with viewed contents while the user is viewing the website. Even in this case, the user terminal 20 may further select an article of food for achieving target information from food in accordance with the viewed contents and may further select an article of food in accordance with a purchase history and an ingestion history of the user.

The present embodiment is also not limited to a configuration in which the target ingestion amount with respect to a nutrient and the target contribution amount with respect to earth's environment of the user are registered in the server 10 (the target amount DB 12f) in advance. For example, a configuration may be adopted in which an input screen of a target ingestion amount with respect to a nutrient and a target contribution amount with respect to earth's environment is displayed on the user terminal 20 at any timing while the user is viewing a website and target amounts are acquired at this point. Even in this case, based on the target amounts of the user and an achieved contribution amount due to the article of food to be proposed to the user, advice on an article of food capable of achieving the target amount to a greater extent can be provided. Alternatively, a target ingestion amount with respect to a nutrient and a target contribution amount with respect to earth's environment may be specified and used from attribute information or the like of the user. In the case of such a configuration, even a user who is not registered as a member can receive a proposal of an article of food in accordance with contents of a website being viewed by the user.

### (Eleventh embodiment)

The eighth embodiment described above represents a configuration which, when a user selects an article of general food via a website being viewed by the user, proposes an article of subscription food suitable for the user. In the present embodiment, an information processing system will be described which proposes a substitutable ingredient (article of food) with respect to each ingredient contained in an article of general food selected by the user in the eighth embodiment described above. Since the information processing system according to the present embodiment can be realized by a similar apparatus to that of the information processing system 100 according to the eighth embodiment, a description of a configuration will be omitted.

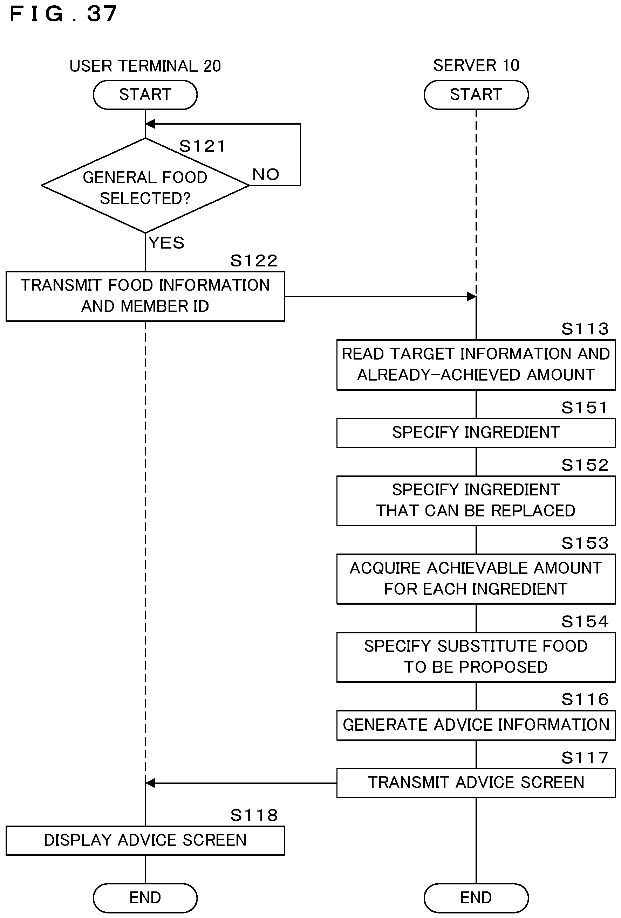

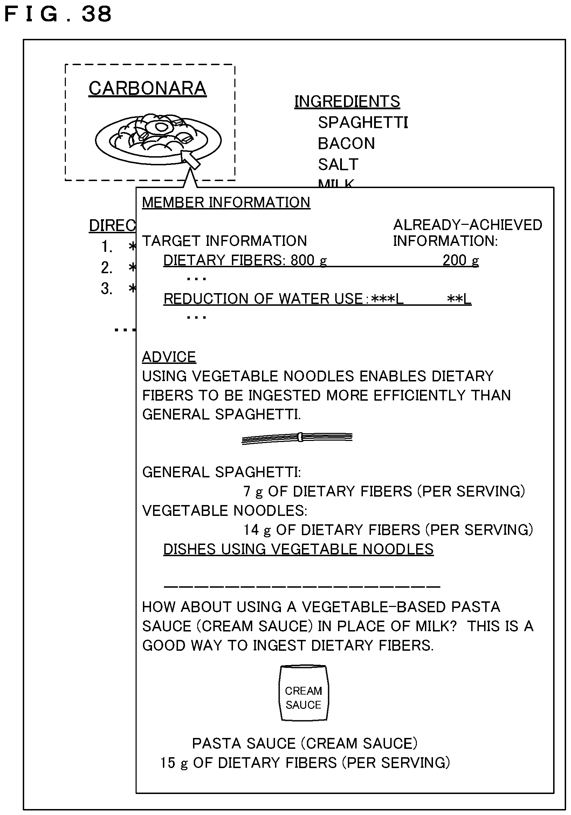

FIG. 37 is a flow chart showing an example of a provision processing procedure of an advice according to an eleventh embodiment, and FIG. 38 is a schematic view showing a screen example of the user terminal 20. The processing shown in FIG. 37 represents an addition of steps S151 to S154 in place of steps S123 and S115 in the processing shown in FIG. 31. Descriptions of same steps as in FIG. 31 will be omitted. In the information processing system according to the present embodiment, the control unit 21 of the user terminal 20 and the control unit 11 of the server 10 perform processing of steps S121 to S122 and S113 in FIG. 31. Accordingly, when an article of general food is selected via the website being displayed, the control unit 21 of the user terminal 20 transmits information on the selected article of food to the server 10. When the photograph of carbonara is selected on the website shown in FIG. 32A, the control unit 21 transmits information on the selected article of food (information on carbonara or ingredients used in carbonara) to the server 10. The control unit 11 of the server 10 reads target information set by the user and an already-achieved amount from the target amount DB 12f (S113).

In the present embodiment, the control unit 11 specifies an ingredient included in the article of general food selected by the user (S151). For example, when a user selects an article of food or when information that enables an article of food to be directly or indirectly specified is displayed on a website and an article of food is selected by the system based on the displayed food information and when information on an ingredient used in the article of food selected by the user or the system (both may be collectively simply referred to as "an article of food selected by the user") has been transmitted from the user terminal 20, the control unit 11 specifies the ingredient included in the article of food selected by the user from the received information. In addition, when a menu of the article of food selected by the user has been transmitted from the user terminal 20, the control unit 11 may acquire an ingredient generally used in the received menu from, for example, a server that publishes menus of various articles of food. Next, the control unit 11 specifies an ingredient that can be replaced with a substitute ingredient (substitute article of food) among ingredients included in the article of food selected by the user (S152). For example, by registering vegetable noodles in association with spaghetti (pasta) in the storage unit 12 as a substitute food of spaghetti (pasta), the control unit 11 can specify spaghetti as an ingredient that can be replaced with vegetable noodles (substitute food). In addition, by registering a pasta sauce (cream sauce) in association with milk or heavy cream in the storage unit 12 as a substitute food of milk or heavy cream, the control unit 11 can specify milk or heavy cream as an ingredient that can be replaced with pasta sauce (substitute food). Therefore, for example, when carbonara (an article of general food) is selected by the user, ingredients such as spaghetti, bacon, and milk are specified as ingredients contained in carbonara and spaghetti and milk are specified as ingredients that can be replaced with articles of substitute food.

In addition, with respect to each of the ingredients that can be replaced with articles of substitute food and the articles of substitute food, the control unit 11 acquires a nutrient amount and a contribution amount toward earth's environment (achievable amounts) which can be achieved by the ingredient (S153). The achievable amount (achieved contribution amount) by each ingredient is stored in advance in, for example, the storage unit 12 or another server, and the control unit 11 acquires an achievable amount corresponding to each ingredient from the storage unit 12 or the other server.

Based on the target information and the already-achieved amount read in step S113 and the achievable amount of each ingredient acquired in step S153, the control unit 11 specifies (selects) an article of food (substitute food) to be proposed to the user with respect to ingredients that can be replaced with articles of substitute food (S154). At this point, with respect to each of the ingredients that can be replaced with articles of substitute food, when an achievable amount due to an article of substitute food is larger than an achievable amount due to each ingredient, the control unit 11 specifies the article of substitute food as the article of food to be provided to the user. The control unit 11 specifies an article of subscription food that should be ingested in order to achieve remaining target information that is calculated by subtracting the already-achieved amount of the user from the target information of the user as an article of food to be provided to the user. The article of substitute food at this point may be subscription food that is a sales object or food being sold via a website or a store.

Subsequently, the control unit 11 of the server 10 and the control unit 21 of the user terminal 20 execute processing of steps S116 to S118. Accordingly, with respect to an ingredient that can be replaced with an article of substitute food among ingredients contained in food selected by the user, the server 10 can provide the user terminal 20 with an advice screen that proposes food (substitute food) for achieving target information of the user. FIG. 38 shows an advice screen example that is displayed when an image of carbonara is selected by the user on the screen shown in FIG. 32A. The advice screen shown in FIG. 38 displays target information (a target ingestion amount of dietary fiber and a target reduction amount of water use) set by the user and already achieved information (an already-ingested amount of dietary fiber and a reduction amount of water use). In addition, the screen shown in FIG. 38 displays, with respect to spaghetti included in the selected menu, a message proposing vegetable noodles that enables dietary fiber to be more efficiently ingested than general spaghetti, and with respect to milk, displays a message proposing a vegetable pasta sauce that enables dietary fiber to be more efficiently ingested than milk. In this manner, with respect to each ingredient that can be replaced with a substitute ingredient among ingredients contained in an article of food selected by the user, an advice proposing replacing the ingredient with the substitute ingredient can be made.

In the present embodiment, a similar advantageous effect to the respective embodiments described above can be obtained. In addition, in the present embodiment, when the user selects any article of food while viewing a website, the user can receive advice related to an article of substitute food for achieving target information of the user with respect to an ingredient that can be replaced with an article of substitute food among ingredients contained in the selected article of food. The configuration according to the present embodiment can also be applied to the information processing system 100 according to the second to seventh and ninth to tenth embodiments, and a similar advantageous effect is obtained even when the configuration according to the present embodiment is applied to the information processing system 100 according to the second to seventh and ninth to tenth embodiments. In addition, in the information processing system 100 according to the present embodiment, a part of a configuration of modifications described from time to time in each of the embodiments described above can be applied to the present embodiment by performing appropriate adjustments including changing object food from subscription food to general food (including food other than subscription food) as necessary and changing users as objects to which the information processing system is to be provided to general users who have not purchased food by a subscription system.

In the information processing system 100 according to each embodiment described above, when generating advice information to be provided to a user, the control unit 11 of the server 10 may also take into consideration an article of subscription food not being currently purchased or ingested by the user among articles of subscription food in addition to the user information stored in the member information DB 12c. Accordingly, for example, advice information can be generated which recommends purchasing subscription food that the user has never purchased nor ingested or subscription food that the user had purchased or ingested in the past but is not currently doing so. Information on subscription food not purchased nor ingested by the user may be input by the user via the user terminal 20 to be registered in the server 10. In addition, the server 10 may specify subscription food other than subscription food having been purchased by the user among subscription food that is a sales object and consider the specified subscription food to be subscription food not being currently purchased or ingested by the user.

In the information processing system 100 according to each embodiment described above, the control unit 21 of the user terminal 20 can display a screen for inputting user information, prompt the user to input user information, and accept each piece of user information. The control unit 11 of the server 10 can determine that a user is a general user based on information indicating that the user has not input user information or, even if the user has input a part of information of user information, identification information of the user is not stored in the member information DB 12c. The control unit 21 of the user terminal 20 of the user having input information indicating that the user is a general user can present the user with a display for executing or not executing member registration. When the user executes member registration, a screen for inputting various kinds of user information is subsequently displayed and user information is accepted, and the server 10 registers user information input via the user terminal 20 in the member information DB 12c. After registration, as various kinds of advantageous effects in procedures up to advice presentation, advantageous effects equivalent to those described earlier are to be obtained. Even if the user defers member registration by selecting, for example, a display such as "later", the control unit 21 of the user terminal 20 can generate, from input information, user member information in accordance with an attribute, preference information, a trend of thought, a physical state, a dietary state, a state of exercise, a state of sleep, or the like of the user input by the control unit 11 of the server 10, and from information stored in the member information DB 12c and similar user information, an optimal advice (using similar ranking information, user thought prediction learning information by an AI, or the like). Alternatively, a classification based on the user information can be performed and an optimal advice can be generated from classification information relevant to the user among the classification condition DB.

In the information processing system 100 according to each embodiment described above, a provision destination to which the server 10 provides an advice is not limited to the user terminal 20 and may be, for example, a terminal (user terminal 20) of a guardian, a family member, a friend, a helper, or the like of the user. In this case, by registering a terminal of a provision destination of advice in the server 10 in advance, when the server 10 generates advice information with respect to the user, the advice information can be provided to the guardian of the user or the like. Therefore, also providing advice information to those other than the user himself/herself enables various kinds of information of the user related to diet, exercise, sleep, and the like to be shared between the user and the guardian or the like, and it is expected that such sharing of information may lead to improvements in lifestyle habits, stress, bodily functions, work efficiency, and cognition of the user.

In the information processing system 100 according to each embodiment described above, processing that is realized by the user terminal 20 by executing the advice app 22AP may be configured to be divided among a plurality of application programs. For example, a configuration may be adopted in which processing of the user terminal 20 acquiring information (user information) related to a user via the input unit 24 or the like and accumulating the information in the server 10 and processing of acquiring, from the server 10, advice in accordance with the user information accumulated in the server 10 are realized by different application programs. In this case, by executing a program for accumulating user information, the control unit 21 of the user terminal 20 performs processing of accepting user information such as attribute information, profile information, biological information, exercise information, sleep information, and a type, an ingestion amount, and an ingestion timing of food eaten by the user (user ingestion information) via the input unit 24 or the like and transmitting the information to the server 10. In addition, by executing a program for acquiring advice, the control unit 21 performs processing of transmitting a member ID (identification information) of the user to the server 10 to request for an advice, acquiring advice in accordance with the user information of the user from the server 10, and displaying the advice on the display unit 25. Even when such a configuration is adopted, similar processing to each embodiment described above can be performed and a similar advantageous effect can be obtained. The user information accumulated in the server 10 may be input via an application program stored in the user terminal 20 or may be input via a predetermined website being made public via the network N. In addition, when the user information is stored in another storage apparatus, the server 10 may be configured to acquire the user information from the other storage apparatus.

In the information processing system 100 according to each embodiment described above, processing performed by the server 10 may be distributed among a plurality of servers. For example, processing by the server 10 of accumulating user information acquired from each user terminal 20 and processing by the server 10 of providing each user terminal 20 with an advice in accordance with the accumulated user information may be performed by different servers. In this case, an accumulation server that accumulates user information performs processing of acquiring various kinds of information (user information) related to the user input using the user terminal 20 and accumulating the information in the member information DB 12c such as that shown in FIG. 4. On the other hand, a provision server that provides advice performs processing of acquiring, in accordance with a request from the user terminal 20 or the like, user information of a user corresponding to the user terminal 20 from the accumulation server, generating advice in accordance with the acquired user information based on stored contents of the advice DB 12d such as that shown in FIG. 5, and outputting the generated advice to the user terminal 20. At this point, for example, the provision server has acquired a member ID (identification information) of the user from the user terminal 20 and has accepted a request for an advice, and the provision server acquires user information corresponding to the accepted member ID from the accumulation server and generates advice in accordance with the acquired user information. The user terminal 20 having acquired an advice generated in this manner can provide the user of the user terminal 20 with an advice in accordance with user information by displaying the acquired advice on the display unit 25. For example, a configuration can be adopted in which a vendor selling subscription food accumulates user information using the accumulation server, and a provider providing advice uses the accumulated user information to have a provision server provide advice suitable for each user.

### [Description of Reference Numerals]

- 100: Information processing system
- 10: Server
- 11: Control unit
- 12: Storage unit
- 13: Communication unit
- 20: User terminal
- 21: Control unit
- 22: Storage unit
- 23: Communication unit
- 25: Display unit
- 27: Camera
- 12a: Product information DB
- 12c: Member information DB
- 12d: Advice DB

## Claims

1. An information processing method for proposing ingestion of subscription food which is sold by a subscription system that allows a user to consume as much products as possible within a certain range within an arbitrary period for a certain price, by which a computer (10) executes processing of:
acquiring, from a terminal (20) of the user, specific information that specifies contents displayed on a display screen (25) of the terminal (20), the specific information including information specifying food selected via the display screen (25) of the terminal;
specifying a replaceable ingredient that can be replaced with a substitute ingredient among ingredients contained in the selected food;
respectively acquiring first and second achieved contribution amounts toward an item that are achieved by ingesting the replaceable ingredient and by ingesting the substitute ingredient;
acquiring a target amount of the user with respect to the item;
selecting, based on the first and second achieved contribution amounts and the target amount of the user, a proposed substitute ingredient for achieving the target amount of the user;
generating, in accordance with the specific information, based on the proposed substitute ingredient, advice information including:
information that compares the first achieved contribution amount and the second achieved contribution amount, and
an ingestion method and a cooking method of the proposed substitute ingredient for achieving the target amount of the user; and
outputting the generated advice information to the terminal (25) of the user.

2. The information processing method according to claim 1, by which the computer (10) executes processing of:
selecting the proposed substitute ingredient for achieving the target amount of the user from ingredients sold by the subscription system.

3. Computer program (12P) having instructions which cause the computer (10) to perform the method according to claim 1 or 2.

4. An information processing apparatus (10) for proposing ingestion of subscription food which is sold by a subscription system that allows a user to consume as much products as possible within a certain range within an arbitrary period for a certain price, comprising:
a specific information acquiring unit (11) configured to acquire, from a terminal (20) of the user, specific information that specifies contents displayed on a display screen (25) of the terminal (20), the specific information including information specifying food selected via the display screen (25) of the terminal (20);
a replaceable ingredient specifying unit (11) configured to specify a replaceable ingredient that can be replaced with a substitute ingredient among ingredients contained in the selected food;
a contribution amount acquiring unit (11) configured to acquire first and second achieved contribution amounts toward an item that are achieved by ingesting the replaceable ingredient and by ingesting the substitute ingredient, respectively;
a target amount acquiring unit (11) configured to acquire a target amount of the user with respect to the item;
a proposed substitute ingredient selecting unit (11) configured to select, based on the first and second achieved contribution amounts and the target amount of the user, a proposed substitute ingredient for achieving the target amount of the user;
a generating unit (11) configured to generate, in accordance with the specific information, based on the proposed substitute ingredient, advice information including:
information that compares the first achieved contribution amount and a second achieved contribution amount, and
an ingestion method and a cooking method of the proposed substitute ingredient for achieving the target amount of the user; and
an output unit (13) configured to output the generated advice information to the terminal of the user.

5. The information processing apparatus according to claim 4, wherein
the substitute ingredient selection unit (11) is configured to select the proposed substitute ingredient for achieving the target amount of the user from ingredients sold by the subscription system.

## Patentansprüche

1. Informationsverarbeitungsverfahren zum Vorschlagen einer Aufnahme von Abonnementlebensmitteln, die durch ein Abonnementsystem verkauft werden, das es einem Benutzer ermöglicht, so viele Produkte wie möglich innerhalb einer gewissen Palette innerhalb eines willkürlichen Zeitraums zu einem gewissen Preis zu konsumieren, wobei ein Computer (10) das Verarbeiten von Folgendem ausführt:
Erfassen spezifischer Informationen von einem Endgerät (20) des Benutzers, die Inhalte vorgeben, die auf einem Anzeigebildschirm (25) des Endgeräts (20) angezeigt werden, wobei die spezifischen Informationen Informationen beinhalten, die Lebensmittel vorgeben, die über den Anzeigebildschirm (25) des Endgeräts ausgewählt werden;
Vorgeben einer ersetzbaren Zutat, die mit einer Alternativzutat aus den in dem ausgewählten Lebensmittel enthaltenen Zutaten ersetzt werden kann;
Erfassen von jeweils einer ersten und zweiten erreichte Beitragsmenge zu einem Artikel, die durch Aufnehmen der austauschbaren Zutat und durch Aufnehmen der Alternativzutat erreicht werden;
Erfassen einer Zielmenge des Benutzers in Bezug auf den Artikel;
Auswählen einer vorgeschlagenen Alternativzutat auf Grundlage der ersten und zweiten erreichten Beitragsmenge und der Zielmenge des Benutzers, um die Zielmenge des Benutzers zu erreichen;
Generieren von Beratungsinformationen, die Folgendes beinhalten, in Übereinstimmung mit den spezifischen Informationen auf Grundlage der vorgeschlagenen Alternativzutat:
Informationen, welche die erste erreichte Beitragsmenge und die zweite erreichte Beitragsmenge vergleichen, und
ein Aufnahmeverfahren und ein Kochverfahren der vorgeschlagenen Ersatzzutat zum Erreichen der Zielmenge des Benutzers; und
Ausgeben der generierten Beratungsinformationen an das Endgerät (25) des Benutzers.

2. Informationsverarbeitungsverfahren nach Anspruch 1, bei dem der Computer (10) das Verarbeiten des Auswählens der vorgeschlagenen Alternativzutat ausführt, um die Zielmenge des Benutzers aus Zutaten zu erreichen, die durch das Abonnementsystem verkauft werden.

3. Computerprogramm (12P), das Anweisungen aufweist, die bewirken, dass der Computer (10) das Verfahren nach Anspruch 1 oder 2 durchführt.

4. Informationsverarbeitungseinrichtung (10) zum Vorschlagen einer Aufnahme von Abonnementlebensmitteln, die durch ein Abonnementsystem verkauft werden, das es einem Benutzer ermöglicht, so viele Produkte wie möglich innerhalb einer gewissen Palette innerhalb eines willkürlichen Zeitraums zu einem gewissen Preis zu konsumieren, umfassend:
eine Einheit (11) zum Erfassen spezifischer Informationen, die dazu konfiguriert ist, spezifische Informationen, die Inhalte vorgeben, die auf einem Anzeigebildschirm (25) eines Endgeräts (20) des Benutzers angezeigt werden, von dem Endgerät (20) zu erfassen, wobei die spezifischen Informationen Informationen beinhalten, die Lebensmittel vorgeben, die über den Anzeigebildschirm (25) des Endgeräts (20) ausgewählt werden;
eine Einheit (11) zum Vorgeben ersetzbarer Zutaten, die dazu konfiguriert ist, eine ersetzbare Zutat vorzugeben, die mit einer Alternativzutat aus den in dem ausgewählten Lebensmittel enthaltenen Zutaten ersetzt werden kann;
eine Einheit (11) zum Erfassen von Beitragsmengen, die dazu konfiguriert ist, eine erste und zweite erreichte Beitragsmenge zu einem Artikel zu erfassen, die jeweils durch Aufnehmen der austauschbaren Zutat und durch Aufnehmen der Alternativzutat erreicht werden;
eine Einheit (11) zum Erfassen von Zielmengen, die dazu konfiguriert ist, eine Zielmenge des Benutzers in Bezug auf den Artikel zu erfassen;
eine Einheit (11) zum Auswählen vorgeschlagener Alternativzutaten, die dazu konfiguriert ist, auf Grundlage der ersten und zweiten erreichten Beitragsmenge und der Zielmenge des Benutzers eine vorgeschlagene Alternativzutat auszuwählen, um die Zielmenge des Benutzers zu erreichen;
eine Generierungseinheit (11), die dazu konfiguriert ist, Beratungsinformationen, die Folgendes beinhalten, in Übereinstimmung mit den spezifischen Informationen auf Grundlage der vorgeschlagenen Alternativzutat zu generieren:
Informationen, welche die erste erreichte Beitragsmenge und eine zweite erreichte Beitragsmenge vergleichen, und
ein Aufnahmeverfahren und ein Kochverfahren der vorgeschlagenen Ersatzzutat zum Erreichen der Zielmenge des Benutzers; und
eine Ausgabeeinheit (13), die dazu konfiguriert ist, die generieren Beratungsinformationen an das Endgerät des Benutzers auszugeben.

5. Informationsverarbeitungsvorrichtung nach Anspruch 4, wobei
die Einheit (11) zur Auswahl von Alternativzutaten dazu konfiguriert ist, die vorgeschlagene Alternativzutat auszuwählen, um die Zielmenge des Benutzers aus Zutaten zu erreichen, die durch das Abonnementsystem verkauft werden.

## Revendications

1. Procédé de traitement d'informations permettant de proposer l'ingestion d'aliments d'abonnement vendus par un système d'abonnement qui permet à un utilisateur de consommer autant de produits que possible dans une plage donnée, pendant une période arbitraire, pour un prix donné, par lequel un ordinateur (10) exécute le traitement de :
l'acquisition, à partir d'un terminal (20) de l'utilisateur, d'informations spécifiques qui spécifient des contenus affichés sur un écran d'affichage (25) du terminal (20), les informations spécifiques comprenant des informations spécifiant les aliments sélectionnés via l'écran d'affichage (25) du terminal ;
la spécification d'un ingrédient remplaçable qui peut être remplacé par un ingrédient de substitution parmi les ingrédients contenus dans l'aliment sélectionné ;
l'acquisition respective de première et seconde quantités d'apport atteintes vis-à-vis d'un article qui sont atteintes par l'ingestion de l'ingrédient remplaçable et par l'ingestion de l'ingrédient de substitution ;
l'acquisition d'une quantité cible de l'utilisateur par rapport à l'article ;
la sélection, sur la base des première et seconde quantités d'apport atteintes et de la quantité cible de l'utilisateur, d'un ingrédient de substitution proposé pour atteindre le montant cible de l'utilisateur ;
la génération, conformément aux informations spécifiques, sur la base de l'ingrédient de substitution proposé, d'informations de conseil comprenant :
des informations qui comparent la première quantité d'apport atteinte et la seconde quantité d'apport atteinte, et
un procédé d'ingestion et un procédé de cuisson de l'ingrédient de substitution proposé pour atteindre la quantité cible de l'utilisateur ; et
la sortie des informations de conseil générées sur le terminal (25) de l'utilisateur.

2. Procédé de traitement d'informations selon la revendication 1, par lequel l'ordinateur (10) exécute le traitement de :
la sélection de l'ingrédient de substitution proposé pour atteindre la quantité cible de l'utilisateur à partir d'ingrédients vendus par le système d'abonnement.

3. Programme informatique (12P) comportant des instructions qui amènent l'ordinateur (10) à exécuter le procédé selon la revendication 1 ou 2.

4. Appareil de traitement d'informations (10) permettant de proposer l'ingestion d'aliments d'abonnement vendus par un système d'abonnement qui permet à un utilisateur de consommer autant de produits que possible dans une plage donnée, pendant une période arbitraire, pour un prix donné, comprenant :
une unité d'acquisition d'informations spécifiques (11) configurée pour acquérir, à partir d'un terminal (20) de l'utilisateur, des informations spécifiques qui spécifient des contenus affichés sur un écran d'affichage (25) du terminal (20), les informations spécifiques comprenant des informations spécifiant les aliments sélectionnés via l'écran d'affichage (25) du terminal (20) ;
une unité de spécification d'ingrédient remplaçable (11) configurée pour spécifier un ingrédient remplaçable qui peut être remplacé par un ingrédient de substitution parmi les ingrédients contenus dans l'aliment sélectionné ;
une unité d'acquisition de quantité d'apport (11) configurée pour acquérir des première et seconde quantités d'apport atteintes vis-à-vis d'un article qui sont atteintes par l'ingestion de l'ingrédient remplaçable et par l'ingestion de l'ingrédient de substitution, respectivement ;
une unité d'acquisition de quantité cible (11) configurée pour acquérir une quantité cible de l'utilisateur par rapport à l'article ;
une unité de sélection d'ingrédient de substitution proposé (11) configurée pour sélectionner, sur la base des première et seconde quantités d'apport atteintes et de la quantité cible de l'utilisateur, un ingrédient de substitution proposé pour atteindre la quantité cible de l'utilisateur ;
une unité de génération (11), configurée pour générer, conformément aux informations spécifiques, sur la base de l'ingrédient de substitution proposé, des informations de conseil comprenant :
des informations qui comparent la première quantité d'apport atteinte et une seconde quantité d'apport atteinte, et
un procédé d'ingestion et un procédé de cuisson de l'ingrédient de substitution proposé pour atteindre la quantité cible de l'utilisateur ; et
une unité de sortie (13) configurée pour sortir les informations de conseil générées sur le terminal de l'utilisateur.

5. Appareil de traitement d'informations selon la revendication 4, dans lequel
l'unité de sélection d'ingrédient de substitution (11) est configurée pour sélectionner l'ingrédient de substitution proposé pour atteindre la quantité cible de l'utilisateur à partir d'ingrédients vendus par le système d'abonnement.
